(19) Europäisches Patentamt
European Patent Office
Office européen des brevets

(11) EP 3 450 570 B1

(12) **EUROPEAN PATENT SPECIFICATION**

(45) Date of publication and mention of the grant of the patent:
**10.03.2021 Bulletin 2021/10**

(21) Application number: **17789981.2**

(22) Date of filing: **28.04.2017**

(51) Int Cl.:
***C12Q 1/42*** *(2006.01)*
***C12N 9/22*** *(2006.01)*
***C40B 40/06*** *(2006.01)*
***G16B 30/00*** *(2019.01)*
***C12N 15/63*** *(2006.01)*
***C12N 15/10*** *(2006.01)*
***C12N 15/11*** *(2006.01)*

(86) International application number:
**PCT/KR2017/004610**

(87) International publication number:
**WO 2017/188797 (02.11.2017 Gazette 2017/44)**

# (54) METHOD FOR EVALUATING, IN VIVO, ACTIVITY OF RNA-GUIDED NUCLEASE IN HIGH-THROUGHPUT MANNER

VERFAHREN ZUR IN-VIVO-BEURTEILUNG DER AKTIVITÄT VON RNA-GESTEUERTER NUKLEASE AUF HOCHDURCHSATZWEISE

PROCÉDÉ D'ÉVALUATION, IN VIVO, DE L'ACTIVITÉ D'UNE NUCLÉASE GUIDÉE PAR ARN DE MANIÈRE TRÈS EFFICACE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2016 KR 20160052365**

(43) Date of publication of application:
**06.03.2019 Bulletin 2019/10**

(73) Proprietor: **Industry-Academic Cooperation Foundation, Yonsei University**
**Seoul 03722 (KR)**

(72) Inventors:
- **KIM, Hyong Bum**
  **Seoul 04751 (KR)**
- **KIM, Hui Kwon**
  **Jincheon-gun**
  **Chungcheongbuk-do 27867 (KR)**
- **SONG, Myung Jae**
  **Seoul 01409 (KR)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) References cited:
**EP-A2- 3 009 511 WO-A1-2014/099744**

- **PATRICK D HSU ET AL: "DNA targeting specificity of RNA-guided Cas9 nucleases", HHS PUBLIC ACCESS AUTHOR MANUSCRIPT, vol. 31, no. 9, 21 July 2013 (2013-07-21), pages 1-17, XP055513966, DOI: 10.1038/nbt.2647**
- **Soobin Jung: "Genome-wide prediction and profiling of Off-target cleavage by CRISPR-Cas9", , 1 June 2018 (2018-06-01), pages 1-57, XP055630777, Retrieved from the Internet: URL:https://ir.ymlib.yonsei.ac.kr/bitstream/22282913/166400/1/T014876.pdf [retrieved on 2019-10-10]**
- **DAQI WANG ET AL: "Optimized CRISPR guide RNA design for two high-fidelity Cas9 variants by deep learning", NATURE COMMUNICATIONS, vol. 10, no. 1, 19 September 2019 (2019-09-19), XP055631062, DOI: 10.1038/s41467-019-12281-8**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- AGROTIS ET AL.: 'A New Age in Functional Genomics Using CRISPR/Cas9 in Arrayed Library Screening' FRONTIERS IN GENETICS vol. 6, no. 300, September 2015, pages 1 - 15, XP055434268
- YAMANO ET AL.: 'Crystal Structure of Cpfl in Complex with Guide RNA and Target DNA' CELL vol. 1, no. 65, 21 April 2016, pages 949 - 962, XP029530759
- NISHIMASU ET AL.: 'Crystal Structure of Cas9 in Complex with Guide RNA and Target DNA' CELL vol. 156, 27 February 2014, pages 935 - 949, XP055423859
- PENG ET AL.: 'High-throughput Screens in Mammalian Cells Using the CRISPR-Cas9 System' THE FEBS JOURNAL vol. 282, 16 March 2015, pages 2089 - 2096, XP055434292
- O'GEEN ET AL.: 'A Genome-wide Analysis of Cas9 Binding Specificity Using ChIP-seq and Targeted Sequence Capture' NUCLEIC ACIDS RESEARCH vol. 43, no. 6, 2015, pages 3389 - 3404, XP055434273
- VIDIGAL ET AL.: 'Rapid and Efficient One-step Generation of Paired gRNA CRISPR-Cas9 Libraries' NATURE COMMUNICATIONS vol. 6, no. 8083, 2015, pages 1 - 7, XP055340818
- KIM ET AL.: 'In vivo High-throughput Profiling of CRISPR-Cpfl Activity' NATURE METHODS vol. 14, no. 2, February 2017, pages 153 - 159, XP055434289

## Description

### TECHNICAL FIELD

The present invention relates to a method for evaluating the activity of an RNA-guided nuclease *in vivo,* specifically in cells, in a high-throughput manner, and more specifically, to a method for evaluating the activity of an RNA-guided nuclease from the indel frequency of a cell library including an isolated oligonucleotide that includes a guide RNA-encoding nucleotide sequence and a target nucleotide sequence.

### BACKGROUND ART

RNA-guided nuclease derived from prokaryotic immunity system of type II clustered regularly interspaced short palindromic repeats and CRISPR-associated protein (CRISPR-Cas) provides a means for genome editing. In particular, studies have been actively conducted on techniques for editing genomes of cells and organs using single-guide RNA (sgRNA) and Cas9 protein (Cell, 2014, 157:1262-1278). In particular, studies for the prediction of sgRNA activity are being carried out in the CRISPR-Cas9 system (ACS Synth Biol., 2017, Feb 10; Sci Rep, 2016, 6:30870, Nat Biotechnol, 34, 184-191), and studies are being conducted in China with regard to the use of CRISPR-Cas9 for the treatment of diseases by injecting cells, where genes encoding PD-1 are removed, by CRISPR-Cas9 (Nature, 2016, 539:479). Recently, Cpf1 protein (CRISPR derived from *Prevotella* and *Francisella* 1) was reported as another nuclease protein of class 2 CRISPR-Cas system (Cell, 2015, 163:759-771), and accordingly, the range of options for genome editing has been expanded. Cpf1 has various advantages in that it cuts in the form of a 5' protrusion, has a shorter length of guide RNA, and has a longer distance between the seed sequence and the cut position. However, there is a lack of studies on the characteristics of Cpf1 in humans and other eukaryotic cells, and particularly in relation to target and off-target effects.

Although the activity and accuracy are very important in the application of RNA-guided nuclease to genome editing, a lot of time and efforts are required for the confirmation of the activity of targets and off-targets of RNA-guided nuclease. The accuracy of prediction with regard to the activity of targets and off-targets *in silico* is limited (Nat Biotechnol, 2014, 32:1262-1267), and there is a need for the characterization of nuclease through comprehensive *in vivo* experiments on RNA-guided nuclease activity so as to develop computer prediction models. EP3009511A2 refers to novel Crispr enzymes and systems. Peng et al, FEBS Journal 282, 2015, 2089-2096 relates to high-throughput screens in mammalian cells using the Crispr-Cas9 system.

### DISCLOSURE OF THE INVENTION

### TECHNICAL PROBLEM

The present inventors have made efforts to develop a system that can evaluate the activity of RNA-guided nuclease *in vivo* conditions in a high-throughput manner, and as a result, have successfully developed a pair library system having guide RNA and a target sequence pair as major constituting elements thereby completing the present invention. The present invention is defined in the appended claims.

### TECHNICAL SOLUTION

An object of the present disclosure is to provide a method for evaluating the activity of an RNA-guided nuclease, which includes: (a) performing sequence analysis using DNA obtained from a cell library, where an RNA-guided nuclease is introduced, which includes an oligonucleotide, containing a guide RNA-encoding nucleotide sequence and a target nucleotide sequence which the guide RNA targets; and (b) detecting the indel frequency of each guide RNA-target sequence pair from the data obtained from the sequence analysis.

Another object of the present disclosure is to provide a cell library including at least two kinds of cells, in which each cell includes an oligonucleotide containing a guide RNA-encoding nucleotide sequence and a target nucleotide sequence which the guide RNA targets.

Still another object of the present disclosure is to provide a vector containing an isolated oligonucleotide, which includes a guide RNA-encoding nucleotide sequence and a target nucleotide sequence which the guide RNA targets; and a vector library.

Still another object of the present disclosure is to provide an isolated oligonucleotide, which includes a guide RNA-encoding nucleotide sequence and a target nucleotide sequence which the guide RNA targets; and an oligonucleotide library.

Still another object of the present disclosure is to provide a method for constructing the oligonucleotide library, which includes: (a) setting a target nucleotide sequence, which is to be targeted with an RNA-guided nuclease; (b) designing a guide RNA-encoding nucleotide sequence, which forms a base pair with a complementary strand of the set target nucleotide sequence; (c) designing an oligonucleotide, which contains the target nucleotide sequence and a guide RNA that targets the same; and (d) repeating steps (a) to (c) at least once.

Still another object of the present disclosure is to provide an isolated guide RNA, which includes a sequence that is able to form a base pair with a complementary strand of a target nucleotide sequence that is adjacent to a protospacer-adjacent motif (PAM) sequence, that is, TTTV or CTTA.

Still another object of the present disclosure is to provide a composition for genome editing, which contains the isolated guide RNA or a nucleic acid encoding the same.

Still another object of the present disclosure is to provide a system for genome editing in a mammalian cell, which includes the isolated guide RNA, or a nucleic acid encoding the same; and a Cpf1 protein or a nucleic acid encoding the same.

Still another object of the present disclosure is to provide a method for genome editing with Cpf1 in a mammalian cell, which includes sequentially or simultaneously introducing the guide RNA or a nucleic acid encoding the same; and a Cpf1 protein or a nucleic acid encoding the same, into an isolated mammalian cell.

## ADVANTAGEOUS EFFECTS

The method for evaluating the activity of an RNA-guided nuclease using the guide RNA-target sequence pair library of the present invention enables the evaluation of the activity of the RNA-guided nuclease in a cell (*in vivo*) in a high-throughput manner, and thus, the method can be very effectively utilized in all of the fields where the RNA-guided nuclease is applied.

## BRIEF DESCRIPTION OF THE DRAWINGS

FIG. 1 shows a schematic diagram illustrating oligonucleotides containing a pair of a target sequence and a guide RNA sequence for evaluating the activity of Cpf1.

FIG. 2 shows a schematic diagram illustrating the map of AsCpf1 lentivirus vector. Psi, packaging signal; RRE, rev response element, WPRE, posttranscriptional regulatory element of woodchuck hepatitis virus; U6, U6 pol III promoter; cPPT, central polypurine tract; EFS, elongation factor 1a short promoter; BlastR, blasticidin resistance gene.

FIG. 3 shows a schematic diagram illustrating the map of LbCpf1 lentivirus vector. Psi, packaging signal; RRE, rev response element, WPRE, posttranscriptional regulatory element of woodchuck hepatitis virus; U6, U6 pol III promoter; cPPT, central polypurine tract; EFS, elongation factor 1a short promoter; BlastR, blasticidin resistance gene.

FIG. 4 shows a schematic diagram of lentivirus vector, which includes backbone vector and a pair of a target sequence and guide RNA sequence, for the preparation of a plasmid library. Psi, packaging signal; RRE, rev response element; WPRE, posttranscriptional regulatory element of woodchuck hepatitis virus; cPPT, central polypurine tract; DR, direct repeat of Cpf1; GS, guide sequence of guide RNA; T, polyT; B, barcode; TS, target sequence; HS, homology sequence; EF1$\alpha$, elongation factor 1 $\alpha$ promoter; PuroR, puromycin resistance gene.

FIG. 5 shows a schematic diagram briefly illustrating the entire process of a high-throughput analysis system using the pair library of the present invention.

FIG. 6 shows the relative copy number of each pair in an oligonucleotide pool, a plasmid library, and a cell library.

FIG. 7 shows the copy number of each pair in a plasmid library and a cell library normalized to the copy number of each pair in an oligonucleotide pool and a plasmid library.

FIG. 8 shows the relative copy number of each pair in a plasmid library and a cell library in the order of the copy number in an oligonucleotide pool.

FIG. 9 shows the relative copy number of each pair in a cell library in the order of the copy number in a plasmid library.

FIG. 10 shows the correlation between the pair copy number of a plasmid library and an oligonucleotide pool by evaluation through deep sequencing.

FIG. 11 shows the correlation between the pair copy number of a cell library and an oligonucleotide pool by evaluation through deep sequencing.

FIG. 12 shows the correlation between the pair copy number of a cell library and a plasmid library by evaluation through deep sequencing.

FIG. 13 shows a schematic diagram of the process for confirming the PAM sequences of AsCpf1 and LbCpf1.

FIG. 14 shows the indel frequency according to the potential PAM sequence of AsCpf1. The ANNNN sequence was experimented as a potential PAM sequence. For the purpose of brief representation, "A" was omitted.

FIG. 15 shows the indel frequency with regard to 4 kinds of TTTN PAM sequences of AsCpf1. Each error bar represents standard error of mean (SEM). *P < 0.05, **P < 0.01, ***P < 0.001.

FIG. 16 shows the indel frequency according to the potential PAM sequence of LbCpf1. The ANNNN sequence was experimented as a potential PAM sequence. For the purpose of brief representation, "A" was omitted.

FIG. 17 shows the indel frequency with regard to 4 kinds of TTTN PAM sequences of LbCpf1. Each error bar represents standard error of mean (SEM). *P < 0.05, **P < 0.01, ***P < 0.001.

FIG. 18 shows graphs illustrating the comparison results of PAM sequences by *in vivo* and *in vitro* analysis, in which a and b represent the results of *in vitro* analysis of the PAM sequence of (a)AsCpf1 and the PAM sequence of (b)LbCpf1, respectively; and c and d represent the results of *in vivo* analysis of the correlation between indel frequency and potential PAM sequences of (c)AsCpf1 and (d)LbCpf1, respectively.

FIG. 19 shows the indel frequency with regard to 4 kinds of NTTTA PAM sequences of AsCpf1 (left) and LbCpf1 (right). Each error bar represents standard error of mean (SEM). *P< 0.05 ANOVA followed by Tukey's post hoc test.

FIG. 20 shows the comparison results with regard to the order of indel frequency between AsCpf1 and SpCas9 using forward or reverse target sequences. The correlation of the order of indel frequency with regard to forward target sequence (left) and reverse target sequence (right) of SpCas9 and AsCpf1 are shown. The 5'-GGG-3' and 5'-TTTA-3' sequences indicated in red were used as PAM sequences for SpCas9 and AsCpf1 target sequences, respectively. The order of activity with regard to the SpCas9 target sequence was referred to the literature (Nat Biotechnol, 2014, 32:1262-1267).

FIG. 21 shows a graph illustrating the nucleotide preference at each position of AsCpf1 target sequence with regard to the guide RNA with top 20% with high activity. The P-values were calculated by binomial distribution with baseline probability of 0.2 using 1,251 pairs of the guide RNA and target sequences from the literature (Nat Biotechnol, 2014, 32:1262-1267).

FIG. 22 shows a graph illustrating the relationship between GC contents of target sequences and indels observed, in which a, b, and c represent each group having statistically different indel frequency ($P > 0.05$), and each error bar represents standard error of mean (SEM). *$P < 0.05$, **$P < 0.01$, ***$P < 0.001$.

FIG. 23 shows a graph illustrating the average indel frequency according to time after delivery of Cpf1-expressing lentivirus vector in a cell library, in which each error bar represents standard error of mean (SEM). **$P < 0.01$, ***$P < 0.001$.

FIG. 24 shows the indel frequency at each target sequence on day 3, 5, and 31 after transduction of Cpf1-expressing lentivirus into a cell library.

FIG. 25 shows a schematic diagram illustrating experimental designs for the analysis of indel frequency according to nucleotide mismatch in guide RNA-encoding sequences and target sequences.

FIG. 26 shows the indel frequency according to the position of nucleotide mismatch in off-target sequences.

FIG. 27 shows a graph illustrating the indel frequency according to the guide RNA length in an off-target sequence with one nucleotide mismatch and an on-target sequence, which is normalized into indel frequency in an on-target sequence.

FIG. 28 shows a graph illustrating relative indel frequency according to the number of nucleotide mismatch in an off-target sequence.

FIG. 29 shows graphs illustrating the effect of the number of mismatch nucleotides according to a region within the on-target sequence, in an off-target indel frequency induced by Cpf1. The off-target indel frequency was normalized to indel frequency in an on-target sequence.

FIG. 30 shows graphs illustrating the effect of multiple-mismatch of nucleotides of a region within the on-target sequence, in an off-target indel frequency induced by Cpf1.

FIG. 31 shows a graph illustrating the effect of mismatch types with regard to the relative indel frequency in a seed region of an off-target sequence. **$P < 0.01$.

FIG. 32 shows a graph illustrating the effect of mismatch types with regard to the relative indel frequency in a trunk region of an off-target sequence. **$P < 0.01$.

FIG. 33 shows a graph illustrating the effect of mismatch types with regard to the relative indel frequency in a promiscuous region of an off-target sequence. **$P < 0.01$.

FIG. 34 shows an illustration illustrating the concept of a high-throughput evaluation system *in vivo* using the pair library of the present invention. Conventionally, RNA-guided nuclease had been measured by an individual and difficult method (a small-scale system, top). The present invention enables high-throughput evaluation (a plant system, bottom), and thus provides a new method for easy evaluation of RNA-guided nuclease on a large-scale.

FIG. 35 shows a schematic diagram illustrating oligonucleotides for evaluation of Cas9 activity, containing a pair of a target sequence and a guide RNA sequence.

FIG. 36 shows a schematic diagram illustrating the map of Cas9 lentivirus vector.

FIG. 37 shows graphs illustrating the results of guide RNA activity measured using a guide RNA-target sequence pair library; and FIG. 38 shows a graph illustrating the results of guide RNA activity measured using the pair library of the present invention.

FIG. 39 shows a schematic diagram illustrating the interaction between crRNA nuclease and the Thr16 in the Cpf1 WED domain at position 1. The hydroxyl side chain of the Thr16 residue within the WED domain exhibits a polar interaction with the $N_2$ of the guanine base (a blue dotted line within the red circle). The side chains of a different nucleobase (*e.g.,* $O_2$ of thymine and uracil) can exhibit a polar interaction similar to that of the Thr16 residue. However, since the above moieties are not present in adenine, the side chains form an unstable binding with thymine present at position 1 of a target DNA strand located adjacent to the PAM motif, in the crRNA adenine ribonucleobase. There exists a complementary interaction between the crRNA ribonucleotide (guanine is indicated) and a target sequence nucleotide (cytocine at position 1 is indicated). The diagram was prepared based on the data of PDB 5B43.

FIG. 40 shows a graph illustrating the correlation between indel frequencies in an endogenous target position and a corresponding introduced synthetic sequence, in which a scatter plot for the 82 analyzed endogenous regions is shown.

FIG. 41 shows a graph illustrating the correlation between indel frequencies in an endogenous target position and a corresponding introduced synthetic sequence, in which a scatter plot for top 25% DNase-sensitive regions among the 82 regions is shown.

FIG. 42 shows graphs illustrating the correlation between indel frequencies in an endogenous target position and an introduced sequence, in which scatter plots for each of the DNase-sensitive regions for (a) top 25% to 50% (b) top 50% to 75%, and (c) 75% to 100% are shown.

FIG. 43 shows a graph illustrating the correlation between indel frequencies in a biological replicate. Two different libraries (library A and library B) were prepared by independent lentivirus production and transduction. The two libraries were transfected with Cpf1-encoding plasmids, and after 4 days, the indel frequency was analyzed in the cell libraries.

FIG. 44 shows a graph illustrating the correlation between indel frequencies after the delivery of Cpf1 by two different delivery methods. The cell library was transfected with a Cpf1 plasmid or transduced with a Cpf1 lentivirus vector. After 4 days (transfection) or 5 days (transduction), the indel frequency of the cell library was analyzed.

FIG. 45 shows graphs illustrating the results of comparison of costs between the conventional method and the high-throughput manner evaluation method for evaluating Cpf1 activity in a target sequence. The costs of material(left) and labor(right) were compared. The cost was indicated in USD and the labor unit was indicated as the amount of maximum work that a skilled person can be performed. In a case where there was a break over one hour (*e.g.*, cultivation time), it was not calculated as labor.

## MODE FOR CARRYING OUT THE INVENTION

Programmable nucleases are widely used for genome editing of cells and individual subjects, and the technology employing the Programmable nucleases is a very useful technology that can be used for various purposes in life sciences, biotechnology, and medicine fields. In particular, recently, Cas9 which is RNA-guided nuclease derived from prokaryotic immunity system of type II CRISPR/Cas (clustered regularly interspaced repeat/CRISPR-associated), and Cpfl, etc., are attracting attention as its usefulness. However, for the utilization of the RNA-guided nucleases, it is important to design guide RNA with regard to its target sequence of these nucleases because on-target activity and off-target activity may vary depending on the sequence possessed by the guide RNA. In this regard, the present inventors have attempted to develop a method for evaluating the activity of RNA-guided nucleases *in vivo* in a high-throughput manner.

Herein below, exemplary embodiments of the present invention will be described in detail. Meanwhile, each of the explanations and exemplary embodiments disclosed herein can be applied to respective other explanations and exemplary embodiments. That is, all of the combinations of various factors disclosed herein belong to the scope of the present disclosure. Furthermore, the scope of the present invention should not be limited by the specific disclosure provided herein below.

To achieve the above objects, an aspect of the present disclosure provides a method for evaluating the activity of an RNA-guided nuclease, which includes: (a) performing sequence analysis using DNA obtained from a cell library, where an RNA-guided nuclease is introduced, including an oligonucleotide that includes a guide RNA-encoding nucleotide sequence and a target nucleotide sequence which the guide RNA targets; and (b) detecting the indel frequency of each guide RNA-target sequence pair from the data obtained from the sequence analysis. The present inventors have named the above method as "guide RNA-target sequence pair library analysis", which refers to a method for evaluating the activity of an RNA-guided nuclease using a cell library where guide RNA-encoding nucleotide sequences and target nucleotide sequences are introduced thereinto as a pair.

In particular, the present inventors have confirmed that the activity of RNA-guided nucleases measured using the pair library has high correlation with the activity of RNA-guided nucleases acting on endogenous genes in a cell, and thereby, they have confirmed that the method for the evaluation of the RNA-guided nucleases of the present invention can not only be useful *in vitro* but also *in vivo.*

The technology of genome editing/gene editing is a technology that can introduce a target-directed modification to a nucleotide sequence of genome of animal/plant cells including humans, and it can also do knock-out or knock-in a particular gene or introduce modification to a non-coding DNA sequence which does not produce a protein. The method of the present invention can analyze on-target activity and off-target activity of RNA-guided nucleases used in the above technology of genome editing/gene editing in a high-throughput manner, and this can be effectively used for the development of a RNA-guided nuclease which only specifically acts on a target position.

As used herein, the term "RNA-guided nuclease" refers to a nuclease which is able to recognize a particular position on a target genome and cleave the same, and in particular, a nuclease having specificity by guide RNA. The RNA-guided nuclease may include Cas9 protein derived from CRISPR (*i.e.*, a microorganism immune system), specifically CRISPR-associated protein 9 (Cas9), and Cpfl, etc., but RNA-guided nuclease is not limited thereto.

The RNA-guided nuclease may recognize a particular nucleotide sequence in the genome of animal/plant cells including human cells and cause a double strand break (DSB), and may form a nick (nicklase activity). The double strand break includes producing both blunt ends and cohesive ends by cleaving double strands of DNA. DSB is efficiently repaired by a mechanism of homologous recombination or non-homologous end-joining (NHEJ) in a cell, and the modification desired by a researcher may be introduced to a target site during this process. The RNA-guided nuclease may be artificial or manipulated non-naturally occurring.

As used herein, the term, "Cas protein" is a major protein constituting element of CRISPR/Cas system, and it is a protein that can act as an activated endonuclease or nickase. The Cas protein may form a complex with CRISPR RNA (crRNA) and trans-activating crRNA (tracrRNA), and thereby exhibit their activity.

The information on Cas protein or genes thereof may be obtained from a known database such as GenBank of National Center for Biotechnology Information (NCBI). Specifically, the Cas protein may be Cas9 protein. Additionally, the Cas protein may be derived from a microorganism of the genus *Streptococcus,* the genus *Neisseria,* the genus *Pasteurella,* the genus *Francisella,* and the genus *Campylobacter,* specifically, it derived from a microorganism of *Streptococcus pyogenes,* and more specifically Cas9 protein may be Cas9 protein derived from a microorganism of *Streptococcus pyogenes,* but is not limited thereto. However, the present invention is not limited to the examples described above, as long as it has the activity of the RNA-guided nuclease described above. In the present invention, the Cas protein may be a recombinant protein.

As used herein, the term "Cpf1" refers to a new nuclease of the CRISPR system, which is distinguished from the CRISPR/Cas system, and it was reported only recently (Cell, 2015, 163(3): 759-71). The Cpf1 is characterized in that it is a nuclease operated by single RNA, does not require tracrRNA, and has a relatively small size. Additionally, it is known that Cpf1 utilizes a thymine-rich protospacer-adjacent motif (PAM) sequence and generates a cohesive end by cleaving the double strand of DNA. The Cpf1 may be derived from a microorganism of the genus *Candidatus Paceibacter,* the genus *Lachnospira,* the genus *Butyrivibrio,* the genus *Peregrinibacteria,* the genus *Acidominococcus,* the genus *Porphyromonas,* the genus *Prevotella,* the genus *Francisella,* the genus *Candidatus Methanoplasma,* or the genus *Eubacterium,* but is not limited thereto. However, the present invention is not limited to the examples described above, as long as it has the activity of the RNA-guided nuclease described above. In the present invention, the Cpf1 protein may be a recombinant protein.

The above term "recombination", for example, when it is used while mentioning cells, nucleic acids, proteins or vectors, *etc*., it means introduction of a heterologous nucleic acid or protein, or a change in native nucleic acid or protein, or a cell, nucleic acid, protein, or vector which is modified by a cell derived from a modified cell. Accordingly, for example, recombinant Cas9 or recombinant Cpf1 protein may be prepared by reconstituting the sequence encoding Cas9 protein or Cpf1 protein using the human codon table.

The Cas9 protein or Cpf1 protein may be in the form where the proteins are able to act in the nucleus, and may be in the form where they can easily be introduced into a cell. For example, the Cas9 protein or Cpf1 protein may be linked to a cell penetrating peptide or protein transduction domain. The protein transduction domain may be poly-arginine or a HIV-derived TAT protein, but is not limited thereto. With regard to the cell penetrating peptide or protein transduction domain, there are many kinds disclosed in the art, and thus those skilled in the art can apply various kinds, not limited to the above examples, to the present invention.

Additionally, any nucleic acid that encoding the Cas9 protein or Cpf1 protein can further include a nuclear localization signal (NLS) sequence. Accordingly, any expression cassette including nucleic acid encoding Cas9 protein or Cpf1 protein can include an NLS sequence, in addition to the control sequence (*e.g.*, a promoter sequence, *etc.*) for the expression of the Cas9 protein or Cpf1 protein, but the sequence to be included is not limited thereto.

The Cas9 protein or Cpf1 protein may be linked to a tag which is useful for isolation and/or purification. For example, a small peptide tag (*e.g.*, His tag, Flag tag, S tag, *etc.*), or a glutathione S-transferase (GST) tag, a maltose-binding protein (MBP) tag, *etc.* may be linked according to the purposes, but the tags are not limited thereto.

The present invention provides a method for analyzing the characteristics of the RNA-guided nuclease. Hereinafter, each step of the method will be described in detail. Meanwhile, as described above, it is apparent that the definitions and aspects of the terms described above are also applied to the following.

Step (a) is a step where deep sequencing is carried out using the DNA obtained from a cell library, which includes isolated oligonucleotide including guide RNA-encoding nucleotide sequences and target nucleotide sequences. The step is which data necessary for analysis are obtained from the cell population where various insertions and deletions (indels) occurred by the activity of on-target and off-target through acting the RNA-guided nuclease on various guide RNAs and target sequences.

Specifically, step (a) may be carried out, which includes:

(i) preparing an oligonucleotide library including a guide RNA-encoding nucleotide sequence and a target nucleotide sequence (*i.e.*, a pair of a guide RNA sequence and a target nucleotide sequence),
(ii) preparing a vector library, specifically a virus vector library, using the oligonucleotide library and specifically preparing a vector library by preparing a vector for each oligonucleotide of the oligonucleotide library,
(iii) preparing a cell library using the vector library, specifically a virus vector library, and specifically, constructing a cell library by introducing each vector of the vector library into a cell, and
(iv) conducting sequence analysis (*e.g.*, deep sequencing) using the DNA obtained from the cell library.

The cell library, where the DNA in step (iv) is obtained, may be one where RNA-guided nucleases are introduced into the cell library constructed in step (iii), and the activity of RNA-guided nuclease is induced by culturing the cells.

As used herein, the term "library" refers to a pool or population where two or more kinds of the same kind of material with different characteristics are included. Accordingly, the oligonucleotide library may be a pool including two or more kinds of oligonucleotides in which include a different nucleotide sequence (*e.g.*, a guide RNA sequence, a PAM sequence) and/or a different target sequence; and the vector library (*e.g.*, a virus vector library) may be a pool including two or more kinds of vectors in which include a different sequence or constituting element, for example, it may be a pool of vectors for each oligonucleotide of the oligonucleotide library, it may be a pool including two or more vectors which have a difference in the oligonucleotide constituting the corresponding vector. The cell library may be a pool of two or more kinds of cells with different characteristics, specifically a pool of cells including each different oligonucleotide for the purposes of the present invention, for example, a pool of cells including each different number of the introduced vectors and/or each different kinds of the introduced vectors, specifically cells including different kinds of the vectors. Since the present invention aims at evaluating the activity of RNA-guided nucleases using a cell library in high-throughput manner, the kinds of oligonucleotides, vectors (*e.g.*, a virus vector), and cells of each library may be two or more kinds, and the upper limit of each library is not limited as long as the evaluation method is operated normally.

As used herein, the term "oligonucleotide" refers to a material where several to several hundred nucleotides are linked by phosphodiester bonds, and for the purposes of the present invention, the oligonucleotide may be double helix DNA. The oligonucleotide used in the present invention may have a length of 20 bp to 300 bp, specifically, 50 bp to 200 bp, and more specifically, 100 bp to 180 bp. In the present invention, the oligonucleotide includes a guide RNA-encoding nucleotide sequence and a target nucleotide sequence. Additionally, the oligonucleotide may include an additional sequence to which a primer can be bound for PCR amplification.

Specifically, in a single oligonucleotide, a guide RNA may be cis-acting on a target nucleotide sequence present adjacent to the same. That is, the guide RNA may be one which is designed so as to confirm whether the adjacent target nucleotide sequence has been cleaved.

The oligonucleotide may be introduced into a cell and integrated into the chromosome.

As used herein, the term "guide RNA" refers to a target DNA-specific RNA, and it may complementarily bind to all or part of a target sequence such that an RNA-guided nuclease cleaves the target sequence.

Conventionally, the guide RNA refers to a dual RNA which includes two RNAs (*i.e.*, CRISPR RNA (crRNA) and trans-activating crRNA (tracrRNA)) as constituting elements; or a form which includes a first region including a complementary sequence in all or part of a sequence in the target DNA and a second region including a sequence interacting with an RNA-guided nuclease, but any form where the RNA-guided nuclease can have activity in a target sequence may be used. In an embodiment, when the guide RNA is applied to Cpf1, the guide RNA may be crRNA, whereas when the guide RNA is applied to Cas, in particular Cas9, the guide RNA may be in the form of a dual RNA including crRNA and tracrRNA as constituting elements, or in the form of a single-chain guide RNA (sgRNA) where the major parts of crRNA and tracrRNA are fused. The sgRNA may include a part which has a sequence complementary to a sequence in the target DNA (this is called spacer region, target DNA recognition sequence, base pairing region, etc.) and a hairpin structure for the binding of Cas (especially Cas9 protein). More specifically, the sgRNA may include a part which has a sequence complementary to all or part of a sequence in the target DNA, a hairpin structure for the binding of Cas (especially Cas9 protein), and a terminator sequence. The structure described above may be one which is present sequentially in the 5' to 3' direction. However, the structure may not be limited thereto, but guide RNA in the form of any structure may be used in the present invention, as long as the guide RNA includes the major part of crRNA or all or part complementary to the target DNA.

The guide RNA, specifically crRNA or sgRNA, may include a sequence all or part of which is complementary to the sequence of the target DNA, an upstream part of crRNA or sgRNA, and specifically at least one additional nucleotide to the 5' terminus of sgRNA or crRNA. The additional nucleotide may be guanine (G), but the nucleotide is not limited thereto.

Additionally, the guide RNA may include a scaffold sequence which helps the attachment of an RNA-guided nuclease.

As used herein, the term, "target nucleotide sequence or target sequence" refers to a nucleotide sequence which an RNA-guided nuclease is expected to target, and in the present invention, it further includes a target sequence to be analyzed by the method of guide RNA-a target nucleotide sequence pair library analysis of the present invention. In the present invention, a guide RNA and a target sequence are present in the form of a pair in each oligonucleotide and vector that constitutes the oligonucleotide library and the vector library, respectively. Therefore, the guide RNA present in one oligonucleotide or vector corresponds to its target sequence.

In the present invention, on-target activity (or on-target effect)/off-target activity (or off-target effect) and the target nucleotide sequence should be understood as completely distinct meanings.

The term "on-target activity" refers to activity, with regard to a sequence which is perfectly complementary to all or part of the sequence of guide RNA, which RNA-guided nuclease cleaves the sequence and further causes an indel on the cleaved region.

The term "off-target activity" refers to activity, with regard to a sequence which is not perfectly complementary to all or part of the sequence of guide RNA but part of the sequence mismatches, which RNA-guided nuclease cleaves the sequence and further causes an indel on the cleaved region. That is, the terms of "on-target activity" and "off-target activity" relate to a concept which is determined whether the sequence cleaved by the RNA-guided nuclease is perfectly complementary to all or part of the guide RNA sequence.

Meanwhile, the term "target sequence" as used herein refers to a sequence to be analyzed as to whether the activity of the RNA-guided nuclease occurred by the guide RNA present in the form of a pair is exhibited. That is, the target sequence can be determined by an operator during the process of design or preparation of each oligonucleotide that constitutes the oligonucleotide library of the present invention, and the operator can select according to the purpose of the embodiment in the designing step, the sequence from which on-target activity is expected and the sequence from which off-target activity is expected, with regard to the pair guide RNA and design the target sequence. The target sequence may include a protospacer-adjacent motif (PAM) sequence, which the RNA-guided nuclease recognizes, but is not limited thereto.

The design of an oligonucleotide may be freely conducted by those skilled in the art under the purpose of evaluating the activity of RNA-guided nucleases. For example, a pair may be comprised of sequences having on-target activity with regard to a particular guide RNA sequence, and also, a pair may be comprised of sequences having off-target activity with regard to the guide RNA sequence. For example, it is designed to a sequence which is perfectly complementary to guide RNA sequence, specifically the crRNA sequence, or it is designed to a sequence which is partially complementary such that part of the nucleotides mismatch.

Additionally, those skilled in the art may include additional constituting elements to oligonucleotides so as to perform the analysis of the guide RNA-target sequence pair library of the present invention. For example, the oligonucleotide may further include at least one selected from the group consisting of a direct repeat sequence, a poly T sequence, a barcode sequence, a constant region sequence, a promoter sequence, and a scaffold sequence, but the constituting elements are not limited thereto.

As described above, the oligonucleotide may be one consisting of a sequence of 100 to 200 nucleotides, but the oligonucleotide is not limited thereto, and may be appropriately adjusted by those skilled in the art according to the kinds, analysis purposes, etc. of the RNA-guided nuclease to be used.

Meanwhile, the oligonucleotide may be designed to include a target sequence and a guide RNA-encoding sequence in the 5' to 3' direction, and in contrast, may be designed to include guide RNA sequence and a target sequence in the 5' to 3' direction.

For example, the oligonucleotide may include a target sequence and a guide RNA-encoding sequence, specifically a target sequence, a barcode sequence, and a guide RNA-encoding sequence, and may be constructed in the following order, but the order is not particularly limited thereto.

The oligonucleotide may include a guide RNA-encoding sequence, a barcode sequence, and a target sequence in the 5' to 3' direction; specifically a guide RNA-encoding sequence, a barcode sequence, a PAM sequence, and a target sequence; a guide RNA-encoding sequence, a barcode sequence, target sequence, and a PAM sequence; a guide RNA-encoding sequence, a poly T sequence, a barcode sequence, a PAM sequence, and a target sequence; and a guide RNA-encoding sequence, a poly T sequence, a barcode sequence, a target sequence, and a PAM sequence.

More specifically, the oligonucleotide may include a direct repeat sequence, a guide RNA-encoding sequence, a barcode sequence, a PAM sequence, and a target sequence; a direct repeat sequence, a guide RNA-encoding sequence, a barcode sequence, a target sequence, and a PAM sequence; a direct repeat sequence, a guide RNA-encoding sequence, a barcode sequence, a PAM sequence, a target sequence, and a constant sequence; a direct repeat sequence, a guide RNA-encoding sequence, a barcode sequence, a target sequence, a PAM sequence, and a constant sequence, but the sequences are not particularly limited thereto.

Additionally, the oligonucleotide may further include a scaffold sequence which is adjacent to a guide RNA-encoding sequence and helps the binding of an RNA-guided nuclease.

For example, the oligonucleotide may include a scaffold sequence, a guide RNA-encoding sequence, a barcode sequence, a PAM sequence, and a target sequence, but the constituting elements are not particularly limited thereto.

Additionally, the oligonucleotide may include a promoter sequence at the 5' end region for expression. In an embodiment of the present invention, a U6 promoter was used.

The oligonucleotide may include, in the 5' to 3' direction, a target sequence, a barcode sequence, and a guide RNA-encoding sequence; specifically may include a target sequence, a PAM sequence, a barcode sequence, and a guide RNA-encoding sequence; may include a PAM sequence, a target sequence, a barcode sequence, and a guide RNA-encoding sequence; may include a target sequence, a PAM sequence, a barcode sequence, a poly T sequence, and a guide RNA-encoding sequence; may include a PAM sequence, a target sequence, a barcode sequence, a poly T sequence, and a guide RNA-encoding sequence; more specifically may include a target sequence, a PAM sequence, a barcode sequence, a guide RNA-encoding sequence, and a direct repeat sequence; may include a PAM sequence, a target sequence, a barcode sequence, a guide RNA-encoding sequence, and a direct repeat sequence; may include a target sequence, a PAM sequence, a barcode sequence, a poly T sequence, a guide RNA-encoding sequence, and a direct repeat sequence; may include a PAM sequence, a target sequence, a barcode sequence, a poly T sequence, a guide RNA-encoding sequence, and a direct repeat sequence; may include a constant sequence, a target sequence, a PAM sequence, a barcode sequence, a poly T sequence, a guide RNA-encoding sequence, and a direct repeat sequence; may include a constant sequence, a PAM sequence, a target sequence, a barcode sequence, a poly T sequence, a guide RNA-encoding sequence, and a direct repeat sequence, but the constituting elements are not particularly limited thereto.

Additionally, the oligonucleotide may further include a scaffold sequence which is adjacent to a guide RNA-encoding sequence and helps the binding of the RNA-guided nuclease.

For example, the oligonucleotide may include a target sequence, a PAM sequence, a barcode sequence, a guide RNA-encoding sequence, and a scaffold sequence, but the constituting elements are not particularly limited thereto. Additionally, the oligonucleotide may include a promoter sequence at the 5' end region for expression.

Additionally, as described above, the oligonucleotide may further include a primer attachment sequence at the 5' end and 3' end for PCR amplification in addition to the constituting elements described above, but the constituting elements are not particularly limited thereto.

The target sequence may have a length of 10 bp to 100 bp, specifically 20 bp to 50 bp, more specifically 23 bp to 34 bp, but the length is not particularly limited thereto.

Additionally, the guide RNA-encoding sequence may have a length of 10 bp to 100 bp, specifically 15 bp to 50 bp, and more specifically 20 bp to 30 bp, but the length is not particularly limited thereto.

Additionally, the barcode sequence refers to a nucleotide sequence for the recognition of each oligonucleotide. In the present disclosure, the barcode sequence may not include two or more of repeated nucleotides (*i.e.*, AA, TT, CC, and GG), but the barcode sequence is not particularly limited as long as it is designed so as to recognize each oligonucleotide. In multiple oligonucleotides, the barcode sequence may be designed such that at least two nucleotides are different so as to distinguish each oligonucleotide. The barcode sequence may have a length of 5 bp to 50 bp, but the length is not particularly limited thereto.

In a specific embodiment of the present invention, with regard to *Acidaminococcus-derived* Cpf1 (AsCpf1) and *Lachnospiraceae-derived* Cpf1 (LbCpf1), pair oligonucleotides were synthesized from 8,327 species and 3,634 species, respectively, by varying the guide RNA and/or target sequence, and thereby an oligonucleotide library including total 11,961 species of guide RNA-target sequence pair oligonucleotide were prepared. Each oligonucleotide constituting the oligonucleotide library had a total length of 122 bp to 130 bp nucleotides, and includes a mutually-different pair of a guide RNA-encoding sequence and a target nucleotide sequence, and the specific constitution is shown in FIG. 1.

Additionally, in another embodiment of the present invention, with regard to *Streptococcus pyogenes-derived* Cas9 (SpCas9), 89,592 oligonucleotides were synthesized and thereby an oligonucleotide library including oligonucleotides of guide RNA-target sequence pairs were prepared. The oligonucleotide had a total length of 120 nucleotides and includes a guide RNA-encoding sequence(guide sequence) and a target sequence (FIG. 35).

Next, a vector library (*e.g.*, a virus vector) can be prepared using the oligonucleotide library.

One of the advantages of the method for evaluating the activity of RNA-guided nucleases using the guide RNA-target sequence pair of the present invention lies in that the pair is introduced into a cell using a virus. Since the guide RNA corresponding to a target sequence is introduced into a cell in the form of a pair, the effects that may occur due to the deviation in copy number in an oligonucleotide library, vector library, and cell library can be minimized, and can be integrated into the genomic DNA through a virus, it is possible to perform analysis of the activity of on-target and off-target according to time unlike the analysis method by transient expression, and furthermore, the effects caused by epigenetic factors can be relatively minimized. When the vector is a virus, a virus library is introduced into a cell and virus can be produced therefrom and obtained, and cells can be infected using the same. This process can be appropriately performed by those skilled in the art using a method known in the art.

In the present disclosure, the vector may include oligonucleotides where each oligonucleotide includes a guide RNA-encoding nucleotide sequence and a target nucleotide sequence. The vector may be a virus vector or plasmid vector, and the virus vector may specifically be a lentivirus vector, retrovirus vector, etc., but the vectors are not limited thereto, and those skilled in the art can freely use any known vector that can achieve the objects of the present invention.

The vector refers to a mediator that can deliver the oligonucleotide to a cell, for example, a genetic construct. Specifically, when the vector is present in cells of an individual subject, it may include an insert, that is, an insert where an essential control element is operably linked thereto such that an oligonucleotide can be expressed.

The vector may be prepared and purified using a standard recombinant DNA technology. The kinds of the vector may not be particularly limited as long as the vector can act in the target cells (*e.g.*, eukaryotes, prokaryotes, etc.). The vector may include a promoter, an initiation codon, and a termination codon terminator. In addition, the vector may appropriately include DNA encoding a signal peptide, and/or an enhancer sequence, and/or an untranslated region in the 5' and 3' sites of a gene, and/or a selective marker region, and/or a replicable unit, *etc.*

In a specific embodiment of the present invention, a lentivirus vector library was prepared by cloning each oligonucleotide of the oligonucleotide library into the lentivirus vector (FIGS. 4 and 36), and the same was expressed in cells and thereby the virus was obtained.

The next step is to prepare a cell library by introducing the vector into a target cell. Specifically, the method of delivering the vector to a cell for the preparation of a library can be achieved by various methods known in the art. These methods may include, for example, calcium phosphate-DNA co-precipitation method, a DEAE-dextran-mediated transfection method, polybrene-mediated transfection method, electroporation, microinjection, liposome fusion method, Lipofectamine® and protoplast fusion method, *etc.* which are known in the art. Additionally, when a virus vector is used, the target product (*i.e.*, the vector) may be delivered using virus particles having the infection as a means. Additionally, the vector may be introduced into a cell by gene bombardment, *etc.*

The introduced vector may be present as a vector itself in a cell or may be integrated into the chromosome, but the vector state is not particularly limited thereto.

The cell library prepared in the present invention refers to a cell population in which oligonucleotides containing a guide RNA-target sequence are introduced. In particular, each cell may be those where the vector was introduced, and specifically the vector was introduced such that the kinds and/or number of the virus were different. However, the analysis method of the present invention is performed using all of the cell library, and the guide RNA-encoding nucleotide sequence and the target sequence are introduced in the form of a pair, and thus the method is not significantly affected by efficiency of cell infection, deviation in the copy number of oligonucleotides, *etc.* (FIGS. 6 to 12) and each pair-dependent interpretation is possible.

An RNA-guided nuclease may be further introduced so as to induce indel to the constructed cell library.

The nuclease may differently exhibit the degree of its activity according to the kinds and/or number of the guide RNA-target sequence pair. The RNA-guided nuclease may be delivered to a cell through a plasmid vector or virus vector, and may be delivered to a cell as an RNA-guided nuclease protein itself, but the introduction method is not particularly limited as long as the RNA-guided nuclease can exhibit its activity in cells. In an embodiment, the RNA-guided nuclease may be delivered in a form where it is linked to a protein transduction domain (*e.g.*, a Cas protein, a Cpf1 protein, *etc.*), but the form is not limited thereto. As the protein transduction domain, various kinds known in the art may be used, and poly-arginine or a HIV-derived TAT protein may be used as described above, but is not particularly limited thereto.

Additionally, the kinds of cells into which the vector can be introduced may be appropriately selected by those skilled in the art according to the kinds of the vector and/or kinds of the target cells, for example, bacterial cells (*e.g.*, *E. coli, Streptomyces, Salmonella typhimurium, etc.*); yeast cells; fungal cells (*e.g., Pichia pastoris, etc.*); insect cells (*e.g.*, Drosophila, *Spodoptera frugiperda* (Sf9), *etc.*); animal cells (*e.g.*, Chinese hamster ovary cells (CHO), SP2/0 (mouse myeloma), human lymphoblastoid, COS, NSO (mouse myeloma), 293T, bow melanoma cells, HT-1080, baby hamster kidney cells (BHK), human embryonic kidney cells (HEK), PERC.6 (human retinal cells), *etc.*); or plant cells.

In the cell library, the activity of nuclease may appear by the introduced guide RNA-target sequence pair oligonucleotide and an RNA-guided nuclease. That is, with regard to the introduced target sequence, a DNA cleavage by an RNA-guided nuclease may occur, and an indel may occur accordingly. As used herein, the term "indel" collectively refers to modification where, in a nucleotide sequence of DNA, part of the nucleotide is inserted or deleted. The indel may be one which, when an RNA-guided nuclease cleaves doublestranded DNA as described above, is introduced to a target sequence during the process while repair is conducted by a mechanism of homologous recombination or non-homologous end-joining (NHEJ).

Additionally, the method of the present disclosure may include obtaining a DNA sequence from the cell where the activity of the introduced RNA-guided nuclease is exhibited. The obtaining of DNA may be carried out using various DNA isolation methods known in the art.

Since it is expected that each cell constituting the cell library undergoes the occurrence of an indel in an introduced target sequence, the relevant data can be obtained by performing sequence analysis for the nucleotides of the target sequence (*e.g.*, deep sequencing or RNA-seq).

Since the analysis method of the present invention using a guide RNA-target sequence pair library is performed *in vivo,* reliable analysis results can be obtained without artifacts compared to other analysis methods *in vitro.*

Accordingly, step (b) is a step of obtaining the indel frequency of each guide RNA-target sequence pair from the data obtained through the sequence analysis.

As described above, each indel may occur in a manner dependent on each guide RNA-target sequence pair, and accordingly, the indel frequency may be evaluated as the degree of activity of RNA-guided nuclease by the guide RNA-target sequence pair.

Each pair can be distinguished by inserting a particular sequence, to each oligonucleotide constituting the oligonucleotide library, which is able to distinguish the oligonucleotide, and thus it is possible to perform analysis by classifying the data based on the distinguished sequence in the step of data analysis. In an embodiment of the present invention, each oligonucleotide was prepared to include a barcode sequence which does not include any repeat of two or more nucleotides (*i.e.*, AA, CC, TT, and GG) and include at least two mutually-different nucleotides.

The pair library of the present invention provides a method for evaluating the activity of RNA-guided nucleases with improved accuracy and predictability by having high correlation with the activity of the RNA-guided nucleases that act on the endogenous genes *in vivo.*

In a specific embodiment of the present invention, it was confirmed that the activity of programmable nucleases measured through libraries were highly correlated with the activity of the programmable nucleases which actually act on endogenous genes *in vivo.*

Additionally, the pair library of the present invention has an advantage in that it enables the evaluation of the activity of RNA-guided nucleases with high accuracy.

Specifically, in a specific embodiment of the present invention, the accuracy of a pair library was evaluated by comparing the activity ranking of the guide RNAs of the human CD15 gene and human MED1 gene with the activity ranking of the guide RNA disclosed previously (Nat Biotechnol, 2014, 32:1262-1267, Nat Biotechnol, 2016, 34:184-191). As a result, both guide RNAs for human CD15 and human MED1 gene showed high Spearman correlation coefficients, and thus it was confirmed that these guide RNAs have high correlation with the activity ranking of the known guide RNA (FIG. 37).

Additionally, the correlation between the degree of activity of the guide RNA obtained using the pair library of the present invention and that of the guide RNA obtained by direct analysis of the target sequences in cells was examined, and as a result, it was confirmed that they exhibited high Spearman correlation coefficients, and therefore, it was confirmed that the method of evaluating the activity of RNA-guided nucleases using the guide RNA-target sequence pair library of the present invention has high accuracy (FIG. 38).

The characteristics of the RNA-guided nucleases analyzed in present invention may include, for example,

(i) a PAM sequence of an RNA-guided nuclease,
(ii) on-target activity of an RNA-guided nuclease, or
(iii) off-target activity of an RNA-guided nuclease.

The characteristics of the RNA-guided nucleases to be analyzed may vary depending on the design of oligonucleotides, and this eventually appears as the results interpreted from the indel frequency being obtained by deep sequencing of the cell library.

In an embodiment, in a case where the PAM sequence of an RNA-guided nuclease is to be confirmed, it is possible to design oligonucleotides such that they have various nucleotide sequences and/or potential PAM sequences where the number of nucleotides of PAM sequences are different at the 5' terminus of a target sequence during the process of these oligonucleotides. Accordingly, the PAM sequence of the corresponding RNA-guided nuclease can be confirmed by analyzing the indel frequency according to PAM sequences.

In a specific embodiment of the present invention, the PAM sequences of the Cpf1 (AsCpf1 and LbCpf1, respectively) derived from *Acidaminococcus* and *Lachnospiraceae* were analyzed using the guide RNA-target sequence pair library, and as a result, it was confirmed that TTTV, and additionally CTTA are true PAM sequences of AsCpf1 and LbCpf1 (FIGS. 13 to 19), contrary to what is previously known with regard to TTTN.

In another embodiment of the present invention, it is possible to perform analysis for the analysis of characteristics of on-target activity by designing various kinds of guide RNAs and target sequences corresponding thereto, or by varying the conditions for applying the RNA-guided nucleases. From the above, it is possible to obtain information that can maximize the target effect during the design of guide RNAs.

In a specific embodiment of the present invention, the characteristics of on-target activity were analyzed by varying the kinds of the RNA-guided nucleases, analyzing the positional characteristics of guide RNAs with high activity, or analyzing the GC content of a target sequence (FIGS. 20 to 22), and in another specific embodiment of the present invention, on-target activity was analyzed by varying the delivery time of lentivirus (FIGS. 23 and 24).

In another embodiment of the present invention, for the analysis of off-target activity, it is possible to design oligonucleotides such that there is a mismatch in part of the sequences between a guide RNA sequence and a target sequence, and in particular, it is possible to design by specifically differentiating the position of the target sequence. Through the above, it is possible to confirm the effect of a nucleotide mismatch according to the position of a target sequence, and this enables obtaining of information that can minimize the off-target activity during the design of a guide RNA.

In a specific embodiment of the present invention, oligonucleotides were designed such that there is a nucleotide mismatch in guide RNA that correspond according to the position of a target sequence, and thereby the relationship between the nucleotide mismatch and off-target effects at each position of the target sequence were analyzed (FIGS. 25 to 33).

The characteristics of the RNA-guided nucleases are to provide one exemplary embodiment for evaluating the activity of RNA-guided nucleases using the guide RNA-target sequence pair library of the present invention, and the present invention should not be interpreted as being limited to the exemplary embodiments above. The characteristics of the core technology of the present invention lies in the evaluation of the activity of RNA-guided nucleases *in vivo* using a cell library including guide RNA-target sequence pairs in a high-throughput manner, and for this purpose, the design methods of the basic oligonucleotides and interpretations of the results thereof can be sufficiently expanded according to the intentions and purposes of those skilled in the art, the kinds of RNA-guided nucleases, *etc.*

Another aspect of the present disclosure provides a cell library including at least two kinds of cells, in which each cell includes an oligonucleotide including a guide RNA-encoding nucleotide sequence, and a target nucleotide sequence which the guide RNA targets.

Still another aspect of the present disclosure provides a vector including an isolated oligonucleotide, which includes a guide RNA-encoding nucleotide sequence and a target nucleotide sequence which the guide RNA targets; and a vector library.

Still another aspect of the present disclosure provides an oligonucleotide including a guide RNA-encoding nucleotide sequence and a target nucleotide sequence which the guide RNA targets; and an oligonucleotide library.

The cell library, vector, vector library, oligonucleotide, and oligonucleotide library are the same as described above.

Still another aspect of the present invention provides a method for constructing the oligonucleotide library, which includes: (a) setting a target nucleotide sequence, which is to be targeted with an RNA-guided nuclease; (b) designing a guide RNA-encoding nucleotide sequence, which forms a base pair with a complementary strand of the set target nucleotide sequence; (c) designing an oligonucleotide, which includes the target nucleotide sequence and a guide RNA that targets the same; and (d) repeating steps (a) to (c) at least once, and specifically two times.

The process of designing oligonucleotides for the constructing of an oligonucleotide library is the same as described above.

The process may be one in which, after determining a target sequence, the sequence of a guide RNA for the target sequence is designed, or a target sequence including a PAM sequence with regard to one guide RNA sequence is designed. That is, it is possible to analyze both on-target activity and off-target activity in the present invention, all or part of the guide RNA sequence may be perfectly complementary to the target sequence, or may be complementary to the target sequence in a state where part of the sequence is mismatched. The design process thereof may be one where several target sequences, which have a deviation in the nucleotide sequence with regard to one guide RNA in terms of the length of the sequence and/or the nucleotide sequence, are designed, and the process may be several guide RNAs, which have a deviation in the nucleotide sequence with regard to one target sequence in terms of the length of the sequence and/or the nucleotide sequence, are designed, and the process may be one such that two processes are achieved in a combined manner.

The step (c) or step (d) may include a step of synthesizing an additionally-designed oligonucleotide.

Still another aspect of the present disclosure provides an isolated guide RNA, which includes a sequence that is able to form a base pair with a complementary strand of a target nucleotide sequence that is adjacent to a protospacer-adjacent motif (PAM) sequence, that is, TTTV or CTTA.

Still another aspect of the present disclosure provides a composition for genome editing, which includes the isolated guide RNA, or a nucleic acid encoding the same.

The isolated guide RNA may be one where the RNA-guided nuclease used in combination is a Cpf1 protein.

Still another aspect of the present disclosure provides a system for genome editing in a mammalian cell, which includes the isolated guide RNA, or a nucleic acid encoding the same; and a Cpf1 protein or a nucleic acid encoding the same.

Still another aspect of the present disclosure provides a method for genome editing with Cpf1 in a mammalian cell, which includes sequentially or simultaneously introducing the guide RNA or a nucleic acid encoding the same; and a Cpf1 protein or a nucleic acid encoding the same, into an isolated mammalian cell.

As described above, it was confirmed in the present invention that the PAM sequences of a Cpf1 protein are TTTV or CTTA, contrary to the previous notion that it is TTTN, and thus, based on the confirmation of the present invention, the guide RNA having TTTV or CTTA as a PAM sequence can be effectively used for genome editing.

## Mode for the Invention

Hereinafter, the present invention will be described in more detail with reference to the following Examples. However, these Examples are for illustrative purposes only and the scope of the present invention is not limited to these Examples.

### Example 1: Preparation of pair library for evaluating activity of Cpf1 and evaluation method thereof

### Example 1-1: Design of oligonucleotides

To construct a plasmid library for the evaluation of the activity of Cpf1 with regard to various guide RNAs in a high-throughput manner, 8,327 oligonucleotides for Cpf1 (AsCpf1) derived from *Acidaminococcus* and 3,634 oligonucleotides for Cpf1 (LbCpf1) derived from *Lachnospiraceae* were synthesized by the CustomArray (Bothell, WA). The oligonucleotides were designed such that they include a guide RNA-encoding sequence (guide sequence) and a target sequence in a length of a total of 122 to 130 nucleotides (FIG. 1).

To compare the indel frequencies at the endogenous position and the introduced position, 82 error-free oligonucleotides including an RNA-encoding sequence and a target sequence were synthesized by the Cellemics, Inc. (Seoul, Korea).

Additionally, a sequence of 27 nucleotides (SEQ ID NO: 1) and a sequence of 22 nucleotides (SEQ ID NO: 2) were included at both ends of the above oligonucleotides, respectively, so that they were able to be used as binding sites for forward and reverse primers during PCR amplification. Additionally, a unique barcode sequence with 15 nucleotides was inserted into the center of each oligonucleotide to enable recognition of each oligonucleotide. The barcode sequence was designed such that it does not include a repetition of two or more nucleotides (*i.e.*, AA, CC, TT, and GG), and all of the barcode sequences were designed such that there is a deviation of at least two nucleotides between the barcode sequences. In each oligonucleotide, the guide RNA sequence and the target sequence were positioned upstream and downstream of the barcode sequence, respectively.

### Example 1-2: Vector cloning

To prepare a Cpf1-expressing lentivirus vector, sequences encoding AsCpfl and LbCpf1 derived from the plasmid (Addgene; #69982, #69988) were replicated into the lentiCas9-Blast plasmid (Addgene; #52962) and they were named as Lenti_AsCpf1-Blast (SEQ ID NO: 3) and Lenti_LbCpf1-Blast (SEQ ID NO: 4), respectively (FIGS. 2 and 3).

Additionally, to obtain a backbone vector for the preparation of a plasmid library, the SpCas9 scaffold region was removed from the lentiGuide-Puro vector (Addgene; #52963), and this vector was named as Lenti-gRNA-Puro vector (SEQ ID NO: 5) (FIG. 4).

### Example 1-3: Preparation of plasmid library

To prepare a plasmid library, the oligonucleotides synthesized in Examples 1 (122 and 130 nucleotides, respectively) were amplified by PCR using the Phusion polymerase (NEB) and gel purification process was performed using the MEGAquick-spin™ Total Fragment DNA Purification Kit (Intron). Then, the Lenti-gRNA-Puro vector and a purified PCR product were assembled using the NEBuidler HiFi DNA Assembly Kit (NEB). After the assembly, the electrocompetent cells (25 μL, Lucigen) were transformed by electroporation using the above reactant (2 μL) using the MicroPulser (BioRad). Then, the transformed cells were inoculated into LB agar medium containing ampicillin (100 μg/mL), and finally, colonies corresponding to a 30-fold number of that of a library were obtained. The colonies were collected and plasmid DNA was extracted therefrom using the Plasmid Maxiprep kit (Qiagen).

### Example 1-4: Production of lentivirus

HEK293T cells (ATCC) were cultured in 100 mm dishes coated with 0.01% poly-L-lysine (Sigma) to a level of 80% to 90% confluency. The transfer plasmid prepared in Example 3 was mixed with psPAX2 and pMD2.G in a weight ratio of 4:3:1. Then, a plasmid mixture (18 μg) was introduced into cells in 100 mm dishes using the iN-fect infection reagent (Intron Biotechnology) according to the manufacturer's directions. 15 Hours after the transfection, the medium was replaced with growth medium (12 mL). The supernatant containing the virus was collected after 39 (= 15 + 24) and 63 (= 15 + 48) hours from the transfection. The primary and secondary batches of the virus-containing medium were mixed, and centrifuged at 4°C at 3,000 rpm for 5 minutes. Then, the supernatant was filtered using the Millex-HV 0.45 μm low protein binding membrane (Millipore) and stored at -80°C until use.

**Example 1-5: Preparation of cell library**

To prepare a cell library, lentivirus vector was transfected to HEK293T cells ($1.5 \times 10^6$ to $2.0 \times 10^6$) which were attached to 100 mm dishes. Three days after the transduction, the cells were treated with puromycin (2 μg/mL) for 3 to 5 days. For the preservation of the library during the progress of the study, the cells containing the library were maintained at a minimum density of $3 \times 10^6$ cells per 100 mm dish. The copy number of lentivirus vector regulatory element (WPRE) was compared with that of endogenous human gene, *ALB,* and the multiplicity of infection (MOI) was confirmed. To measure the copy number of provirus and *ALB* in a genomic DNA sample, real-time qPCR was performed using primers specific to SYBR Advantage qPCR Premix (Clontech), and WPRE or *ALB.* The results are shown in standard curves with lentiGuide-Puro (Addgene; #52963) and pAlbumin. To prevent the quantification bias by the plasmid DNA formation, all of the templates were digested with AhdI before performing PCR. Since the standard plasmid DNA was used in the qPCR analysis, salmon sperm DNA was contained as the background to remedy the efficiency deviation in the quantification of genomic DNA and plasmid DNA. Although the HEK293 cells have almost 3-ploid chromosomes, the chromosome number 4 where the *ALB* gene is located has two pairs and thus the ratio of provirus to the cellular DNA (MOI) was calculated by copy number of WPRE/copy number of *ALB* × 0.5.

**Example 1-6: Transduction of Cpf1 to cell library**

For the transduction of AsCpf1- or LbCpf1-expressing lentivirus vector, first, a cell library ($2 \times 10^6$ to $3 \times 10^6$ cells) was inoculated into 100 mm culture dishes 24 hours before transduction. Then, the AsCpf1-expressing virus vector was transduced into cells in DMEM containing 10% fetal bovine serum (FBS, Gibco), and maintained in DMEM containing 10% FBS and blasticidin S (10 μg/mL, InvivoGen).

In the case of transduction of AsCpf1- or LbCpf1-encoding plasmid, first, the cell library ($3 \times 10^6$ cells) were inoculated into three 60 mm dishes 6 hours before transduction. Then, the cells were transduced with Lenti_AsCpf1-Blast or Lenti_LbCpf1-Blast plasmid (4 μg) and Lipofectamine® 2000 (Invitrogen) (8 μL). The cells were incubated overnight and the medium was replaced with DMEM containing 10% FBS. Then, the transduced cells were cultured in culture medium containing blasticidin (10 μg/mL) from the first day of the transduction for 4 days.

**Example 1-7: deep sequencing**

Genomic DNA was isolated from a cell library using the Wizard Genomic DNA purification kit (Promega). Then, for the analysis of indel frequency, the inserted target sequence was first amplified by PCR using the Phusion polymerase (NEB). To achieve a 100-fold or more of coverage of the cell library, the genomic DNA was used as a template in an amount of 13 μg/sample in the primary PCR (assuming that the genomic DNA for 293T cells ($1 \times 10^6$) as 10 μg). For each sample, 13 independent reactions (50 μL) were performed using the genomic DNA (1 μg) per reaction, and the reaction products were combined.

To compare the indel frequency at the endogenous site and the introduced site, 100 ng of DNA per sample was used as the DNA for the introduced target sequence and the endogenous target sequence for PCR amplification.

Then, the PCR products were purified using the MEGAquick-spin™ Total Fragment DNA Purification Kit (Intron). In the secondary PCR, the purified product of the primary PCR (20 ng) was attached along with the Illumina adaptor and a barcode sequence. The primers used in PCR reactions are shown in Table 1 below. The final products were separated, purified, and mixed, and subjected to analysis using the MiSeq or HiSeq (Illumina).

[Table1]

| Primer | | Sequence (5'-3') |
|---|---|---|
| Lenti_gRNA_ Puro cloning | FP1 | CAC CGG AGA CGT TGA CTA TCG TCT CGC TAC TCT ACC ACT TGT ACT TCA GCG GTC A (SEQ ID NO: 6) |
| | RP1 | AAG CTG ACC GCT GAA GTA CAA GTG GTA GAG TAG CGA GAC GAT AGT CAA CGT CTC C (SEQ ID NO: 7) |
| | FP2 | GCT TAC TCG ACT TAA CGT GCA CGT GAC ACG TTC TAG ACC GTA CAT GCT TAC ATG GGA TGA (SEQ ID NO: 8) |
| | RP2 | AGC TTC ATC CCA TGT AAG CAT GTA CGG TCT AGA ACG TGT CAC GTG CAC GTT AAG TCG AGT (SEQ ID NO: 9) |
| AsCpfl oligo library amplification | FP | ATT TCT TGG CTT TAT ATA TCT TGT GGA AAG GAC GAA ACA CCG TAA TTT CTA CTC TTG TAG (SEQ ID NO: 10) |
| LbCpf1 oligo library amplification | FP | TTT CTT GGC TTT ATA TAT CTT GTG GAA AGG ACG AAA CAC CGT AAT TTC TAC TAA GTG TAG (SEQ ID NO: 11) |
| As/LbCpf1 oligo library amplification | RP | GAG TAA GCT GAC CGC TGA AGT ACA AGT GGT AGA GTA GAG ATC TAG TTA CGC CAA GCT (SEQ ID NO: 12) |
| Targeted deep sequencing | FP | ACA CTC TTT CCC TAC ACG CTC TTC CGA TCT CTT GTG GAA AGG ACG AAA CAC C (SEQ ID NO: 13) |
| | RP | GTG ACT GGA GTT CAG ACG TGT GCT CTT CCG ATC TTT GTG GAT GAA TAC TGC CAT TTG TC (SEQ ID NO: 14) |

(continued)

| Primer | | Sequence (5'-3') |
|---|---|---|
| Indexing of Illumina | FP | AAT GAT ACG GCG ACC GAG ATC TAC AC (SEQ ID NO: 15) - (8bp barcode sequence) - ACA CTC TTT CCC TAC ACG AC (SEQ ID NO: 16) |
| | RP | CAA GCA GAA GAC GGC ATA CGA GAT (SEQ ID NO: 17) - (8bp barcode) - GTG ACT GGA GTT CAG ACG TGT (SEQ ID NO: 18) |
| | | |
| qPCR for WPRE | FP | GAT ACG CTG CTT TAA TGC CTT TG (SEQ ID NO: 19) |
| | RP | GAG ACA GCA ACC AGG ATT TAT ACA AG (SEQ ID NO: 20) |
| qPCR for *ALB* | FP | GCT GTC ATC TCT TGT GGG CTG T (SEQ ID NO: 21) |
| | RP | ACT CAT GGG AGC TGC TGG TTC (SEQ ID NO: 22) |
| Endogenous target 1-5 | FP | TTG CTG TGG CAG AGC CAG CG (SEQ ID NO: 29) |
| | RP | TTG CTT CAC TTT AAT CCT TTC TTG CAG (SEQ ID NO: 30) |
| Endogenous target 6~10 | FP | CTC CTG CAA GAA AGG ATT AAA GTG (SEQ ID NO: 31) |
| | RP | ACC TAC CTA ATA GTT ACT TCC TGA AGG G (SEQ ID NO: 32) |
| Endogenous target | FP | CTC GTT CTT TCC ATC AAA TAG TGT GGT G (SEQ ID NO: 33) |
| 11~14 | RP | CTG CAG TAA TTG TTA CTC TGT GTC TTC C (SEQ ID NO: 34) |
| Endogenous target | FP | TTG AGC TGA CCC ATA AAT ACA GG (SEQ ID NO: 35) |
| 15~17 | RP | CCC TCT TAA CTG GAT CAG CAA CGG (SEQ ID NO: 36) |
| Endogenous target | FP | TGG GGT CGC CAT TGT AGT TCC C (SEQ ID NO: 37) |
| 18 | RP | GTC ACA AAG ATC AGC ATC AGG CAT GG (SEQ ID NO: 38) |
| Endogenous | FP | CGT TCA CCT GGG AGG GGA AG (SEQ ID NO: 39) |
| target 19~22 | RP | TCT GCA AAG AAC TTT ATT CCG AGT AAG C (SEQ ID NO: 40) |
| Endogenous target | FP | CCC AAA AGA CAT ATT CAC CCA GAA TCC C (SEQ ID NO: 41) |

(continued)

| Primer | | Sequence (5'-3') |
|---|---|---|
| 23~28 | RP | CAA CAT CAA GGT GTG GGC AGG GCT GC (SEQ ID NO: 42) |
| | | |
| Endogenous target 29~30 | FP | ACC TGG AGT CTG CAG AGC TGG (SEQ ID NO: 43) |
| | RP | AAG CGG TAA ACA AAG GAT AGC TGG (SEQ ID NO: 44) |
| Endogenous target 31~35 | FP | CCA TGG GAA ACG AAT ACA GGT CTC G (SEQ ID NO: 45) |
| | RP | CTT CAG AAG AAA AAC CTC CAC TC (SEQ ID NO: 46) |
| Endogenous target 36~37 | FP | AAC TGA GAA ACA GCC AGA GAG GAA G (SEQ ID NO: 47) |
| | RP | CAT CTG ATG CTG ACT CAG AGC GC (SEQ ID NO: 48) |
| Endogenous target 38~ 42 | FP | GCT GCC ACC CCC TGC TC (SEQ ID NO: 49) |
| | RP | ATC AGA ATG AAA AAT CTC ACC CCT CC (SEQ ID NO: 50) |
| Endogenous target 43~46 | FP | GTC TCC GTG ATG GGG GTG G (SEQ ID NO: 51) |
| | RP | CTG CCT TGT AAG ACT TTA AAT ATT CTG CTC C (SEQ ID NO: 52) |
| Endogenous target 47~48 | FP | AAG CCA TAT TCA GTT TTA GGG AAA AGC (SEQ ID NO: 53) |
| | RP | ATT TCC AAG TAA GCT GCA AGG AAA GC (SEQ ID NO: 54) |
| Endogenous target 49~52 | FP | AAG TCT TAC AAG GCA GAG TAA AGA TC (SEQ ID NO: 55) |
| | RP | GCA GGG TAA AAC AAT CGG ACC (SEQ ID NO: 56) |
| Endogenous target 53~57 | FP | CAA CCA CCT CAG AAG AGC CAG ATT CC (SEQ ID NO: 57) |
| | RP | CTC TGT AGT TAT TTG AGC AAT GCC AC (SEQ ID NO: 58) |
| Endogenous target 58~64 | FP | CAG TGA ATA TAC AGG ATT GGG GTT GTG (SEQ ID NO: 59) |
| | RP | ACA ACT GGT AAG GTG GGC CCA GG (SEQ ID NO: 60) |
| Endogenous target 65~72 | FP | CAA GCA CAA ACA AAT CAG GCT AAA TCC (SEQ ID NO: 61) |
| | RP | CCC TGA GCT TGG GGG AGA GTT AC (SEQ ID NO: 62) |

(continued)

| Primer | | Sequence (5'-3') |
|---|---|---|
| Endogenous target 73~78 | FP | TCC TCT GGG GAA AGA GTG GCC (SEQ ID NO: 63) |
| | RP | TGT GGG GTC GTT CCT GAT GAA AC (SEQ ID NO: 64) |
| Endogenous target 79~82 | FP | AAC TGG TTT AGC TAG TGC ATA CAT GC (SEQ ID NO: 65) |
| | RP | GGT GGG AGT TTC TGT TAC AGG CAA C (SEQ ID NO: 66) |
| FP: forward primer, RP: reverse primer. | | |

**Example 1-8: Analysis of pair copy number**

For the evaluation of copy number of each pair in a library, the readings were normalized using the following equation.

$$\text{the number of normalized reading per pair} = (\text{the number of reading per pair} / \text{total number of readings for all of the pairs in a sample}) \times 10^6 + 1$$

**Example 1-9: Analysis of indel frequency**

Deep sequencing data was classified and analyzed using the custom Python scripts. Data classification of each guide RNA-target pair was performed based on a 15-bp barcode sequence and a 4-bp constant sequence downstream thereof (i.e., a total 19-bp sequence). The insertion or deletion located in the periphery of the expected cleavage site (i.e., an 8 bp region in the middle of the cleavage site) was considered as a mutation induced by Cpf1. Single nucleotide substitution was removed from the analysis. The actual indel frequency derived from the activity of Cpf1 and guide RNA was calculated by deducting the background indel frequency with the cell library in which Cpf1 was not delivered in the observed indel frequency. The background indel frequency mostly occurs in the synthesis of oligonucleotides. To increase the accuracy of analysis, the deep sequencing data was classified according to the number of reading and the background indel frequency per pair (Table 2).

[Table2]

| Purpose | Minimum reading per pair | Maximum value permitting background indel frequency to be removed from analysis |
|---|---|---|
| Confirmation of AsCpf1 PAM | 100 | 8 % |
| Confirmation of LbCpf1 PAM | 30 | 8 % |
| Profiling of on-target effect of AsCpf1 | 100 | 8 % |
| Profiling of off-target effect of AsCpf1 | 100 | 8 % |
| Analysis of time-dependent indel frequency | 300 | 8 % |
| Profiling of off-target effect of guide RNA fragment | 300 | 8 % |

**Example 1-10: Comparison of indel frequency**

HEK293T cells were seeded into a 48-well dish and transduced with an independent lentivirus vector containing a guide RNA-encoding sequence and a target sequence. After 3 days of the transduction, the cells were treated with puromycin (2 μg/mL) to remove the cells which were not transduced. Cpf1 was delivered to the transduced cells using the AsCpf1-expressing lentivirus vector as described above. Five days after the Cpf1 introduction, DNA was isolated from the cells and was subjected to deep sequencing.

**Example 1-11: Calculation of chromatin accessibility**

Except the chromosome nos. 17 and 22, where 4 copies are present per cell, 4 genome regions were randomly selected. A total of 82 guide RNAs were designed such that they target random loci within the four regions. The DNase I sensitivity score was calculated using the DNase-seq (ENCFF000SPE) data drawn from Encyclopedia of DNA element (ENCODE). The DNase I sensitivity score at each position of the target region was calculated by first counting the overlapping the number of DNase-seq sequencing read fragments at the corresponding position.

For example, when there are two sequencing reading overlaps at the position 5 of the target region, the score at the above position was assumed to be 2. Each region including the PAM and target sequences has a length of 27 bp. As such, the DNase I sensitivity score at the target region was obtained by averaging the 27 scores at each position.

When the DNase I scores at the 82 target regions within 3.2 billion positions of human's genome (hg19/GRCh37 from UCSC genome browser), the scores were shown to be widely distributed (0% to 99.99%).

**Example 2: Preparation of pair library for evaluating activity of Cas9 and evaluation method thereof**

The present inventors have confirmed the method of evaluating activity of the RNA-guided nucleases of the present disclosure using SpCas9, which is a different kind of RNA-guided nuclease.

**Example 2-1: Design of oligonucleotides**

To construct a plasmid library for the evaluation of SpCas9 activity with regard to various guide RNAs in a high-throughput manner, the present inventors have designed guide RNA-target sequence oligonucleotides by a method similar to Examples described above.

Specifically, 89,592 oligonucleotides were synthesized for the Cas9 derived from *Streptococcus pyogenes* (SpCas9) by CustomArray (Bothell, WA) and Twist Bioscience (San Francisco, CA). The oligonucleotides had a total length of 120 nucleotides and they were designed to include a guide RNA-encoding sequence (guide sequence) and a target sequence (FIG. 35). Additionally, a sequence of 26 nucleotides (TATCTTGTGGAAAGGACGAAACACCG, SEQ ID NO: 23) and a sequence of 29 nucleotides (GTTTTAGAGCTAGAAATAGCAAGTTAAAA, SEQ ID NO: 24) were included at both ends of the above oligonucleotides, respectively, so that they were able to be used as binding sites for forward and reverse primers during PCR amplification. Additionally, a unique 15-bp barcode sequence was inserted into the center of each oligonucleotide for the identification of each oligonucleotide. The barcode sequence was designed such that it does not include a repetition of two or more nucleotides (*i.e.*, AA, CC, TT, and GG), and all of the barcode sequences were designed such that there is a deviation of at least two nucleotides between the barcode sequences. In each oligonucleotide, the target sequence and the guide RNA were positioned upstream and downstream of barcode sequence, respectively.

**Example 2-2: Preparation of plasmid library**

To prepare a plasmid library including the oligonucleotides prepared in Examples above, the oligonucleotides (each of 120 nucleotides) were amplified by PCR using the Phusion polymerase (NEB), and gel purification process was performed using the MEGAquick-spin™ Total Fragment DNA Purification Kit (Intron). Then, the LentiGuide_Puro (Addgene, #52963) vector and the purified PCR products were assembled using the NEBuidler HiFi DNA Assembly Kit (NEB). After the assembly, the electrocompetent cells (2 μL, Lucigen) were transformed by electroporation using the above reactant (2 μL) using the MicroPulser (BioRad). Then, the transformed cells were inoculated into LB agar medium containing ampicillin (100 μg/mL), and finally, colonies corresponding to a 17 to 18-fold number of that of a library were obtained. The colonies were collected and plasmid DNA was extracted therefrom using the Plasmid Maxiprep kit (Qiagen).

**Example 2-3: Production of lentivirus**

HEK293T cells (ATCC) were cultured in 100 mm dishes coated with 0.01% poly-L-lysine (Sigma) to a level of 80% to 90% confluency. The transfer plasmid prepared in Example 2-2 was mixed with psPAX2 and pMD2.G in a weight ratio of 4:3:1. Then, a plasmid mixture (18 μg) was introduced into cells in 100 mm dishes using the iN-fect infection reagent (Intron Biotechnology) according to the manufacturer's directions. 15 Hours after the transfection, the medium was replaced with growth medium (12 mL). The supernatant containing the virus was collected after 39 (= 15 + 24) and 63 (= 15 + 48) hours from the transfection. The primary and secondary batches of the virus-containing medium were mixed, and centrifuged at 4°C at 3,000 rpm for 5 minutes. Then, the supernatant was filtered using the Millex-HV 0.45 μm low protein binding membrane (Millipore) and stored at -80°C until use.

**Example 2-4: Preparation of cell library**

To prepare a cell library including the oligonucleotides, the lentivirus vector prepared in Examples above was transfected to HEK293T cells ($7.0 \times 10^6$ cells/dish) which were attached to three 150 mm dishes. Three days after the transduction, the cells were treated with puromycin (2 μg/mL) for 3 to 5 days. For the preservation of the library during the progress of the study, the cells containing the library were maintained at a cell density ($7.0 \times 10^6$ cells/dish) in three 150 mm dishes.

**Example 2-5: Transfer of Cas9 to cell library**

For the transduction of SpCas9-expressing lentivirus vector, the cell library ($2.1 \times 10^7$ cells) prepared in Examples above were first inoculated into three 150 mm culture dishes 24 hours before transduction.

Then, the SpCas9-expressing virus vector was transduced into cells in DMEM containing 10% fetal bovine serum (FBS, Gibco), and maintained in DMEM containing 10% FBS and blasticidin S (10 μg/mL, InvivoGen).

**Example 2-6: Deep sequencing**

Genomic DNA was isolated from the cell library prepared in Examples above using the Wizard Genomic DNA purification kit (Promega).

Then, for the analysis of indel frequency, the inserted target sequence was first amplified by PCR using the Phusion polymerase (NEB). To achieve a 100-fold or more of coverage of the cell library, the genomic DNA was used as a template in an amount of 180 μg/sample in the primary PCR (assuming that the genomic DNA for 293T cells ($1 \times 10^6$) as 10 μg). For each sample, 90 independent reactions (50 μL) were performed using the genomic DNA (2 μg) per reaction, and the reaction products were combined.

Then, the PCR products were purified using the MEGAquick-spin™ Total Fragment DNA Purification Kit (Intron).

In the secondary PCR, the purified product of the primary PCR (20 ng) was attached along with the Illumina adaptor and a barcode sequence. The primers used in PCR reactions are shown in Table 3 below. The final products were separated, purified, and mixed, and subjected to analysis using the MiSeq or HiSeq (Illumina).

[Table3]

| Primer | | Sequence (5'-3') |
|---|---|---|
| SpCas9 oligo library amplification | FP | TTG AAA GTA TTT CGA TTT CTT GGC TTT ATA TAT CTT GTG GAA AGG ACG AAA CAC C (SEQ ID NO: 25) |
| | RP | TTT CAA GTT GAT AAC GGA CTA GCC TTA TTT TAA CTT GCT ATT TCT AGC TCT AAA AC (SEQ ID NO: 26) |
| | | |

(continued)

| Primer | | Sequence (5'-3') |
|---|---|---|
| Targeted deep sequencing (SpCas9) | FP | ACA CTC TTT CCC TAC ACG CTC TTC CGA TCT TGG ACT ATC ATA TGC TTA CCG TAA CTT G (SEQ ID NO: 27) |
| | RP | GTG ACT GGA GTT CAG ACG TGT GCT CTT CCG ATC TTT TGT CTC AAG ATC TAG TTA CGC CAA G (SEQ ID NO: 28) |

The present inventors have performed the evaluation of Cas9 activity in a manner similar to that for the evaluation of Cpf1 activity in Examples above, using the pair library prepared in Examples above.

**Experimental Example 1: Evaluation of Cpf1 activity using pair library**

**Experimental Example 1-1: Development of guide RNA-target sequence pair library**

For the evaluation of Cpf1 activity along with various guide RNAs in a high-throughput manner, the present inventors have prepared a guide RNA-target sequence pair library. They have amplified by PCR a pool of 11,961 array-synthesized oligonucleotides including the target sequences and the guide RNA sequence corresponding thereto (FIG. 1), and cloned with a lentivirus plasmid using the Gibson assembly (FIG. 4). The direct repeat sequence (SEQ ID NO: 20) is a position to which the forward primer binds, and the guide sequence is a sequence for crRNA. The target sequence includes a PAM sequence, and the constant sequence (SEQ ID NO: 21), being a constant region vector annealing site, is a position to which the reverse primer binds. The sequence of the plasmid cloned through the above process has the nucleotide sequence of SEQ ID NO: 3.

To prepare a cell library, which expresses a guide RNA and includes its corresponding sequence in the genome, the lentivirus library prepared from the plasmid library was treated on the HEK293T cells (FIG. 5). Then, to induce the cleavage by the guide RNA and indel formation to the target sequence inserted into the genome, the Cpf1-encoding plasmid was transduced into cells or the Cpf1-expressing lentivirus vector was transduced into cells thereby delivering Cpf1 to the cell library.

Then, the target sequence was amplified by PCR, and deep sequencing-based analysis was performed for the evaluation of indel frequency. As a result, it was confirmed through deep sequencing that the relative copy number of each pair varies in the pool of oligonucleotides. That is, based on the copy number, the copy number showed a deviation of up to the maximum of 130-fold in 99% of the oligonucleotides, excluding the top 0.5% of oligonucleotides having the highest copy number and the bottom 0.5% of oligonucleotides having the lowest copy number (FIG. 6). The plasmid and cell libraries showed a slightly higher level of deviation in copy number compared to that of the oligonucleotide pool. As such, the pair copy numbers of the plasmid and cell libraries were standardized relative to the pair copy number of the oligonucleotide and the plasmid, respectively. As a result, it was confirmed that a low level of deviation was shown compared to the deviation of copy number of the oligonucleotide pool (FIG. 7). The deviation that occurs additionally in most of the copy number during the process of forming the plasmid library and cell library was shown to be within the range of pair copy number deviation of the oligonucleotide and plasmid libraries, respectively (FIGS. 8 and 9). The copy number of each pair in the oligonucleotide pool, plasmid library, and cell library showed a very high correlation (FIGS. 10 to 12). To summarize, these deviations increase along with the progress of the processes of preparing a cell library (*i.e.*, Gibson assembly, transformation, preparation of lentivirus vector, transduction, *etc.*) and the deviation in copy number of each pair in a cell library is mostly caused by the copy number deviation of the oligonucleotide. Meanwhile, the MOI in the cell library was shown to be about 7.0.

The following Table 4 provides a summary of conditions for design and filtering of oligonucleotides for the analysis purpose.

[Table4]

| Category (Purpose) | Number of designed pairs | Filtering conditions | Number of filtered pairs | PAM sequence used | Number of different guide sequences designed | Number of different guide sequences after filtering |
|---|---|---|---|---|---|---|
| Determination of PAM of AsCpf1 | 1,540 | 100 or more (read), 8% or less Background indels | 1,074 | 70 different types | 22 | 18 |
| Determination of PAM of LbCpf1 | 1,540 | 30 or more (read), 8% or less Background indels | 940 | 70 different types | 22 | 16 |
| AsCpf1 activity | 2,381 | 100 or more (read), 8% or less Background indels | 1,251 | ATTTA | 2,381 | 1,251 |
| AsCpf1 activity using truncated guide | 420 | 300 or more (read), 8% or less Background indels | 315 | ATTTA | 7 | 7 |
| AsCpf1 activity for mismatched target sequence | 2,580 | 100 or more (read), 8% or less Background indels | 1,543 | ATTTA | 4 | 3 |
| LbCpf1 activity for mismatched target sequence | 1,342 | 30 or more (read), 8% or less Background indels | 742 | ATTTA | 4 | Not analyzed due to insufficient reading number |
| Comparison of indel frequency in biological replicate | 8,327 | 100 or more (read), 8% or less Background indels | 156 | 70 different types | 3, 794 | 47 |
| Comparison of indel frequency between two different methods of Cpf1 delivery | 8,327 | 200 or more (read), 8% or less Background indels | 233 | 70 different types | 3, 794 | 49 |

The following Table 5 shows a table in which the number of pairs in oligonucleotide pool and cell library are summarized.

[Table5]

| Category | AsCpf1 | | LbCpf1 | |
|---|---|---|---|---|
| | oligonucleotide pool | cell library | oligonucleotide pool | cell library |
| Number of designed pairs | 8,327 | | 3, 634 | |
| Number of pairs included (1 or more: read) | 8,313 | 8,146 | 3, 626 | 3, 497 |
| Percentage of included/designed pairs (%) | 99.8% | 97.8% | 99.8% | 96.2% |
| Number of total reads by deep sequencing | 1,238,978 | 10,378,634 | 475,610 | 584,771 |

**Experimental Example 1-2: Comparison of indel frequencies at endogenous target position and introduced position**

The present inventors have confirmed that there is a strong correlation between indel frequencies of a particular target sequence positioned at the endogenous genome site and the introduced synthesis site by the corresponding lentivirus (FIG. 40). Such a high correlation showed a higher level compared to when a library not forming a pair was used.

Although the chromatin accessibility that affects the efficiency of Cas9-mediated indel formation varies depending on the endogenous region, the lentivirus is integrated more in active transcription region, and thus the chromatin accessibility is expected to be higher in the introduced region. To reduce the changes in indel frequency due to the deviation of chromatin accessibility in the endogenous region, the present inventors compared the correlation between indel frequencies in a subset of the endogenous region and the introduced region with similar chromatin accessibility.

For this purpose, the chromatin accessibility of HEK293T cells was calculated using the DNase I sensitive data obtained from the DNase0seq value obtained from the Encyclopedia of DNA element (ENCODE).

As a result, it was confirmed that the correlation was higher in the target region subset with a similar chromatin accessibility score, and in particular, it was even higher at the subset with higher chromatin accessibility (FIGS. 41 and 42). In most target sequences, the indel frequency in the introduced sequence was higher than that at the endogenous target region, and in particular, was higher in the region with low chromatin accessibility.

Additionally, with regard to the copy number of each constituting element, the cell library showed volatility similar to the libraries used in the previous studies (FIGS. 6 to 11).

Meanwhile, the average MOI of the cell libraries was about 7.0, and there was a strong correlation between the two biological replicates. The delivery of Cpf1 with regard to the two different cell libraries caused a similar indel frequency (FIG. 43).

Additionally, the present inventors have confirmed that there is a clear correlation in indel frequency when Cpf1 was delivered by two different methods (*i.e.*, transient transfection of a Cpf1-encoding plasmid and transduction of a Cpf1-encoding lentivirus vector) (FIG. 44).

In most of the analyzed target sequences, it was confirmed that the indel frequency became higher after the transduction of the Cpf1-encoding lentivirus vector (FIG. 44).

Accordingly, the present inventors have conducted experiments by means of Cpf1 transduction through the lentivirus vector, except the experiment on determining the LbCpf1 PAM which was conducted by transient plasmid transfection.

**Experimental Example 1-3: Confirmation of PAM sequence in mammalian cells**

The present inventors have attempted to confirm the protospacer adjacent motif (PAM) sequence utilized by Cpf1 derived from *Acidaminococcus* (As) or *Lachnospiraceae* (Lb) by *in vivo* system of the present invention. Until today, the PAM sequence, which is used by RNA-programmable nucleases, has been confirmed only in *in vitro* conditions or in a bacterial system, not in mammalian cells. When the Cpf1 derived from As and Lb was analyzed in *in vitro* conditions, 70 (*i.e.*, $4^3$ (indicated as ANNNA) + 3 (indicated as ATTTB) + 3 (indicated as BTTTA)) mutually-different PAM sequences were prepared with regard to 18 (As) or 16 (Lb) guide sequences, considering that TTTN is the most-frequently-used PAM sequence and the structure of AsCpf1 supports TTTN as a potential PAM sequence (a total of 1,260 ($70 \times 18$) target sequences for AsCpf1; and a total of 1,120 ($70 \times 16$) target sequences for LbCpf1, FIG. 13). As a result, the highest indel frequency was shown in both AsCpf1 (FIGS. 14 and 15) and LbCpf1 (FIGS. 16 and 17), when TTTA, TTTC, or TTTG was used as a PAM sequence, except TTTT, in HEK293T cells. These results suggest that TTTV, not TTTN, is the PAM sequence most frequently used in mammalian cells by the above two enzymes. Additionally, except TTTV, CTTA showed the highest indel frequency for Cpf1 derived from As and Lb, and can be considered as a secondary PAM sequence. The deviation in the PAM sequences used in *in vitro* conditions and mammalian cell conditions (FIG. 18) agreed with the deviation in the genome editing efficiency between the two systems, and it suggests that it is very important to verify the PAM sequence in a mammalian cell, not *in vitro,* so as to establish an efficient method for editing mammalian genome.

The co-crystal structure of AsCpf1, crRNA, and target DNA represents that the first three nucleotides (5'-TTT-3') not including forth nucleotide of PAM sequence interacts with the Cpf1 protein, and supports the "5'-TTTN-3'" as a PAM sequence. The *in vivo* verification study of the present inventors helps to understand the PAM preference from TTTN to TTTV in mammalian cells.

Additionally, with regard to the indel frequency of AsCpf1 (not the indel frequency of 1bCpf1), it was confirmed that when TTTA was used as a PAM sequence, there was a high significance in a low level. This suggests that TTTA has a slightly higher preference as a PAM sequence of AsCpf1 to other potential PAM sequences.

Then, the present inventors have evaluated whether the modification of a nucleotide proximal to the 5' terminus of the TTTA PAM can affect the efficiency of genome editing. As a result, it was confirmed that there was no change in indel frequency between aTTTA, tTTTA, cTTTA, and gTTTA (FIG. 19, and FIG. 39a), whereas the indel frequency of LbCpf1 showed a high significance in a low level compared to aTTTA or tTTTA, when cTTTA was used as a PAM sequence (FIG. 39b).

**Experimental Example 1-4: High-throughput profiling of on-target activity**

Then, the present inventors have attempted to confirm the characteristics of target sequences related to the efficiency of guide RNA. Considering that screening of a plurality of guide RNAs is an essential starting point in genome editing, the verification of characteristics of target sequences will be able to promote the development of genome editing technology.

First, the present inventors have evaluated whether the AsCpf1 and the *Streptococcus pyogenes-derived* Cas9 (SpCas9) have similar activity to the same target sequence. Considering difference between positions of PAM sequence of Cas9 and Cpf1, they have compared the activity ranking of Cas9 and Cpf1, which target both the original target sequence and the reverse target sequence (FIG. 20). As a result, it was confirmed that there is no correlation between Cas9 and Cpf1 in all cases.

Then, the nucleotide preference of the AsCpf1 target sequence at each position was examined for 20% of guide RNAs with highest activity. The most striking difference was observed at position 1, which is the nucleotide immediately next to the PAM sequence. In the guide RNA with high activity, thymine was significantly reduced at position 1 (FIG. 21). Although there is a deviation in sequence-specific characteristics, the position immediately next to the PAM is very important in SpCas9 as well.

The present inventors have determined that the lack of preference to thymine at position 1 was due to the instability of interaction between Cpf1 protein and crRNA ribonucleotide that binds to position 1 of a target nucleotide. Based on the structure of DNA-binding AsCpf1 (PDB 5B43), the hydroxy side chain of the Thr16 within the WED domain forms a stable polar interaction with the $N_2$ of guanine base, and also forms the same with $O_2$ of uracil and thymine (FIG. 39).

However, there is no corresponding moiety that can interact with the hydroxy side chain of the Thr16 in adenine, and thus the position of the crRNA adenine ribonucleotide is unstable. Therefore, the thymine at position 1 of the target DNA strand is not preferred.

Finally, the present inventors have confirmed that AsCpf1 exhibits the highest activity with regard to a target sequence having a GC content of 40% to 60% (FIG. 22). This result is similar to the previous result with regard to SpCas9.

Indel frequency is also affected by the length of time for the expression of Cas9 and guide RNA in cells. It was reported in the previous study that when cells were subjected to a long-term culture, for example, 6 to 11 days after the transduction of the lentivirus vector that expresses Cas9 and guide RNA, the indel frequency and knock-out efficiency increase in a time-dependent manner. However, these previous studies were tested for a relatively short period (up to 14 days) with regard to only a small number of guide RNAs (1, 5, or 6), and thus, it had not been explicitly confirmed whether a long-term culture may cause an indel frequency sufficient for overcoming the limitations by sequences with regard to the guide RNA efficiency. In the screening studies at the genomic level where the indel frequency significantly affects in the screening efficiency, major nuclease (i.e., Cas9) and guide RNA are delivered to the lentivirus vector, this is a very important issue. Therefore, the present inventors have attempted to explain the above issue by the analysis of indel frequencies for the 220 guide RNAs expressed for up a month (31 days). When AsCpf1 was delivered to the lentivirus vector, the average and each indel frequency were both significantly increased by increasing the culture period to 5 days (FIGS. 23 and 24). This result is similar to the previous result with regard to SpCas9. However, 5, 10 and 31 days after transduction, the indel frequencies were no difference. These results suggest that the cultivation of 5 or more days cannot increase the indel frequency beyond a particular level, which is mainly determined by the target sequence and the guide RNA sequence.

**Experimental Example 1-4: High-throughput profiling of off-target activity**

Then, the present inventors have attempted to evaluate the off-target activity profile of Cpf1. As a first step, they have attempted to confirm the mismatch effect of the guide RNA sequence with high target cleavage efficiency. In this regard, four guide RNAs for AsCpf1 and four target sequences corresponding thereto were designed, and the target indel frequencies to these were shown to be 53 %, 34 %, 32 %, and 15 % at 5 days after transduction, respectively. Among these, the three guide RNAs with the highest target cleavage efficiency were selected for off-target effect profiling, and their mismatch effects with the target sequences at each position of the guide RNAs were analyzed (FIG. 25). As a result, it was confirmed that one bp mismatch in positions from 1 to 6 significantly reduced the indel frequency (FIG. 26). These results suggest that the above positions are a seed region. As described above, the seed region of guide RNA for AsCpf1, which is verified *in vivo* conditions of the present invention, is similar to the results of conventional *in vitro* experiments where the seed region of the guide RNA with regard to the *Francisella novicida*-derived Cpf1 (FnCpf1) was predicted to be present within the first five positions. Meanwhile, in a case where there is a mismatch of one nucleotide sequence at positions 19 to 23, the indel frequency was shown to decrease slightly (FIG. 26). Accordingly, the present inventors have named this region as a promiscuous region.

Furthermore, in a case where there is a mismatch of one nucleotide sequence at positions 7 to 18, the indel frequency was shown to decrease moderately (FIG. 26). Accordingly, the present inventors have named this region as a trunk region.

From the above results, the present inventors have determined that, in AsCpf1, the nucleotide sequence mismatch in the seed region of the guide RNA and within the 18 nucleotides (nt) in the trunk region is intolerable, whereas the nucleotide sequence mismatch in the promiscuous region is tolerable. These results are consistent with the results of the previous studies that, in *in vitro* DNA cleavage of FnCpf1, it is sufficiently efficient even though the 6 nt at the 3' terminus of guide RNA is cleaved or 18 nt of guide sequence is conserved. Additionally, even with regard to Cas9, it was previously reported that a guide RNA region located distant from a PAM sequence is not important.

Accordingly, the present inventors then analyzed the on-target and off-target effects using a cleaved guide RNA. As a result, it was confirmed that when the 3' terminus of a guide RNA was cut to a size of 4 nt or the length of the guide RNA was shortened to a minimum 19 nt, the on-target indel frequency was maintained and the off-target indel frequency was slowly reduced (FIG. 27). These results indicate that the off-target effect can be reduced without a decrease in on-target effect using a cut guide RNA, similar to the effect observed in SpCas9.

**Experimental Example 1-5: Library-based evaluation of Cpf1 activity having high correlation with indel frequency of endogenous target position**

The present inventors have analyzed the correlation between the number of nucleotide mismatch and off-target effect. As a result, it was confirmed that as the number of nucleotide mismatch at a potential off-target position increased, the off-target effect reduced (FIG. 28).

Furthermore, the present inventors have evaluated the effect of the number of nucleotide mismatch in the five regions consisting of a seed region, a region where a seed is connected to a trunk, a trunk region, a region where a trunk is connected to a promiscuous region, and a promiscuous region. As a result, it was confirmed that as the number of nucleotide mismatch increased, the indel frequency became low in all of the regions. However, in the promiscuous region where a significant indel frequency was shown even when there were 4 to 5 mismatches, this trend was not explicitly shown (FIGS. 29 and 30). Additionally, in the seed region or the region where a seed is connected to a trunk, the mismatch of 3 or more of nucleotides perfectly inhibited indel formation.

Then, the present inventors have examined whether the form of a mismatch can affect the off-target effect. In the seed region and the trunk region, it was confirmed that wobble transition mismatches were correlated with a high indel frequency, compared to non-wobble transition or transversion mismatches (FIGS. 31 to 33). These results are consistent with the unbiased analysis result with regard to the off-target effect of SpCas9. However, such a phenomenon was not observed in the promiscuous region where all types of mismatches only slightly reduced the indel frequency.

**Experimental Example 2: Evaluation of Cas9 activity using pair library**

**Experimental Example 2-1: Preparation of pair library for evaluation of Cas9 activity**

For the evaluation of the activity of Cas9 along with various guide RNAs in a high-throughput manner, the present inventors have prepared a guide RNA-target sequence pair library. They have amplified by PCR a pool of 89,592 array-synthesized oligonucleotides including the target sequences and the guide RNA sequences corresponding thereto (FIG. 35), and cloned with a lentivirus plasmid using the Gibson assembly (FIG. 36).

To prepare a cell library, which expresses a guide RNA and includes its corresponding sequence in the genome, the lentivirus library prepared from the plasmid library was treated on the HEK293T cells (FIG. 5).

Then, to induce the cleavage by the guide RNA and indel formation to the target sequence inserted into the genome, the Cas9-expressing lentivirus vector was transduced into cells thereby delivering Cas9 to the cell library. Then, the target sequence was amplified by PCR, and deep sequencing-based analysis was performed for the evaluation of indel frequency.

**Experimental Example 2-2: Evaluation of Cas9 activity with regard to guide RNA of human CD15 gene and human MED1 gene**

The Cas9 activity with regard to the guide RNA of human CD15 gene and human MED1 gene was evaluated using the pair library prepared in Examples above.

Specifically, the accuracy of the pair library was evaluated by comparing the activity ranking of the guide RNAs using the pair library and the activity ranking of the guide RNA disclosed in the literature (Nat Biotechnol, 2014, 32:1262-1267, Nat Biotechnol, 2016, 34:184-191).

As a result, the guide RNAs with regard to human CD15 gene showed the Spearman correlation coefficient of R = 0.634, whereas the guide RNAs with regard to human MED1 gene (designed within top 80% of the entire length of the exon) showed the Spearman correlation coefficient of R = 0.582, thus confirming that the two pair libraries have high correlation with the activity ranking of known guide RNAs (FIG. 37).

**Experimental Example 2-3: Comparison of guide RNA activity for intracellular target sequence and guide RNA activity of pair library**

The present inventors have attempted to compare the correlation between the degree of activity of the guide RNA obtained using the pair library method and the degree of activity of the guide RNA obtained by direct analysis of the target sequence present in cells.

Specifically, HEK293T cells were inoculated into a 48-well dish, and transduced with the lentivirus vector including a guide RNA-target sequence pair. 3 Days after the transduction, the cells were treated with puromycin (2 μg/mL) and only the transduced cells were selected.

Then, the SpCas9-expressing virus vector was transduced into cells in DMEM containing 10% fetal bovine serum (FBS, Gibco), and maintained in DMEM containing 10% FBS and blasticidin S (10 μg/mL, InvivoGen). After 6 days of transduction of the SpCas9-expressing virus, genomic DNA was isolated from the cell library using the Wizard Genomic DNA purification kit (Promega). Then, the target sequence inserted into the lentivirus and the target sequence present in the cell were first amplified by PCR using Phusion polymerase (NEB) for the analysis of indel frequency. For each sample, the reaction (20 μL) was performed using the genomic DNA (100 ng) per reaction. Then, the PCR products were purified using the MEGAquick-spin™ Total Fragment DNA Purification Kit (Intron).

In the secondary PCR, the purified product of the primary PCR (20 ng) was attached along with the Illumina adaptor and a barcode sequence. The primers used in PCR reactions are shown in Table 3 below. The final products were separated, purified, and mixed, and subjected to analysis using the MiSeq or HiSeq (Illumina).

As a result, it was confirmed that the guide RNA activity for the intracellular target sequence and the guide RNA activity of the pair library were shown to have high correlation (R = 0.546).

From the above result, it was confirmed that the evaluation performed in a high-throughput manner using the SpCas9 guide RNA-target sequence pair library of the present invention has high accuracy (FIG. 38).

**Experimental Example 3: Comparison with conventional method of evaluating Cpf1 activity in target sequence**

The high-throughput method of the present invention for evaluating activity was compared to the existing individual evaluation method.

Specifically, the cost is in USD, and the unit of labor represents the maximum amount of work that can be achieved by those skilled in art for one hour. If there is a break of more than one hour, such as incubation time, it was not counted as labor.

The results are shown in Table 6 below.

[Table6]

| Category | Conventional individual test | | | Method of the present invention | | |
|---|---|---|---|---|---|---|
| | process | cost (USD) | labor (unit) | process | cost (USD) | labor (unit ) |
| Synthesis of oligonucleotide | synthesis | 54,000 | - | synthesis | 2,200 | - |
| Preparation of library | phosphorylation | 4,480 | 100 | amplification of oligonucleotide library | 53 | 0.5 |
| | ligation | 128 | 20 | Gibson assembly | 159 | 0.5 |
| | transformation and plating | - | 220 | transformation and plating | 165 | 1 |
| | plasmid preparation and sequencing | 30,000 | 200 | plasmid preparation and cell library preparation | 112 | 3 |

(continued)

| Category | Conventional individual test | | | Method of the present invention | | |
|---|---|---|---|---|---|---|
| | process | cost (USD) | labor (unit) | process | cost (USD) | labor (unit ) |
| Delivery of CRISPR-Cpf1 | transfection | 749 | 100 | transduction | - | 1 |
| Preparation of sample for deep sequencing | isolation of genomic DNA | of - | 500 | isolation of genomic DNA | of - | 2 |
| | PCR for deep sequencing | deep - | 100 | PCR for deep sequencing | deep - | 1 |
| Subtotal | | 89,357 | 1,240 | | 2, 689 | 9 |

To summarize the above results, the present invention provides a method for high-throughput evaluation of the activity of guide RNA with regard to a particular target sequence in a mammalian cell. It is confirmed that, for genome editing on a particular region of genome or knock-out of a particular gene, guide RNAs can be designed, and in particular, indel frequency can be confirmed by a simple delivery means such as transient transfection. However, indel frequency is not only affected by the efficiency of the guide RNA itself, but also by the transfection efficiency. Accordingly, such a method for identifying the indel frequency may not be able to stably confirm the optimal guide RNA sequence due to the deviation in transfection or delivery efficiency. In the present invention, the efficiency of 10, 000 or more of guide RNAs was confirmed by one trial due to a transduction and/or transfection of a single batch with regard to a cell population, and the errors that may be induced were minimized by a deviation in delivery between different batches. A slightly lower efficiency of transduction or transfection may be able to reduce the efficiency of all of the guide RNAs tested, however, the activity ranking and "relative" activity of guide RNA are maintained, and thus it is possible to select the guide RNA with the highest activity among the tested one. One of the methods to minimize the errors that may be caused due to the different delivery efficiency is to perform repeated experiments, but this requires efforts and costs. Furthermore, the method of using a pair library of the present invention is hardly affected by epigenetic factors that variously appear according to the state and kinds of cells. Since the lentivirus vector is mostly inserted into the transcription active region, when a pair library is delivered to a cell population using the lentivirus vector, the deviation that may be induced by epigenetic state in indel frequency can be minimized. The deviations in delivery efficiency, cell state, and cell types have been raised as one of the most serious problems in comparing the efficiency of guide RNAs. However, the pair library of the present invention enables stable evaluation of the guide RNA efficiency based on sequences, and reduces the possibility that the deviation in delivery or epigenetic state may affect the efficiency.

In the case of a mid-sized unpair double library approach method that can confirm the parameters such as nucleotide sequences and epigenetic state, which may affect the activity of guide RNA, by co-transfection of about 1,400 guide RNA-encoding plasmids to cells, it is difficult to analyze off-target effect because a plurality of guide RNA libraries are co-transfected in each cell, and thus it has a disadvantage in that it is difficult to determine the confirmed indel was formed by which guide RNA. Furthermore, the copy number of the guide RNA significantly affects the cleavage efficiency, and in this case, there is a significant deviation in the copy number within a library thus making it difficult to predict the activity of each guide RNA. The library of the present invention also has a deviation in copy number similarly as in the existing libraries. However, in the present invention, the guide RNA and target sequence are used in the form of a pair, the reaction between the synthesized target sequence and the guide RNA which does not respond to its sequence can be ignored when several pairs are delivered to cells. In addition, a particular guide RNA and the DNA which encodes a synthesized target sequence corresponding thereto are present as a single copy in almost all cells, and thus the deviation associated with copy number can be prevented. Even when a similar on-target sequence is used for the evaluation of off-target, as more copy numbers are introduced than the diversity of the guide RNA sequence, the reaction between a different pair of a guide RNA and a target sequence may not appear at a significant level and thus off-target effect can be evaluated. Moreover, the number of copies to be introduced can be controlled by diluting the lentivirus vector.

The present invention enables the determination of parameters that may affect the manipulation of the RNA-guided genome. That is, the indel frequency can be confirmed at the on-target and off-target positions by various factors, such as a target sequence, kinds of effector nuclease orthologs, structural regions of guide RNA, epigenetic state of target DNA, concentration and duration being exposed to guide RNA and effector nuclease, delivery efficiency of guide RNA and effector nuclease, etc. It is expected that the effects of each parameter in various target sequences can be tested in a high-throughput manner through the pair library of the present invention.

To summarize the above results, the present invention provides a new method for detecting off-target effect.

The off-target effect can be predicted through the *in silico* approach based on the guide-sequence similarity, and can be experimentally measured. Unbiased experimental methods, such as GUIDE-seq, Digenome-seq, BLESS, IDLV capture, HTGTS, etc. have been introduced, but they are not perfectly sensitive or elaborate.

The present study may be considered as "industrial revolution" in the RNA-guided nuclease field. From now on, due to the present invention, the activity of RNA-guided nucleases can be measured *in vivo* in a high-throughput manner (a factory system) based on libraries, instead of relying on the conventional difficult and individual measurement system (a cottage system) (FIG. 34).

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting.

## Claims

**1.** A method for evaluating the activity of an RNA-guided nuclease, comprising:

(a) performing sequence analysis using DNA obtained from a cell library, where an RNA-guided nuclease is introduced, which has a genome comprising an oligonucleotide, wherein the oligonucleotide comprises a guide RNA-encoding nucleotide sequence and a target nucleotide sequence which the guide RNA targets, wherein the target sequence is designed to evaluate on-target activity or off-target activity; and

(b) detecting the indel frequency of each guide RNA-target sequence pair from the data obtained from the sequence analysis.

**2.** The method of claim 1, wherein the oligonucleotide includes a protospacer adjacent motif (PAM) sequence.

**3.** The method of claim 2, wherein the oligonucleotide comprises the guide RNA-encoding sequence, a barcode sequence, the PAM sequence, and the target nucleotide sequence in the 5' to 3' direction or in the reverse direction.

**4.** The method according to any one of claims 1 to 3, wherein the oligonucleotide consists of a sequence of 100 to 200 nucleotides.

**5.** The method according to any one of claims 1 to 3, wherein the guide RNA present in one oligonucleotide is cis-acting on the target nucleotide sequence present in the same oligonucleotide.

**6.** The method according to any one of claims 1 to 3, wherein the method comprises:

(a) introducing an RNA-guided nuclease into a cell library which has a genome comprising an oligonucleotide, wherein the oligonucleotide comprises a guide RNA-encoding nucleotide sequence and a target nucleotide sequence which the guide RNA targets, wherein the target sequence is designed to evaluate on-target activity or off-target activity, wherein the oligonucleotide is introduced into the genome by a virus vector;
(b) performing deep sequencing using the DNA obtained from the cell library where an RNA-guided nuclease is introduced; and
(c) detecting the indel frequency of each guide RNA-target sequence pair from the data obtained from the deep sequencing.

**7.** The method according to any one of claims 1 to 3, wherein the RNA-guided nuclease is a Cas9 protein or Cpf1 protein.

**8.** The method of claim 7, wherein the Cas9 protein is derived from at least one microorganism selected from the group consisting of the genus *Streptococcus,* the genus *Neisseria,* the genus *Pasteurella,* the genus *Francisella,* and the genus *Campylobacter.*

**9.** The method of claim 7, wherein the Cpf1 protein is derived from at least one microorganism selected from the group consisting of the genus *Candidatus Paceibacter,* the genus *Lachnospira,* the genus *Butyrivibrio,* the genus *Peregrinibacteria,* the genus *Acidominococcus,* the genus *Porphyromonas,* the genus *Prevotella,* the genus *Francisella,* the genus *Candidatus Methanoplasma,* and the genus *Eubacterium.*

**10.** The method according to any one of claims 1 to 3, wherein the method analyzes characteristics of the RNA-guided nuclease, wherein the characteristics of the RNA-guided nuclease include at least one selected from the group consisting of:

(i) a PAM sequence of the RNA-guided nuclease;
(ii) on-target activity of the RNA-guided nuclease; or
(iii) off-target activity of the RNA-guided nuclease.

**11.** A virus vector comprising an isolated oligonucleotide, which comprises a guide RNA-encoding nucleotide sequence and a target nucleotide sequence which the guide RNA targets.

**12.** A virus vector library comprising at least two kinds of virus vectors, wherein each virus vector includes an oligonucleotide comprising a guide RNA-encoding nucleotide sequence and a target nucleotide sequence which the guide RNA targets.

**13.** A method for constructing an oligonucleotide library, comprising:

(a) setting a target nucleotide sequence, which is to be targeted with an RNA-guided nuclease;
(b) designing a guide RNA-encoding nucleotide sequence, which forms a base pair with a complementary strand of the set target nucleotide sequence;
(c) designing an oligonucleotide, which comprises the target nucleotide sequence and a guide RNA that targets the same; and

(d) repeating steps (a) to (c) at least once, wherein the oligonucleotide library comprises at least two isolated oligonucleotides, the isolated oligonucleotide comprises a guide RNA-encoding nucleotide sequence and a target nucleotide sequence,

wherein step (c) or step (d) further comprises synthesizing a designed oligonucleotide.

**Patentansprüche**

1. Verfahren zur Bewertung der Aktivität einer RNA-geführten Nuclease, umfassend:

   (a) Durchführen einer Sequenzanalyse unter Verwendung von DNA, die aus einer Zellbibliothek, in die eine RNA-geführte Nuclease eingeführt ist, erhalten wurde und die ein Genom aufweist, das ein Oligonucleotid umfasst, wobei das Oligonucleotid eine Nucleotidsequenz, die eine Führungs-RNA codiert, und eine Zielnucleotidsequenz, die von der Führungs-RNA angesteuert wird, umfasst, wobei die Zielsequenz so gestaltet ist, dass sie On-Target-Aktivität oder Off-Target-Aktivität bewerten kann; und
   (b) Nachweisen der Indelfrequenz jedes Führungs-RNA-Zielsequenz-Paars anhand der aus der Sequenzanalyse erhaltenen Daten.

2. Verfahren gemäß Anspruch 1, wobei das Oligonucleotid eine PAM-Sequenz (Protospacer Adjacent Motif) umfasst.

3. Verfahren gemäß Anspruch 2, wobei das Oligonucleotid die eine Führungs-RNA codierende Sequenz, eine Barcode-Sequenz, die PAM-Sequenz und die Zielnucleotidsequenz in der 5'→'-Richtung oder in der umgekehrten Richtung umfasst.

4. Verfahren gemäß einem der Ansprüche 1 bis 3, wobei das Oligonucleotid aus einer Sequenz von 100 bis 200 Nucleotiden besteht.

5. Verfahren gemäß einem der Ansprüche 1 bis 3, wobei die Führungs-RNA, die in einem Oligonucleotid vorhanden ist, in Bezug auf die Zielnucleotidsequenz, die in demselben Oligonucleotid vorhanden ist, cis-wirkend ist.

6. Verfahren gemäß einem der Ansprüche 1 bis 3, wobei das Verfahren umfasst:

   (a) Einführen einer RNA-geführten Nuclease in eine Zellbibliothek, die ein Genom aufweist, das ein Oligonucleotid umfasst, wobei das Oligonucleotid eine Nucleotidsequenz, die eine Führungs-RNA codiert, und eine Zielnucleotidsequenz, die von der Führungs-RNA angesteuert wird, umfasst, wobei die Zielsequenz so gestaltet ist, dass sie On-Target-Aktivität oder Off-Target-Aktivität bewerten kann, wobei das Oligonucleotid durch einen viralen Vektor in das Genom eingeführt wird;
   (b) Durchführen einer Tiefensequenzierung unter Verwendung der DNA, die aus der Zellbibliothek, in die eine RNA-geführte Nuclease eingeführt ist, erhalten wurde; und
   (c) Nachweisen der Indelfrequenz jedes Führungs-RNA-Zielsequenz-Paars anhand der aus der Tiefensequenzierung erhaltenen Daten.

7. Verfahren gemäß einem der Ansprüche 1 bis 3, wobei die RNA-geführte Nuclease ein Cas9-Protein oder Cpf1-Protein ist.

8. Verfahren gemäß Anspruch 7, wobei das Cas9-Protein aus wenigstens einem Mikroorganismus stammt, der aus der Gruppe ausgewählt ist, die aus der Gattung *Streptococcus,* der Gattung *Neisseria,* der Gattung *Pasteurella,* der Gattung *Francisella* und der Gattung *Campylobacter* besteht.

9. Verfahren gemäß Anspruch 7, wobei das Cpf1-Protein aus wenigstens einem Mikroorganismus stammt, der aus der Gruppe ausgewählt ist, die aus der Gattung *Candidatus Paceibacter,* der Gattung *Lachnospira,* der Gattung *Butyrivibrio,* der Gattung *Peregrinibacteria,* der Gattung *Acidominococcus,* der Gattung *Porphyromonas,* der Gattung *Prevotella,* der Gattung *Francisella,* der Gattung *Candidatus Methanoplasma* und der Gattung *Eubacterium* besteht.

10. Verfahren gemäß einem der Ansprüche 1 bis 3, wobei das Verfahren Merkmale der RNA-geführten Nuclease analysiert, wobei die Merkmale der RNA-geführten Nuclease wenigstens eines umfassen, das aus der Gruppe ausgewählt ist, die aus

(i) einer PAM-Sequenz der RNA-geführten Nuclease;
(ii) der On-Target-Aktivität der RNA-geführten Nuclease; oder
(iii) der Off-Target-Aktivität der RNA-geführten Nuclease besteht.

**11.** Viraler Vektor, der ein isoliertes Oligonucleotid umfasst, das eine Nucleotidsequenz, die eine Führungs-RNA codiert, und eine Zielnucleotidsequenz, die von der Führungs-RNA angesteuert wird, umfasst.

**12.** Virusvektorbibliothek, die wenigstens zwei Arten von viralen Vektoren umfasst, wobei jeder virale Vektor ein Oligonucleotid umfasst, das eine Nucleotidsequenz, die eine Führungs-RNA codiert, und eine Zielnucleotidsequenz, die von der Führungs-RNA angesteuert wird, umfasst.

**13.** Verfahren zum Aufbau einer Oligonucleotidbibliothek, umfassend:

(a) Festlegen einer Zielnucleotidsequenz, die mit einer RNA-geführten Nucleases anzusteuern ist;
(b) Gestalten einer Nucleotidsequenz, die eine Führungs-RNA codiert und die ein Basenpaar mit einem komplementären Strang der festgelegten Zielnucleotidsequenz bildet;
(c) Gestalten eines Oligonucleotids, das die Zielnucleotidsequenz und eine Führungs-RNA, die dieselbe ansteuert, umfasst; und
(d) Wiederholen der Schritte (a) bis (c) wenigstens einmal, wobei die Oligonucleotidbibliothek wenigstens zwei isolierte Oligonucleotide umfasst, wobei das isolierte Oligonucleotid eine Nucleotidsequenz, die eine Führungs-RNA codiert, und eine Zielnucleotidsequenz, die von der Führungs-RNA angesteuert wird, umfasst, wobei Schritt (c) oder Schritt (d) weiterhin das Synthetisieren eines gestalteten Oligonucleotids umfasst.

## Revendications

**1.** Procédé pour évaluer l'activité d'une nucléase guidée par ARN, comprenant les étapes consistant à :

(a) effectuer une analyse séquentielle en utilisant un ADN obtenu d'une bibliothèque de cellules où une nucléase guidée par ARN a été introduite, qui présente un génome comprenant un oligonucléotide, dans lequel ledit oligonucléotide comprend une séquence nucléotidique codant pour un ARN guide, et une séquence nucléotidique cible qui est ciblée par ledit ARN guide, dans lequel la séquence cible est conçue pour évaluer l'activité sur cible ou l'activité hors cible, et
(b) détecter la fréquence d'insertion-délétion de chaque paire d'ARN guide-séquence cible à partir des données obtenues de l'analyse séquentielle.

**2.** Procédé selon la revendication 1, dans lequel ledit oligonucléotide comprend une séquence PAM (protospacer adjacent motif).

**3.** Procédé selon la revendication 2, dans lequel ledit oligonucléotide comprend ladite séquence codant pour un ARN guide, une séquence de codes à barres, ladite séquence PAM, et ladite séquence nucléotidique cible dans le sens 5' vers 3', ou dans le sens inverse.

**4.** Procédé selon l'une quelconque des revendications 1 à 3, dans lequel ledit oligonucléotide consiste en une séquence de 100 à 200 nucléotides.

**5.** Procédé selon l'une quelconque des revendications 1 à 3, dans lequel ledit ARN guide présent dans un oligonucléotide est un élément agissant en cis sur la séquence nucléotidique cible présente dans le même oligonucléotide.

**6.** Procédé selon l'une quelconque des revendications 1 à 3, dans lequel ledit procédé comprend les étapes consistant à :

(a) introduire une nucléase guidée par ARN dans une bibliothèque de cellules qui présente un génome comprenant un oligonucléotide, dans lequel ledit oligonucléotide comprend une séquence nucléotidique codant pour un ARN guide, et une séquence nucléotidique cible qui est ciblée par ledit ARN guide, dans lequel la séquence cible est conçue pour évaluer l'activité sur cible ou l'activité hors cible, dans lequel ledit oligonucléotide est introduit dans le génome au moyen d'un vecteur viral,
(b) effectuer un séquençage exhaustif en utilisant l'ADN obtenu de la bibliothèque de cellules où une nucléase guidée par ARN a été introduite, et
(c) détecter la fréquence d'insertion-délétion de chaque paire d'ARN guide-séquence cible à partir des données obtenues dudit séquençage exhaustif.

**7.** Procédé selon l'une quelconque des revendications 1 à 3, dans lequel ladite nucléase guidée par ARN est une protéine Cas9 ou une protéine Cpf1.

**8.** Procédé selon la revendication 7, dans lequel ladite protéine Cas9 est dérivée d'au moins un microorganisme choisi dans le groupe consistant en le genre *Streptococcus,* le genre *Neisseria,* le genre *Pasteurella,* le genre *Francisella,* et le genre *Campylobacter.*

**9.** Procédé selon la revendication 7, dans lequel ladite protéine Cpf1 est dérivée d'au moins un microorganisme choisi dans le groupe consistant en le genre *Candidatus Paceibacter,* le genre *Lachnospira,* le genre *Butyrivibrio,* le genre *Peregrinibacteria,* le genre *Acidominococcus,* le genre *Porphyromonas,* le genre *Prevotella,* le genre *Francisella,* le genre *Candidatus Methanoplasma,* et le genre *Eubacterium.*

**10.** Procédé selon l'une quelconque des revendications 1 à 3, dans lequel le procédé analyse des caractéristiques de ladite nucléase guidée par ARN, dans lequel les caractéristiques de la nucléase guidée par ARN comprennent au moins une choisie dans le groupe consistant en :

(i) une séquence PAM de la nucléase guidée par ARN,
(ii) l'activité sur cible de la nucléase guidée par ARN, ou
(iii) l'activité hors cible de la nucléase guidée par ARN.

**11.** Vecteur viral comprenant un oligonucléotide isolé, qui comprend une séquence nucléotidique codant pour un ARN guide, et une séquence nucléotidique cible qui est ciblée par ledit ARN guide.

**12.** Bibliothèque de vecteurs viraux comprenant au moins deux espèces de vecteurs viraux, dans laquelle chaque de vecteur viral comprend un oligonucléotide comprenant une séquence nucléotidique codant pour un ARN guide, et une séquence nucléotidique cible qui est ciblée par ledit ARN guide.

**13.** Procédé pour la construction d'une bibliothèque d'oligonucléotides, comprenant les étapes consistant à :

(a) définir une séquence nucléotidique cible qui doit être ciblée avec une nucléase guidée par ARN,
(b) concevoir une séquence nucléotidique codant pour un ARN guide, qui forme une paire de bases avec un brin complémentaire de la séquence nucléotidique cible ainsi définie,
(c) concevoir un oligonucléotide comprenant la séquence nucléotidique cible et un ARN guide qui la cible, et
(d) répéter les étapes (a) à (c) au moins une fois, dans lequel ladite bibliothèque d'oligonucléotides comprend au moins deux oligonucléotides isolés, ladite oligonucléotide isolé comprend une séquence nucléotidique codant pour un ARN guide, et une séquence nucléotidique cible,
dans lequel ladite étape (c) ou étape (d) comprend en outre l'étape consistant à synthétiser un oligonucléotide défini.

FIG. 1

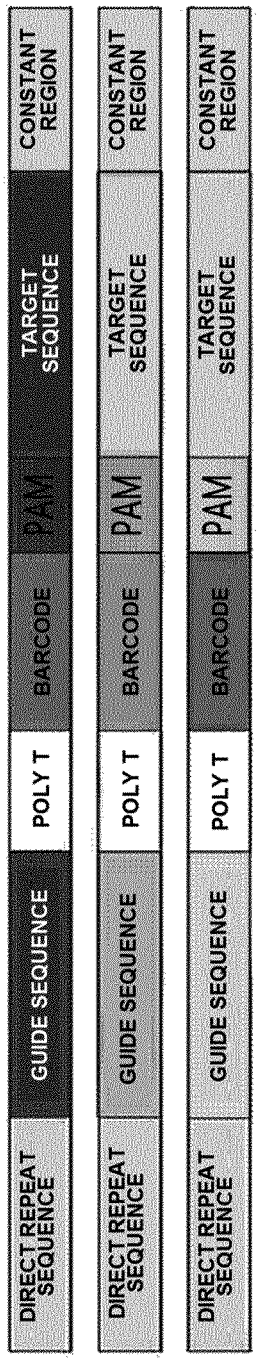
DIRECT REPEAT SEQUENCE
GUIDE SEQUENCE
POLY T
BARCODE
PAM
TARGET SEQUENCE
CONSTANT REGION
DIRECT REPEAT SEQUENCE
GUIDE SEQUENCE
POLY T
BARCODE
PAM
TARGET SEQUENCE
CONSTANT REGION
DIRECT REPEAT SEQUENCE
GUIDE SEQUENCE
POLY T
BARCODE
PAM
TARGET SEQUENCE
CONSTANT REGION

FIG. 2

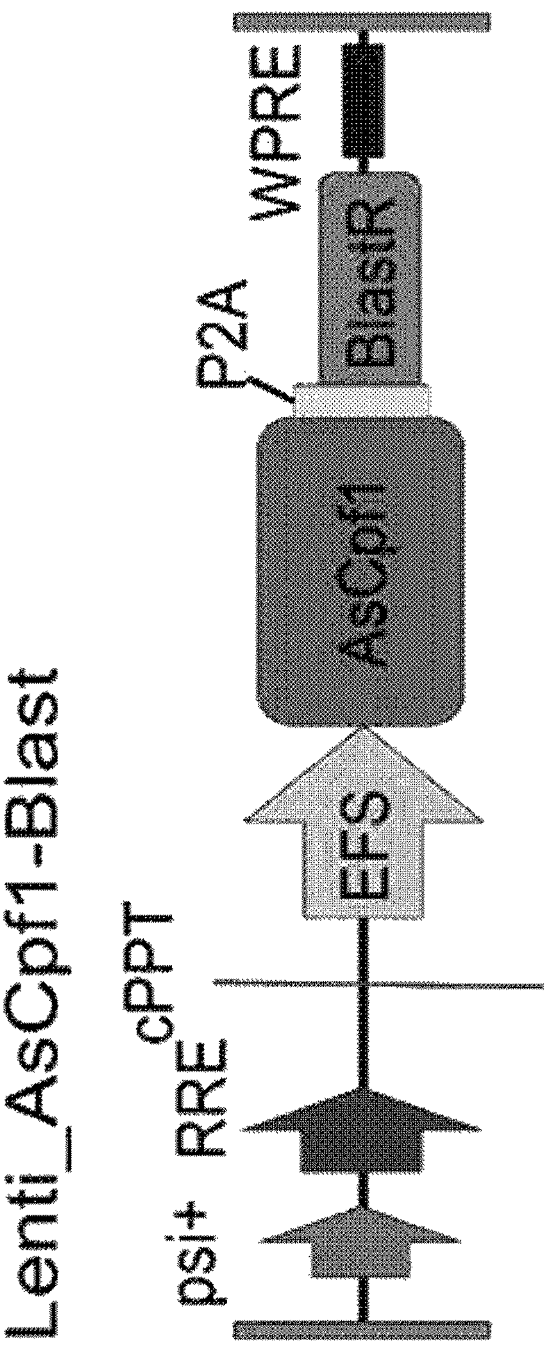

FIG. 3

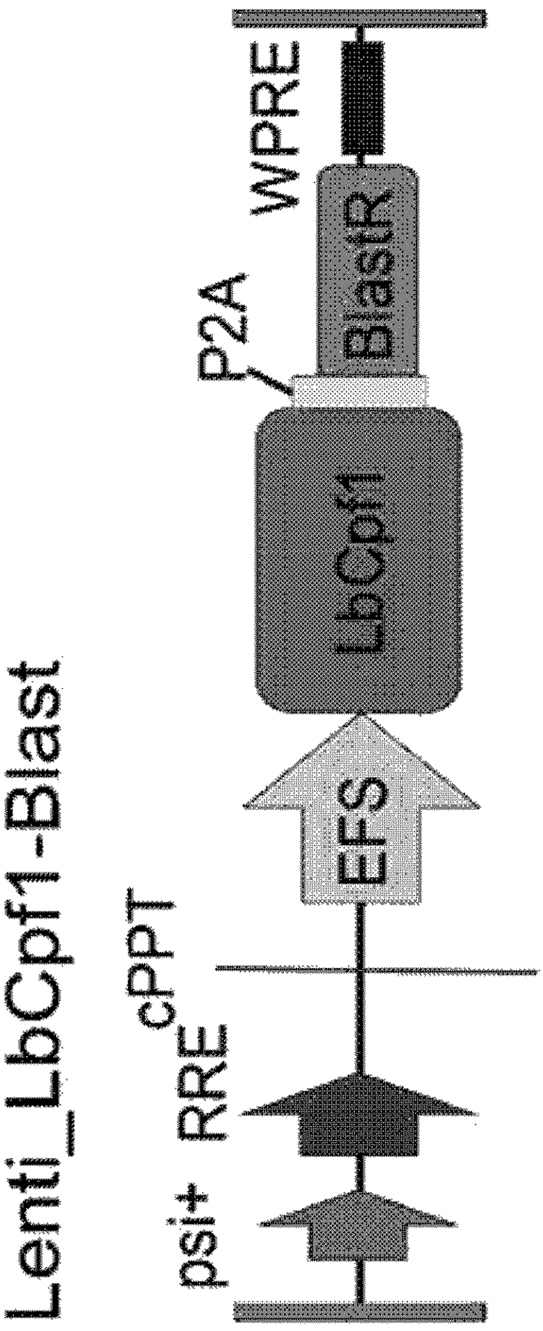

FIG. 4

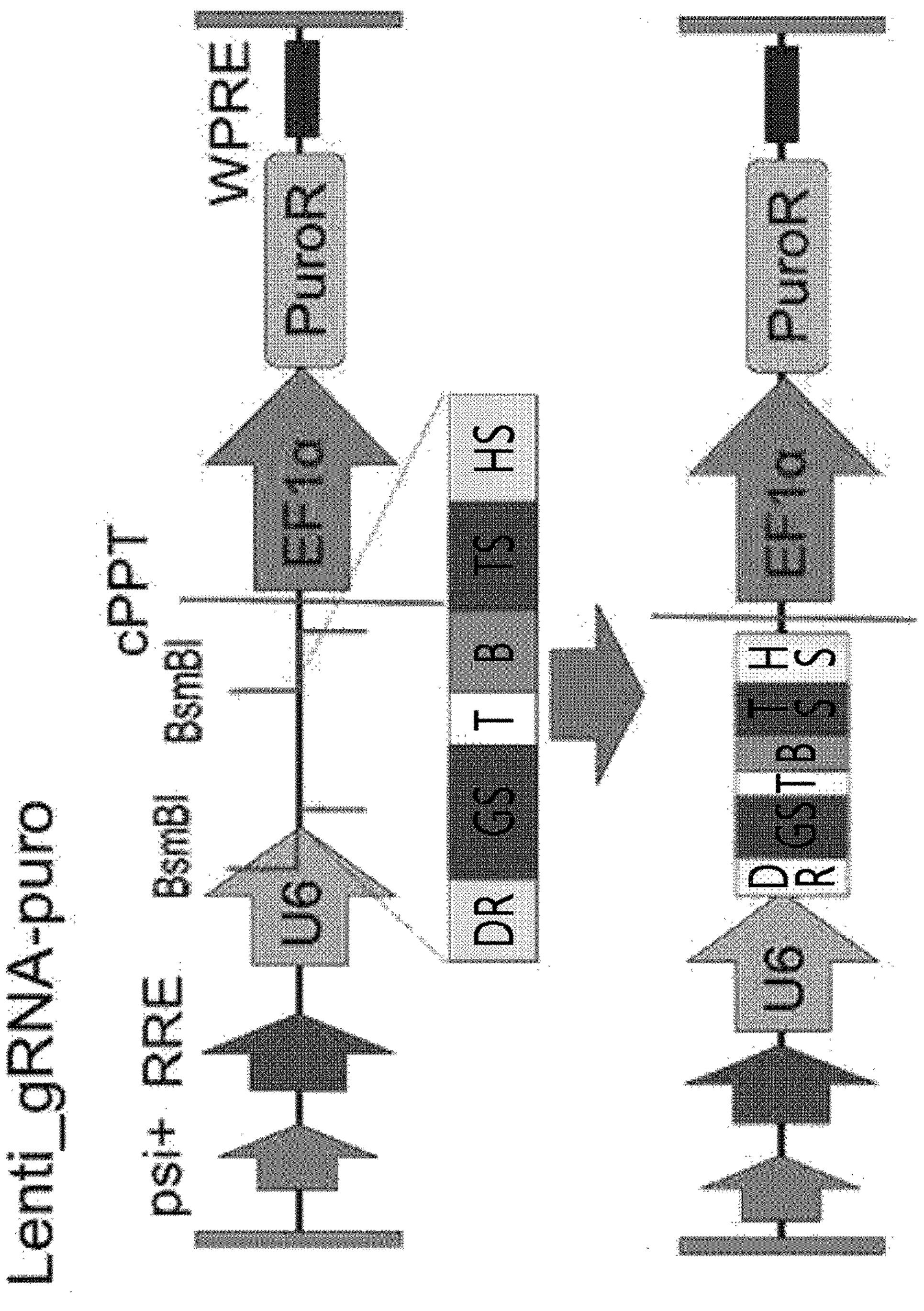
Lenti_gRNA-puro
psi+
RRE
U6
BsmBI
BsmBI
cPPT
EF1α
PuroR
WPRE
DR
GS
T
B
TS
HS

FIG. 5

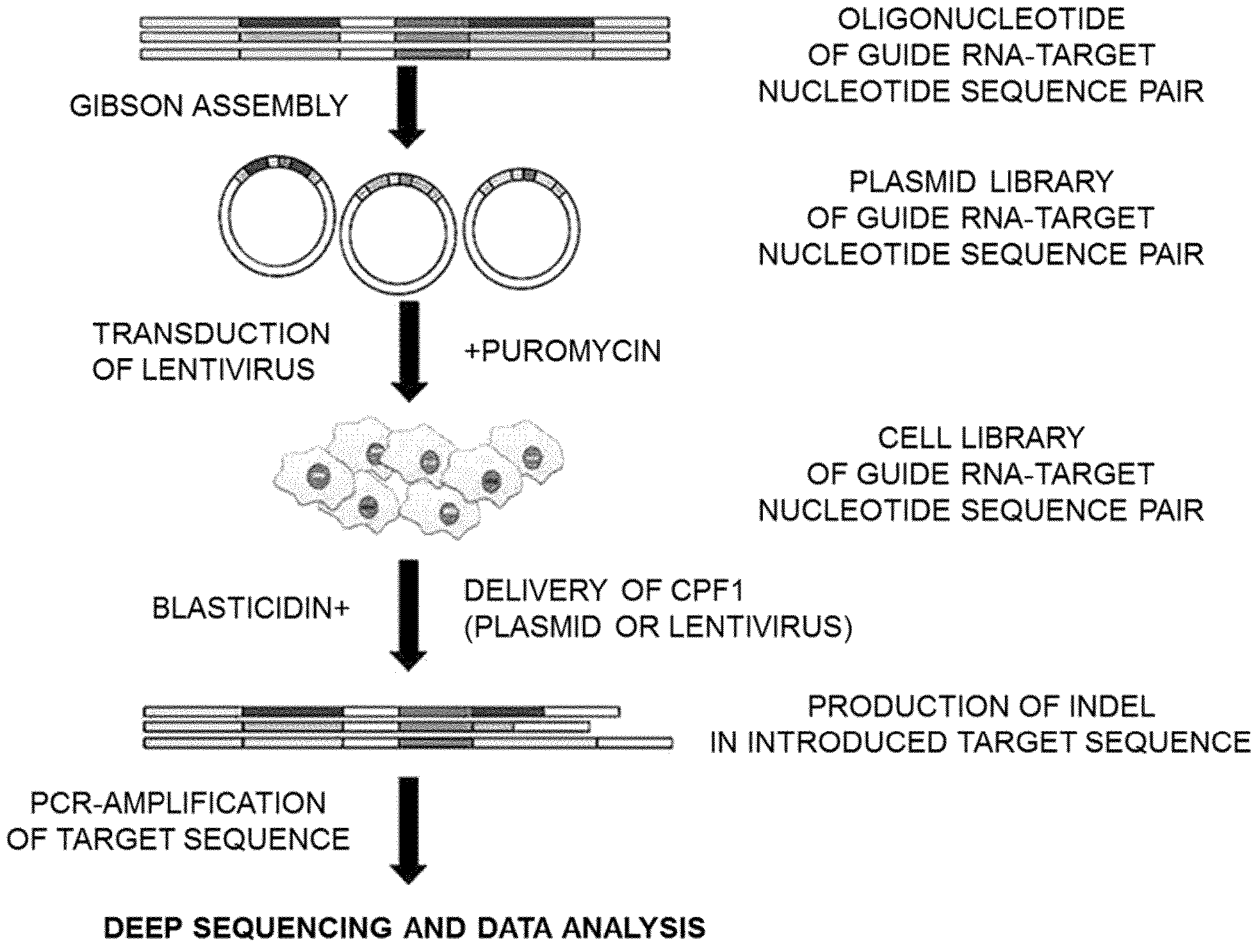
OLIGONUCLEOTIDE
OF GUIDE RNA-TARGET
NUCLEOTIDE SEQUENCE PAIR
GIBSON ASSEMBLY
PLASMID LIBRARY
OF GUIDE RNA-TARGET
NUCLEOTIDE SEQUENCE PAIR
TRANSDUCTION
OF LENTIVIRUS
+PUROMYCIN
CELL LIBRARY
OF GUIDE RNA-TARGET
NUCLEOTIDE SEQUENCE PAIR
BLASTICIDIN+
DELIVERY OF CPF1
(PLASMID OR LENTIVIRUS)
PRODUCTION OF INDEL
IN INTRODUCED TARGET SEQUENCE
PCR-AMPLIFICATION
OF TARGET SEQUENCE
DEEP SEQUENCING AND DATA ANALYSIS

FIG. 6

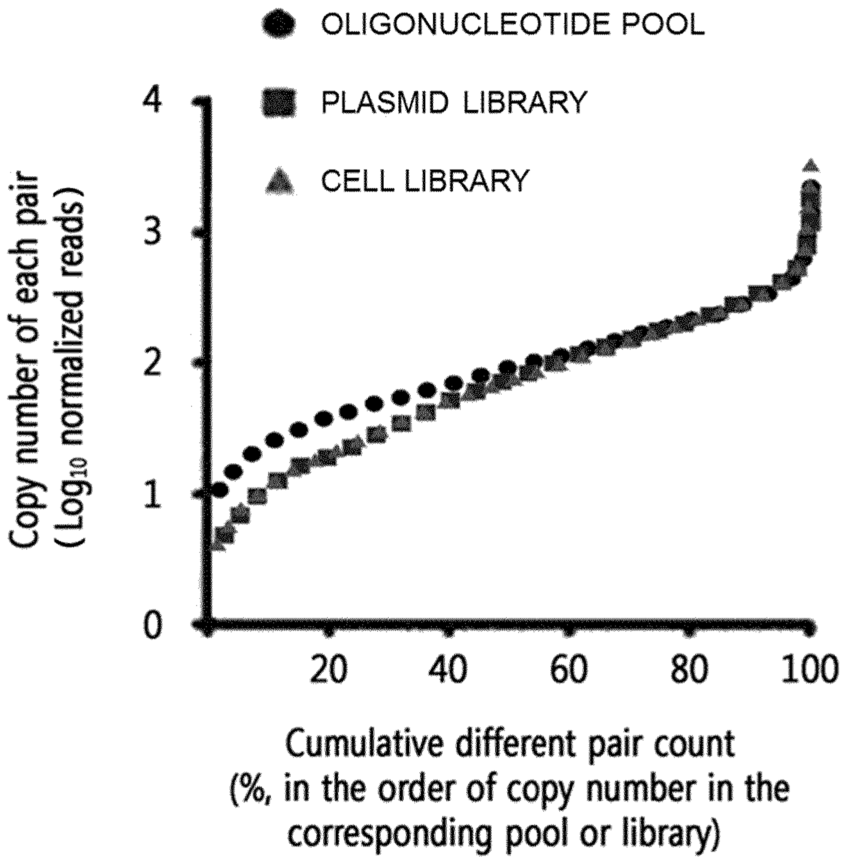
OLIGONUCLEOTIDE POOL
PLASMID LIBRARY
CELL LIBRARY
4
3
2
1
0
Copy number of each pair
( $Log_{10}$ normalized reads)
20
40
60
80
100
Cumulative different pair count
(%, in the order of copy number in the corresponding pool or library)

FIG. 7

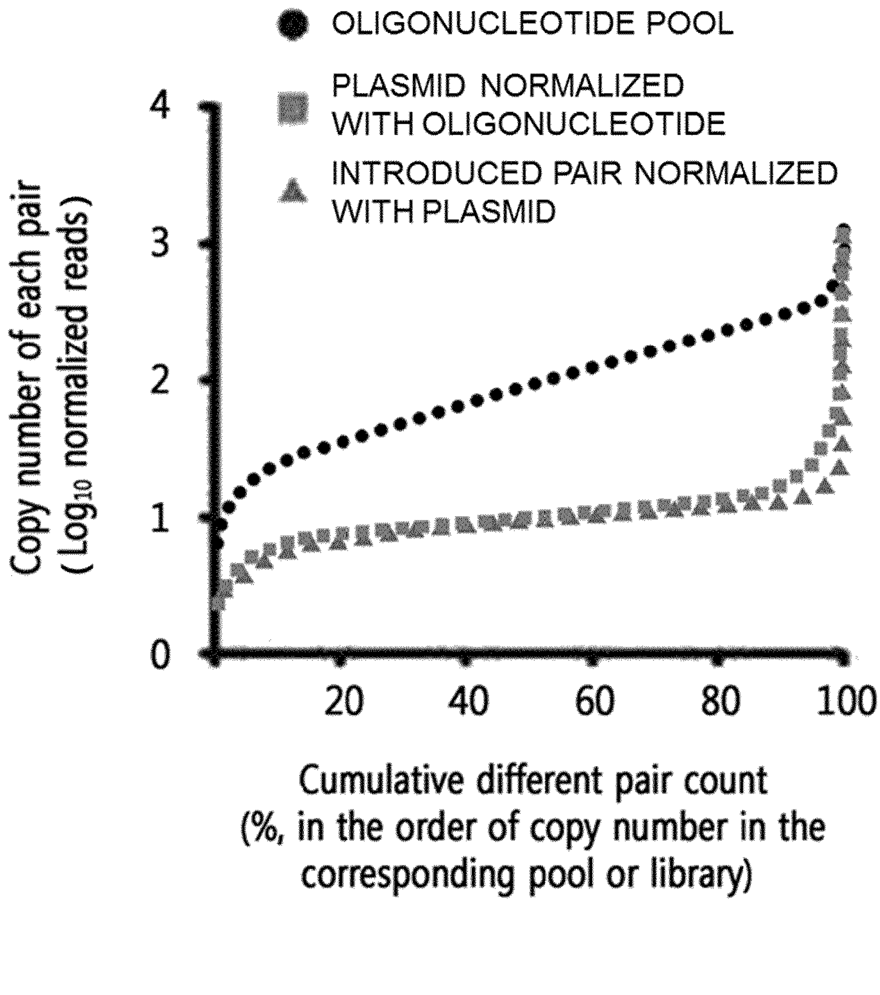
OLIGONUCLEOTIDE POOL
PLASMID NORMALIZED WITH OLIGONUCLEOTIDE
INTRODUCED PAIR NORMALIZED WITH PLASMID
Copy number of each pair
( $Log_{10}$ normalized reads)
0
1
2
3
4
20
40
60
80
100
Cumulative different pair count
(%, in the order of copy number in the corresponding pool or library)

FIG. 8

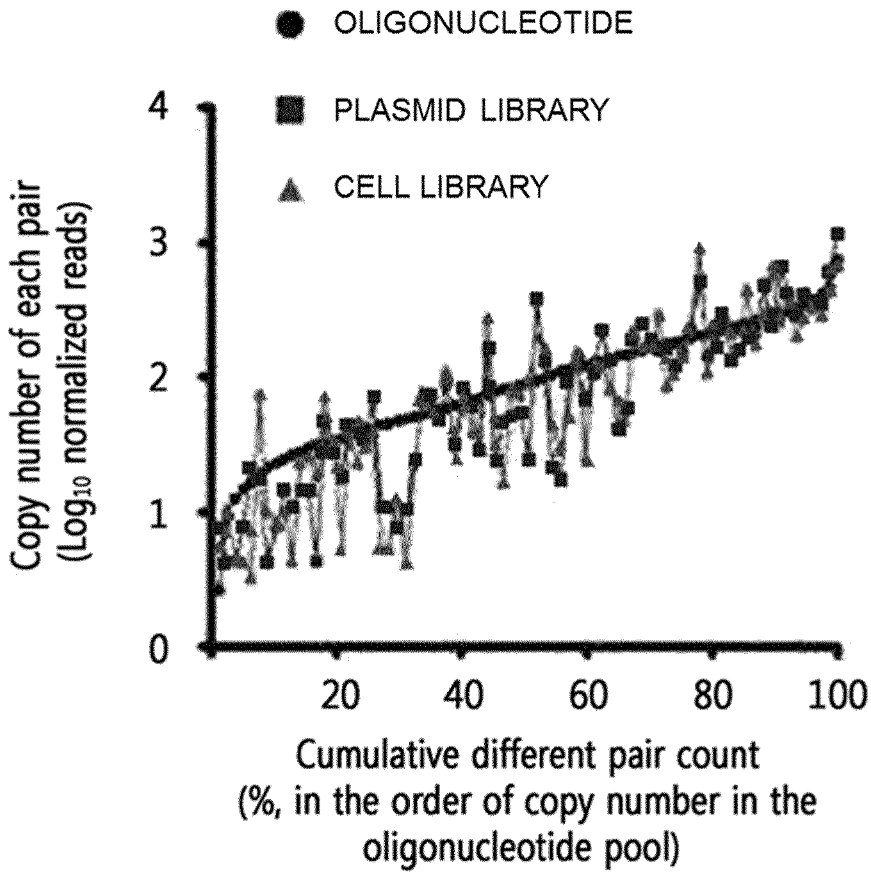
OLIGONUCLEOTIDE
PLASMID LIBRARY
CELL LIBRARY
Copy number of each pair
(Log10 normalized reads)
0
1
2
3
4
20
40
60
80
100
Cumulative different pair count
(%, in the order of copy number in the
oligonucleotide pool)

FIG. 9

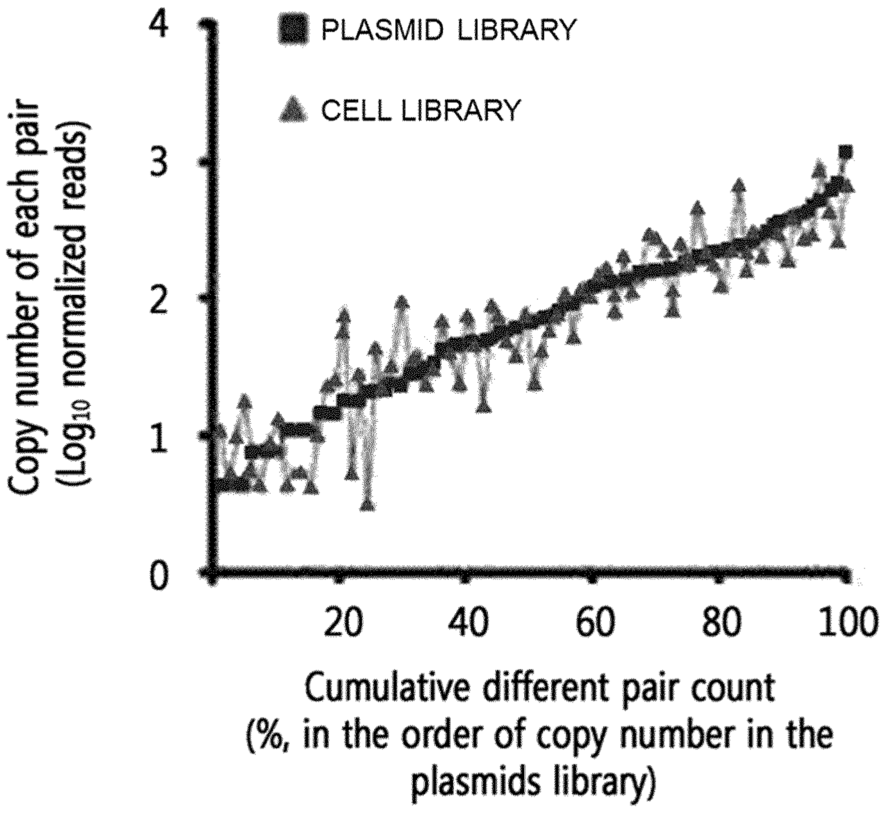
PLASMID LIBRARY
CELL LIBRARY
Copy number of each pair
(Log10 normalized reads)
0
1
2
3
4
20
40
60
80
100
Cumulative different pair count
(%, in the order of copy number in the
plasmids library)

FIG. 10

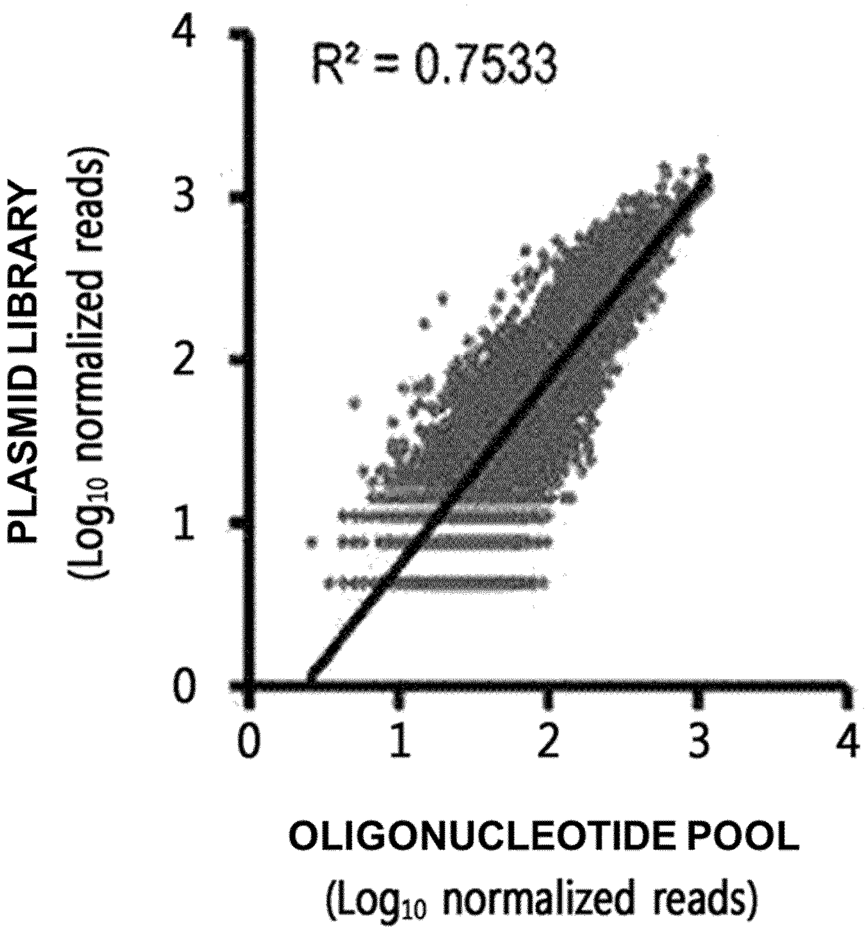
R² = 0.7533
PLASMID LIBRARY
(Log10 normalized reads)
0
1
2
3
4
0
1
2
3
4
OLIGONUCLEOTIDE POOL
(Log10 normalized reads)

FIG. 11

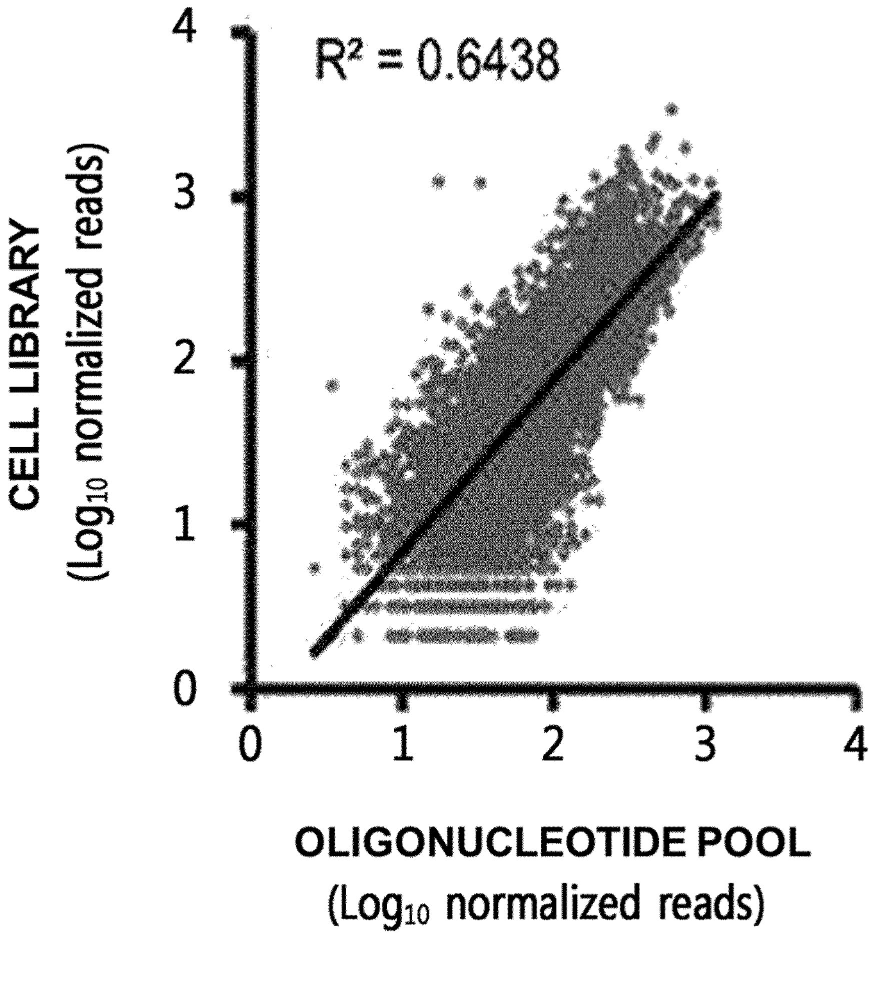

$R^2$ = 0.6438
CELL LIBRARY
(Log10 normalized reads)
OLIGONUCLEOTIDE POOL
(Log10 normalized reads)
0
1
2
3
4

FIG. 12

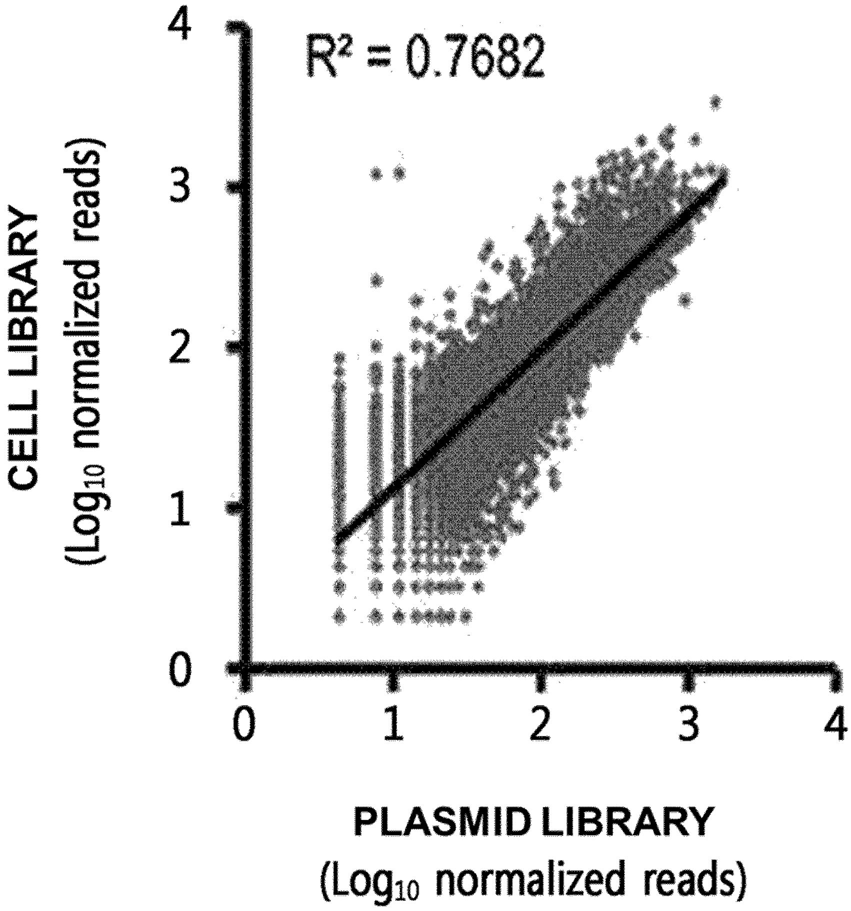
R² = 0.7682
CELL LIBRARY
(Log10 normalized reads)
0
1
2
3
4
PLASMID LIBRARY
(Log10 normalized reads)

FIG. 13

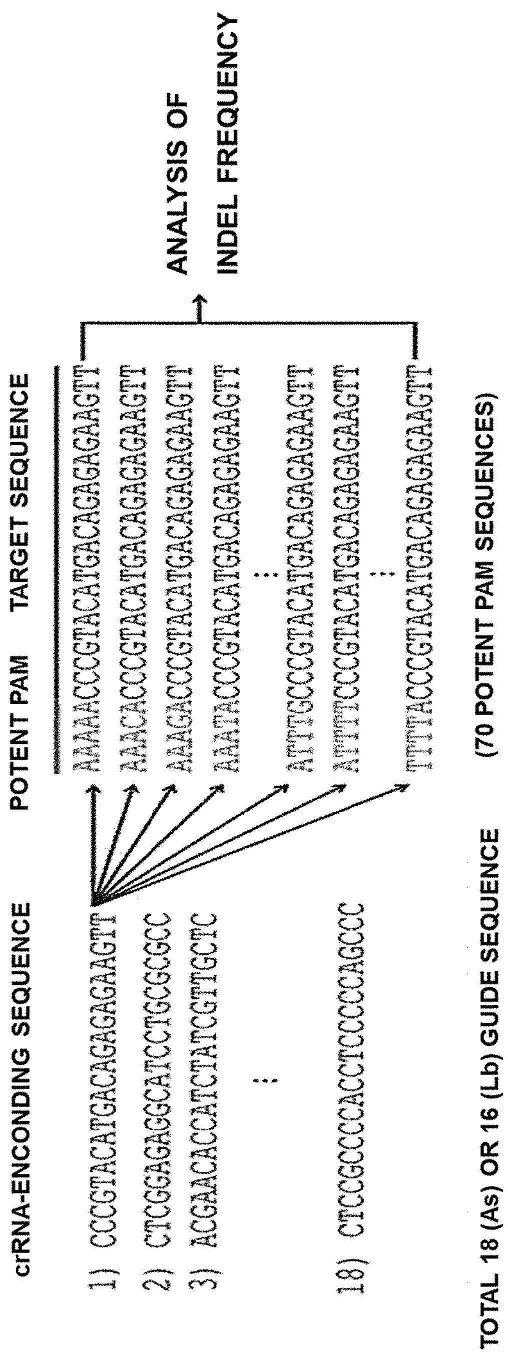
crRNA-ENCONDING SEQUENCE
1) CCCGTACATGACAGAGAGAAGTT
2) CTCGGAGAGGCATCCTGCGCGCC
3) ACGAACACCATCTATCGTTGCTC
18) CTCCGCCCCACCTCCCCCAGCCC
TOTAL 18 (As) OR 16 (Lb) GUIDE SEQUENCE
POTENT PAM
TARGET SEQUENCE
AAAAACCCGTACATGACAGAGAGAAGTT
AAACACCCGTACATGACAGAGAGAAGTT
AAAGACCCGTACATGACAGAGAGAAGTT
AAATACCCGTACATGACAGAGAGAAGTT
ATTTGCCCGTACATGACAGAGAGAAGTT
ATTTTCCCGTACATGACAGAGAGAAGTT
TTTTACCCGTACATGACAGAGAGAAGTT
(70 POTENT PAM SEQUENCES)
ANALYSIS OF
INDEL FREQUENCY

FIG. 14

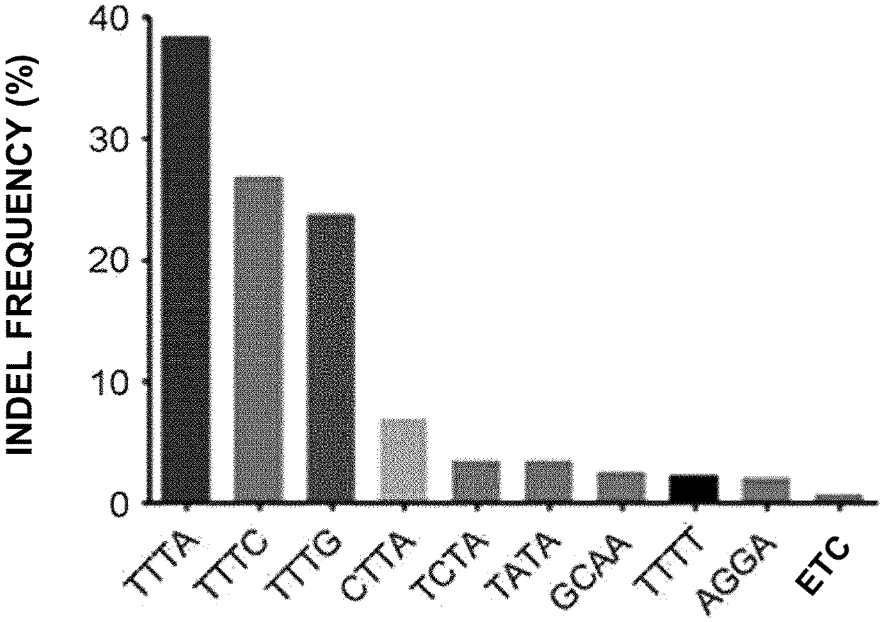
INDEL FREQUENCY (%)
40
30
20
10
0
TTTA
TTTC
TTTG
CTTA
TCTA
TATA
GCAA
TTTT
AGGA
ETC

FIG. 15

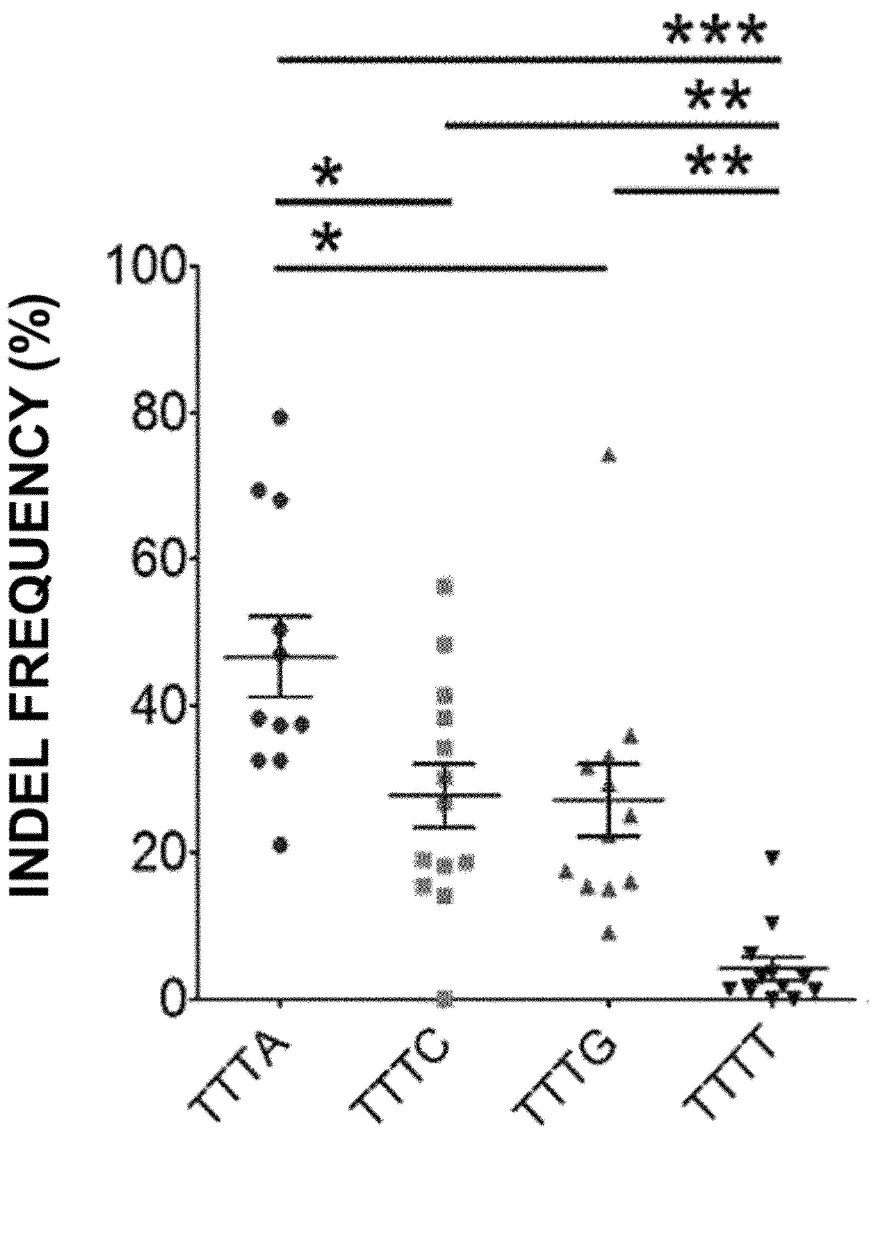

FIG. 16

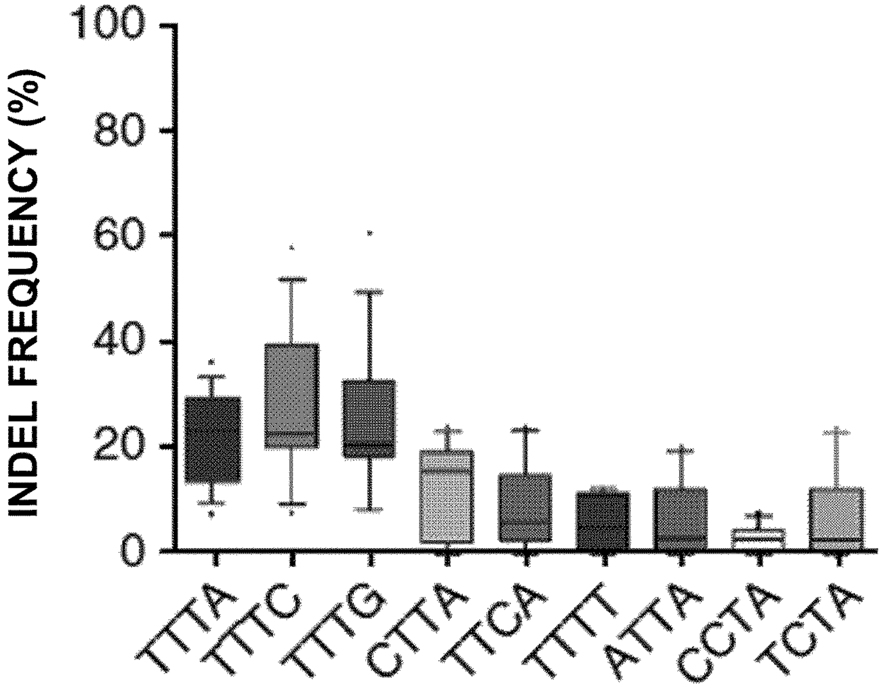
INDEL FREQUENCY (%)
100
80
60
40
20
0
TTTA
TTTC
TTTG
CTTA
TTCA
TTTT
ATTA
CCTA
TCTA

FIG. 17

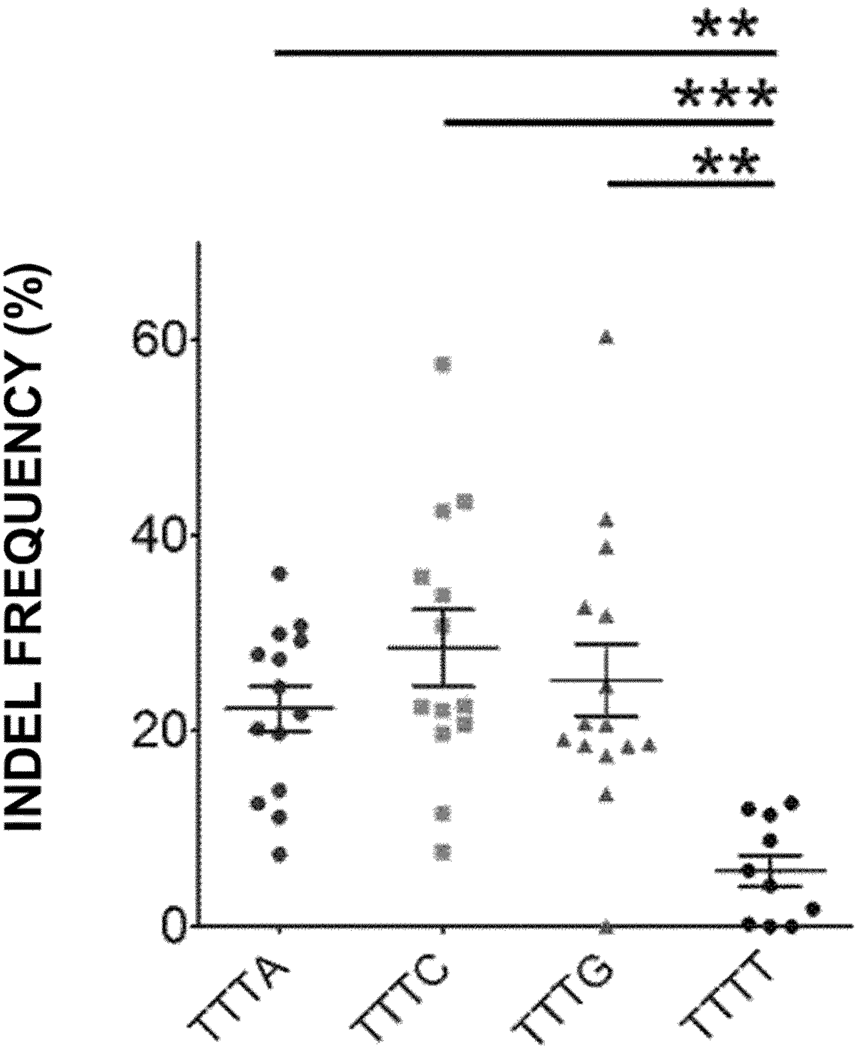

FIG. 18

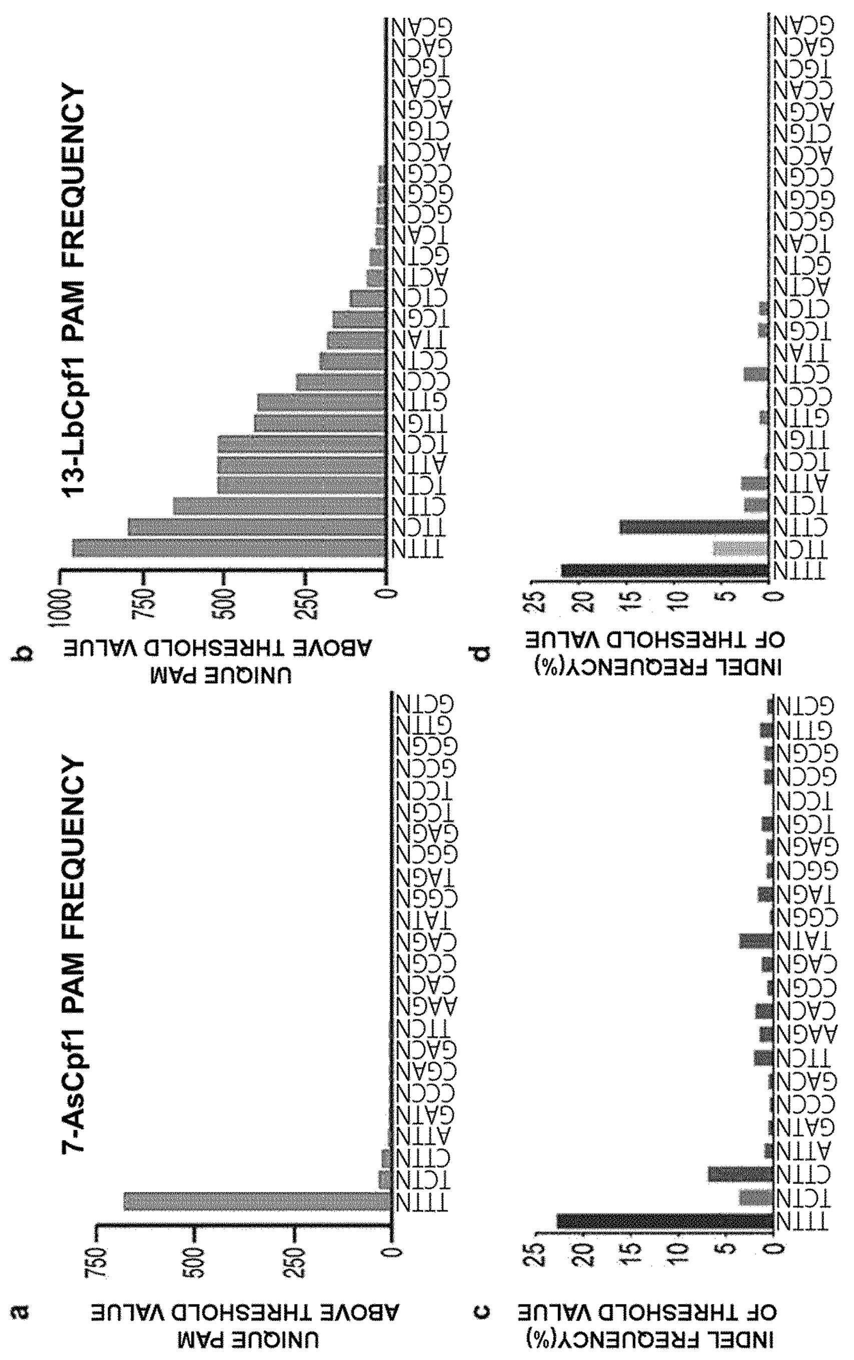
a
UNIQUE PAM ABOVE THRESHOLD VALUE
7-AsCpf1 PAM FREQUENCY
b
UNIQUE PAM ABOVE THRESHOLD VALUE
13-LbCpf1 PAM FREQUENCY
c
INDEL FREQUENCY(%) OF THRESHOLD VALUE
d
INDEL FREQUENCY(%) OF THRESHOLD VALUE

FIG. 19

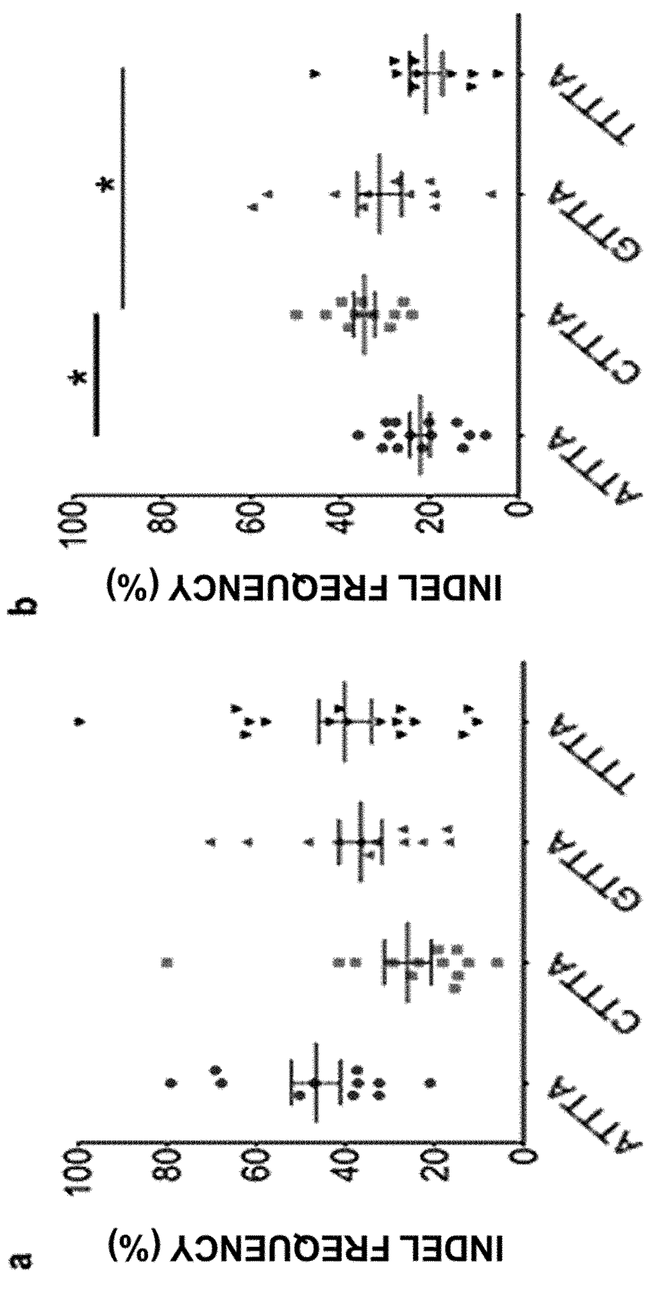
a
INDEL FREQUENCY (%)
100
80
60
40
20
0
ATTTA
CTTTA
GTTTA
TTTTA
b
INDEL FREQUENCY (%)
100
80
60
40
20
0
ATTTA
CTTTA
GTTTA
TTTTA
*
*

FIG. 20

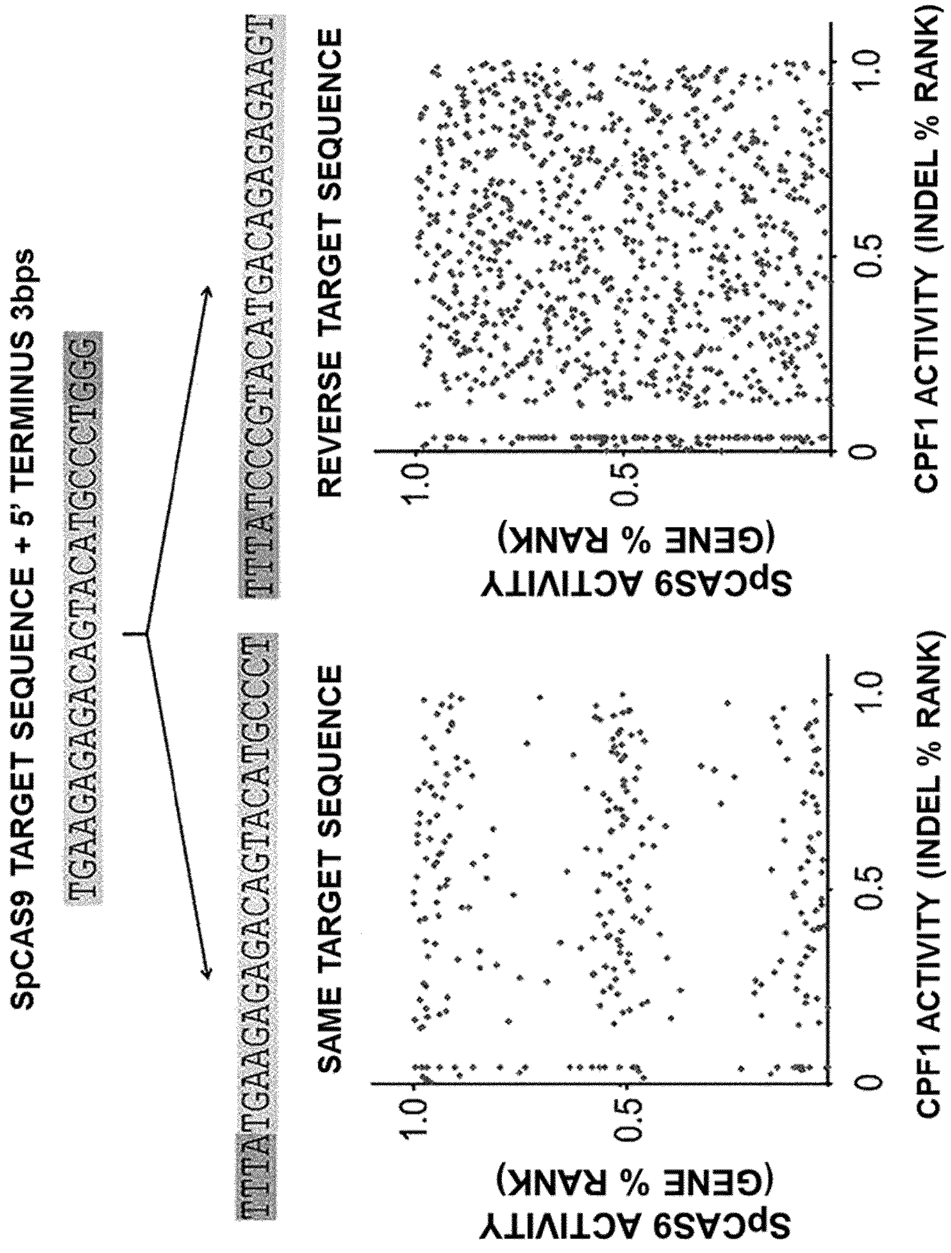
SpCAS9 TARGET SEQUENCE + 5' TERMINUS 3bps
TGAAGAGAGACAGTACATGCCCTGGG
TTTATGAAGAGAGACAGTACATGCCCT
TTTATCCCGTACATGACAGAGAGAAGT
SAME TARGET SEQUENCE
REVERSE TARGET SEQUENCE
SpCAS9 ACTIVITY (GENE % RANK)
CPF1 ACTIVITY (INDEL % RANK)
1.0
0.5
0

FIG. 21

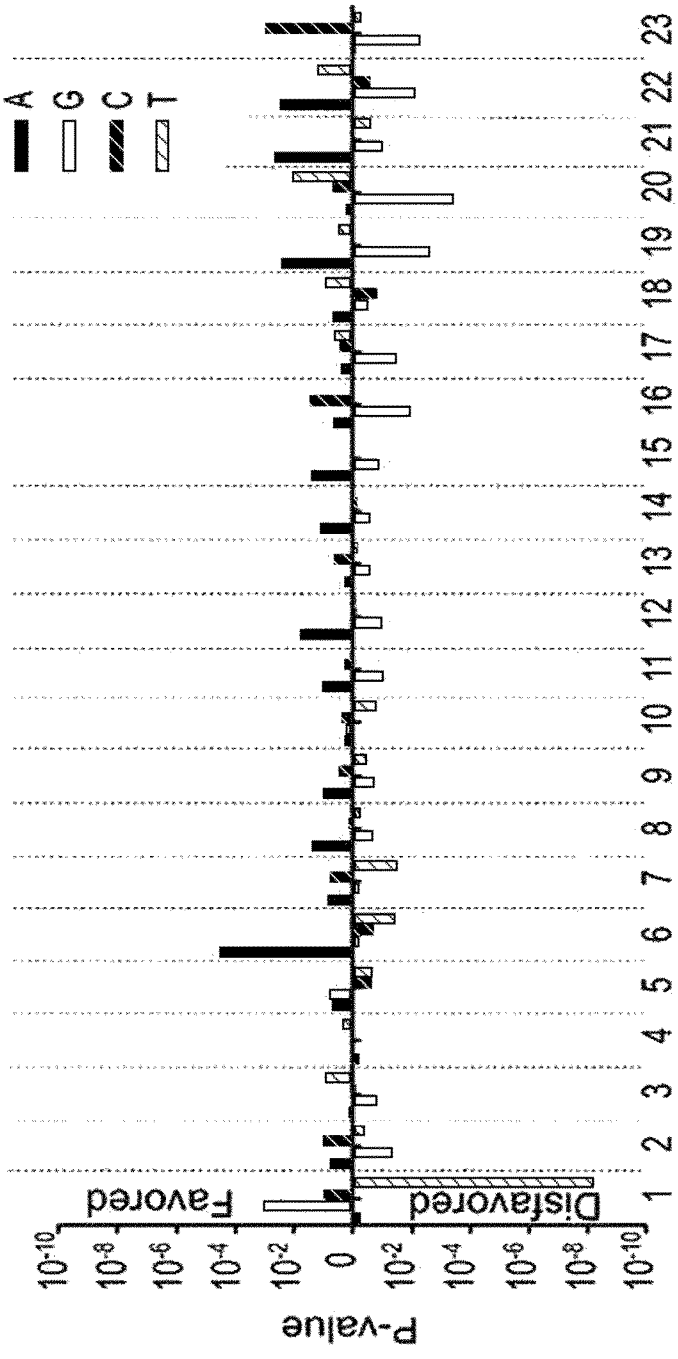

A
G
C
T
Favored
Disfavored
P-value
$10^{-10}$
$10^{-8}$
$10^{-6}$
$10^{-4}$
$10^{-2}$
0
$10^{-2}$
$10^{-4}$
$10^{-6}$
$10^{-8}$
$10^{-10}$
1
2
3
4
5
6
7
8
9
10
11
12
13
14
15
16
17
18
19
20
21
22
23

FIG. 22

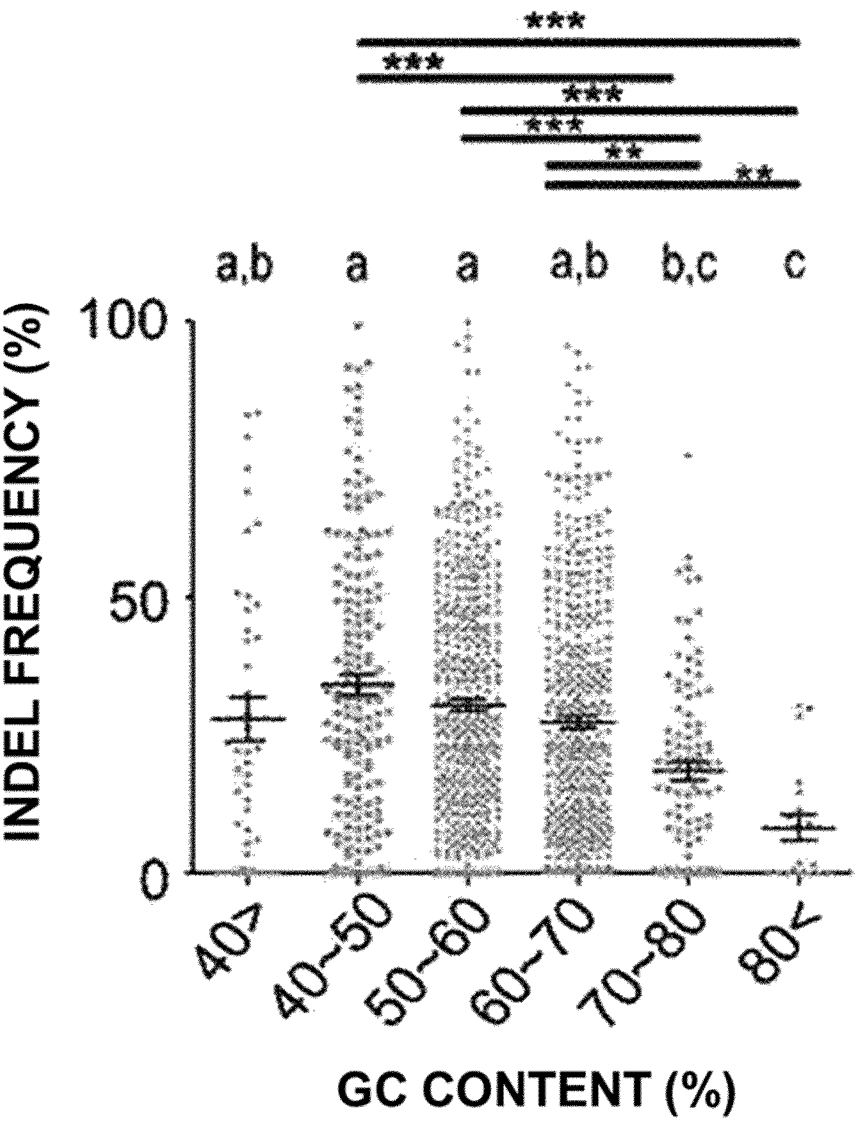
***
***
***
***
**
**
a,b
a
a
a,b
b,c
c
100
50
0
INDEL FREQUENCY (%)
40>
40~50
50~60
60~70
70~80
80<
GC CONTENT (%)

FIG. 23

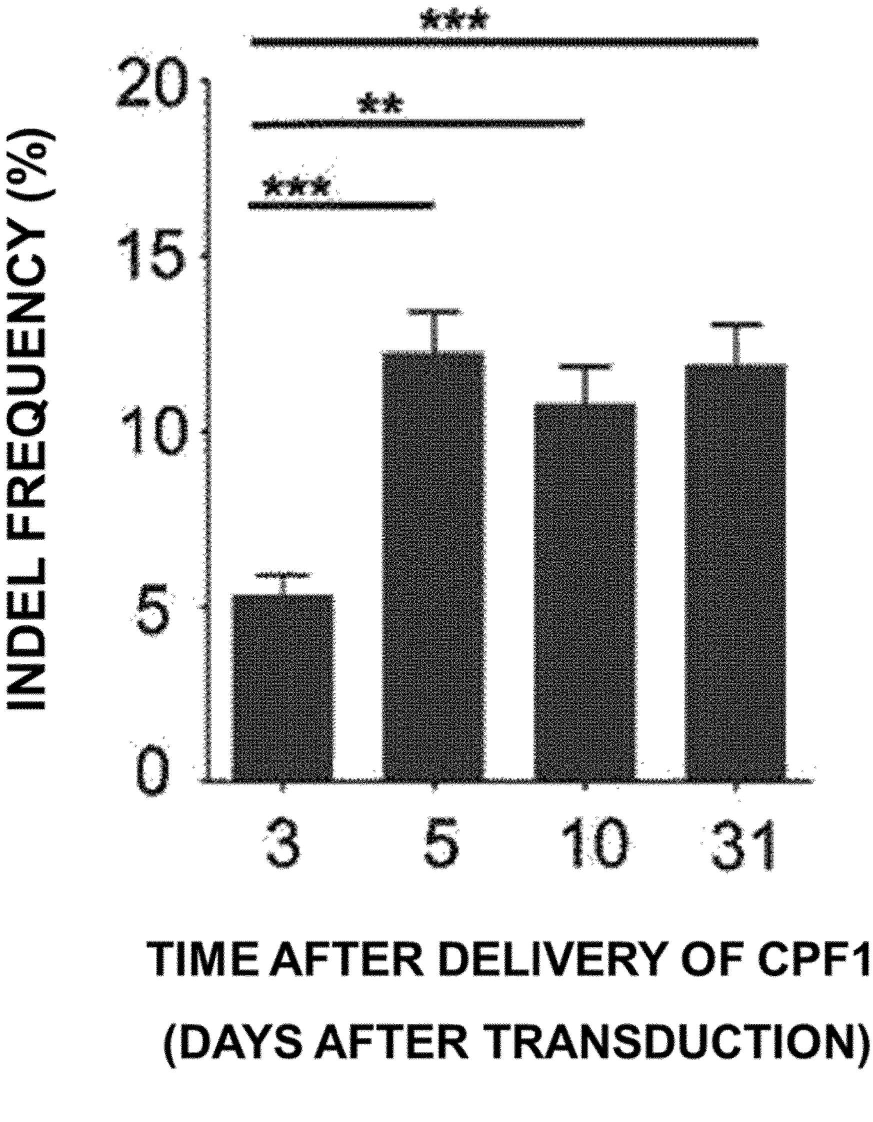
***
**
***
20
15
10
5
0
INDEL FREQUENCY (%)
3
5
10
31
TIME AFTER DELIVERY OF CPF1
(DAYS AFTER TRANSDUCTION)

FIG. 24

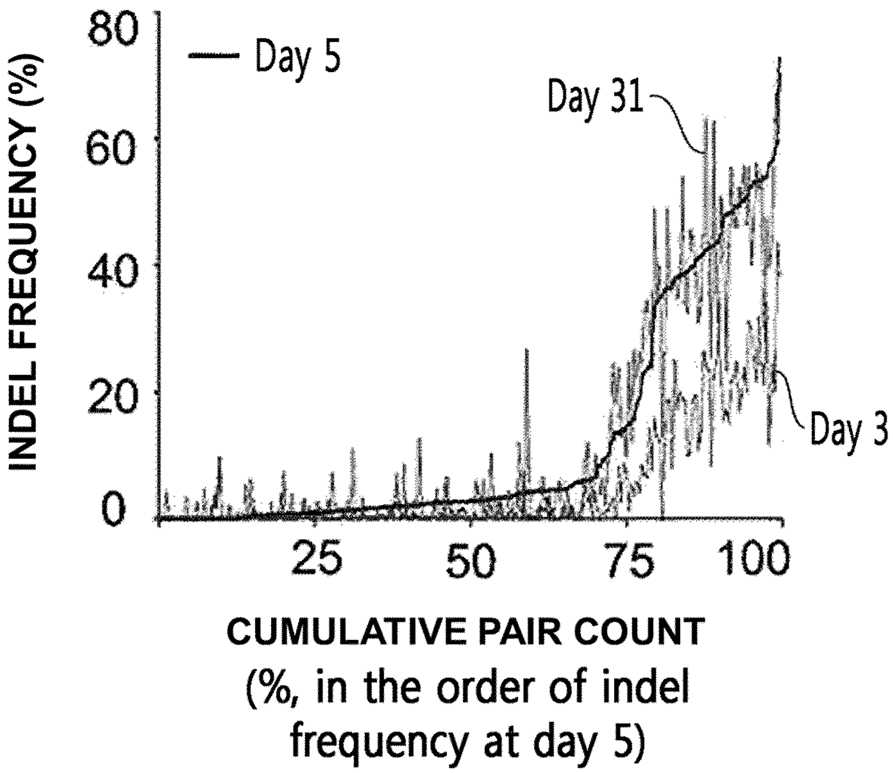
80
60
40
20
0
INDEL FREQUENCY (%)
— Day 5
Day 31
Day 3
25
50
75
100
CUMULATIVE PAIR COUNT
(%, in the order of indel
frequency at day 5)

FIG. 25

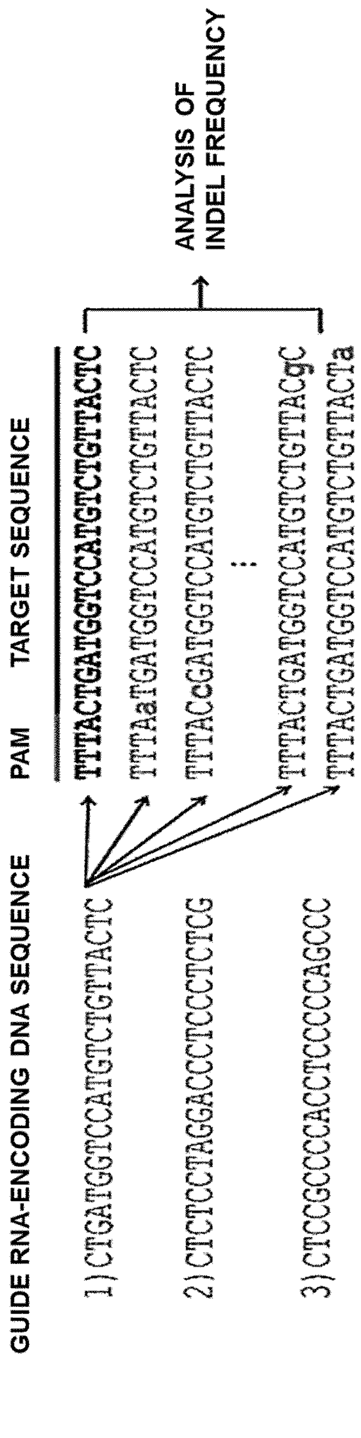
GUIDE RNA-ENCODING DNA SEQUENCE
PAM
TARGET SEQUENCE
1) CTGATGGTCCATGTCTGTTACTC
2) CTCTCCTAGGACCCTCCCTCTCG
3) CTCCGCCCCACCTCCCCCAGCCC
TTTACTGATGGTCCATGTCTGTTACTC
TTTAaTGATGGTCCATGTCTGTTACTC
TTTACcGATGGTCCATGTCTGTTACTC
TTTACTGATGGTCCATGTCTGTTACgC
TTTACTGATGGTCCATGTCTGTTACTa
ANALYSIS OF INDEL FREQUENCY

FIG. 26

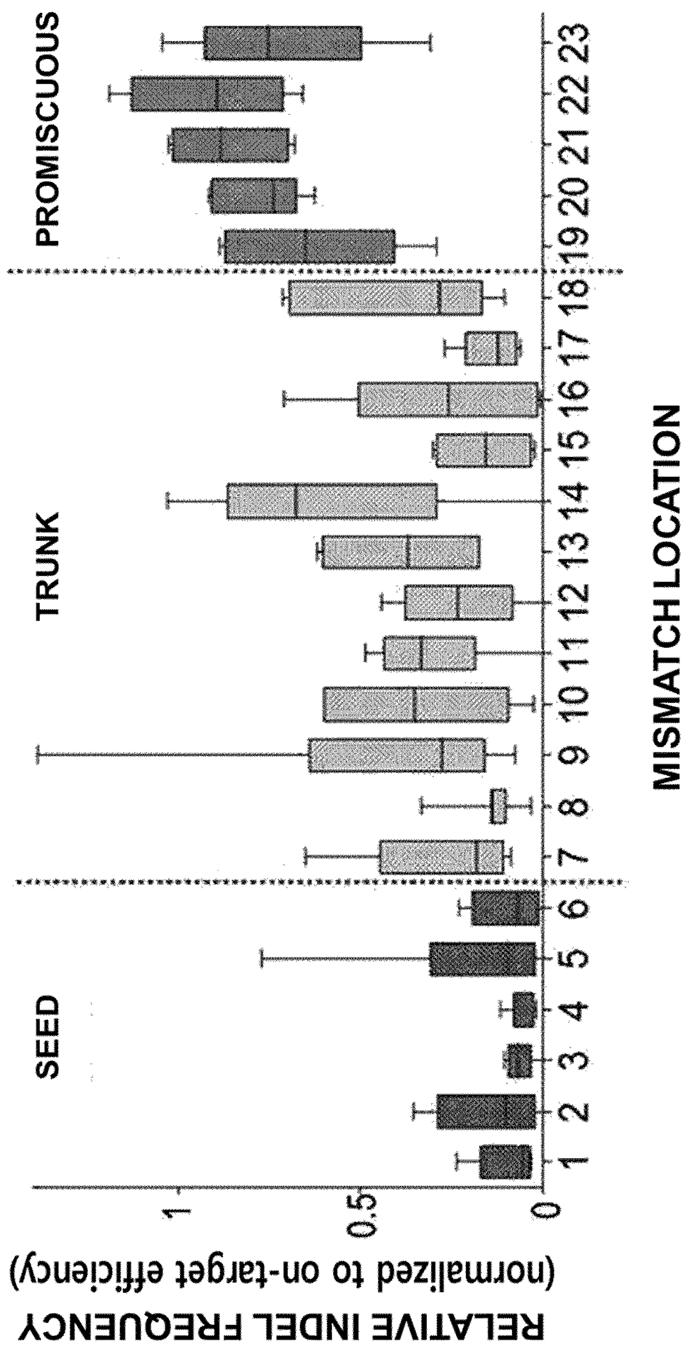
SEED
TRUNK
PROMISCUOUS
RELATIVE INDEL FREQUENCY
(normalized to on-target efficiency)
0
0,5
1
1
2
3
4
5
6
7
8
9
10
11
12
13
14
15
16
17
18
19
20
21
22
23
MISMATCH LOCATION

FIG. 27

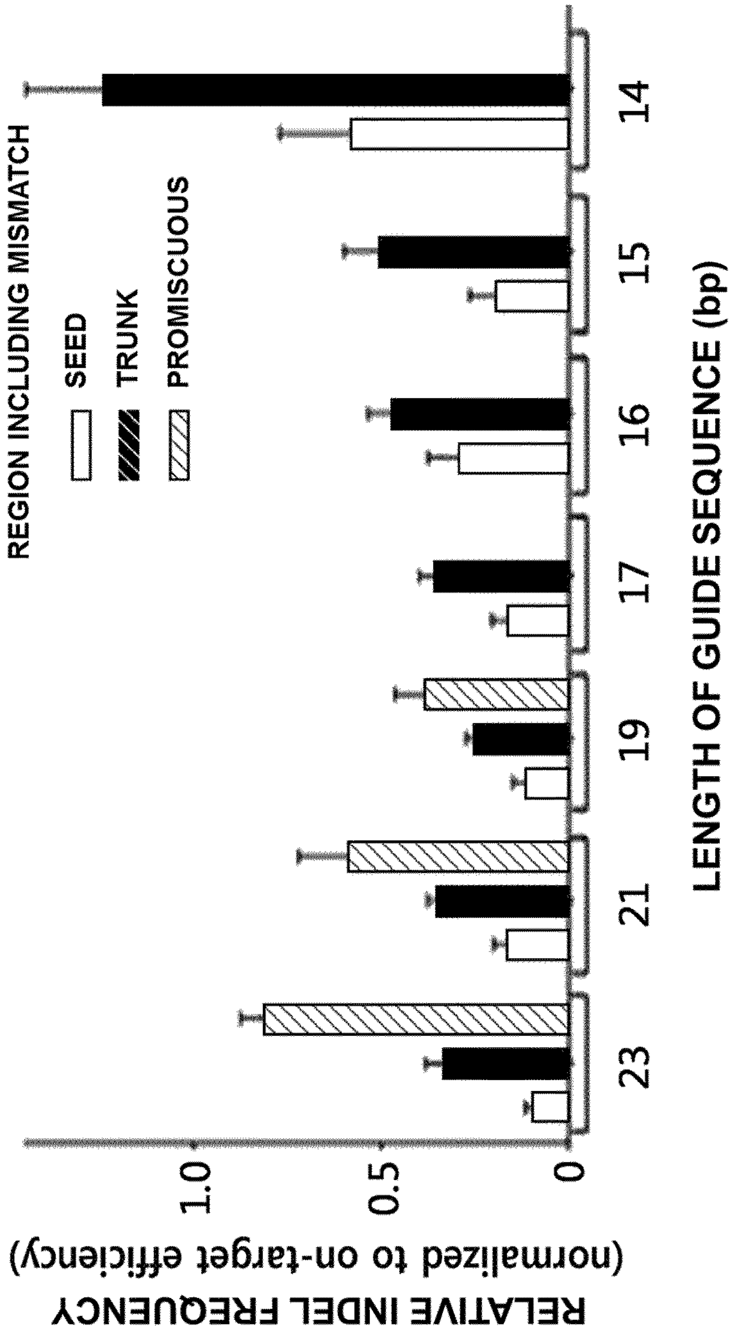
REGION INCLUDING MISMATCH
SEED
TRUNK
PROMISCUOUS
RELATIVE INDEL FREQUENCY
(normalized to on-target efficiency)
1.0
0.5
0
23
21
19
17
16
15
14
LENGTH OF GUIDE SEQUENCE (bp)

FIG. 28

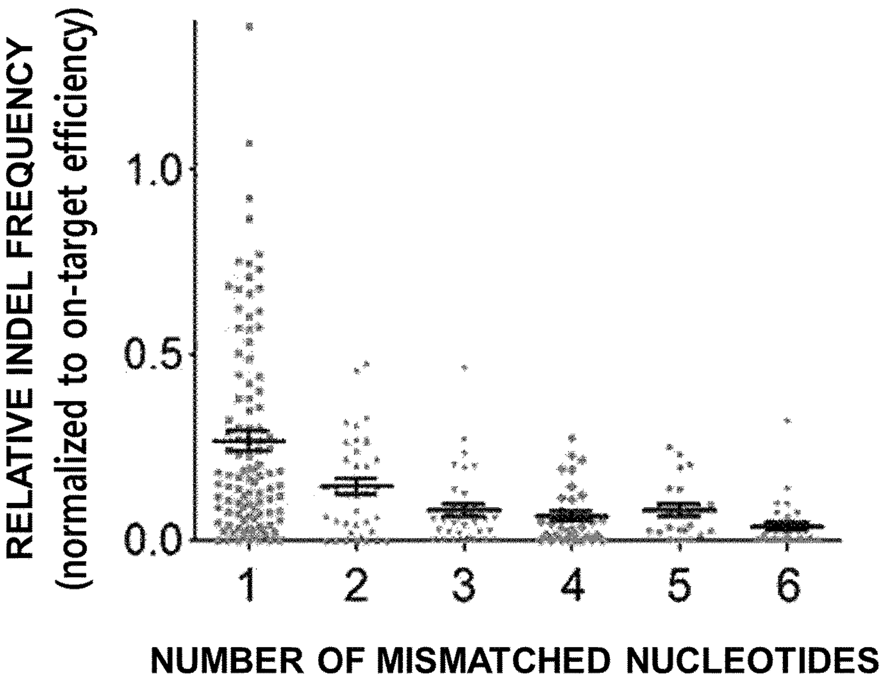
RELATIVE INDEL FREQUENCY
(normalized to on-target efficiency)
1.0
0.5
0.0
1
2
3
4
5
6
NUMBER OF MISMATCHED NUCLEOTIDES

FIG. 29

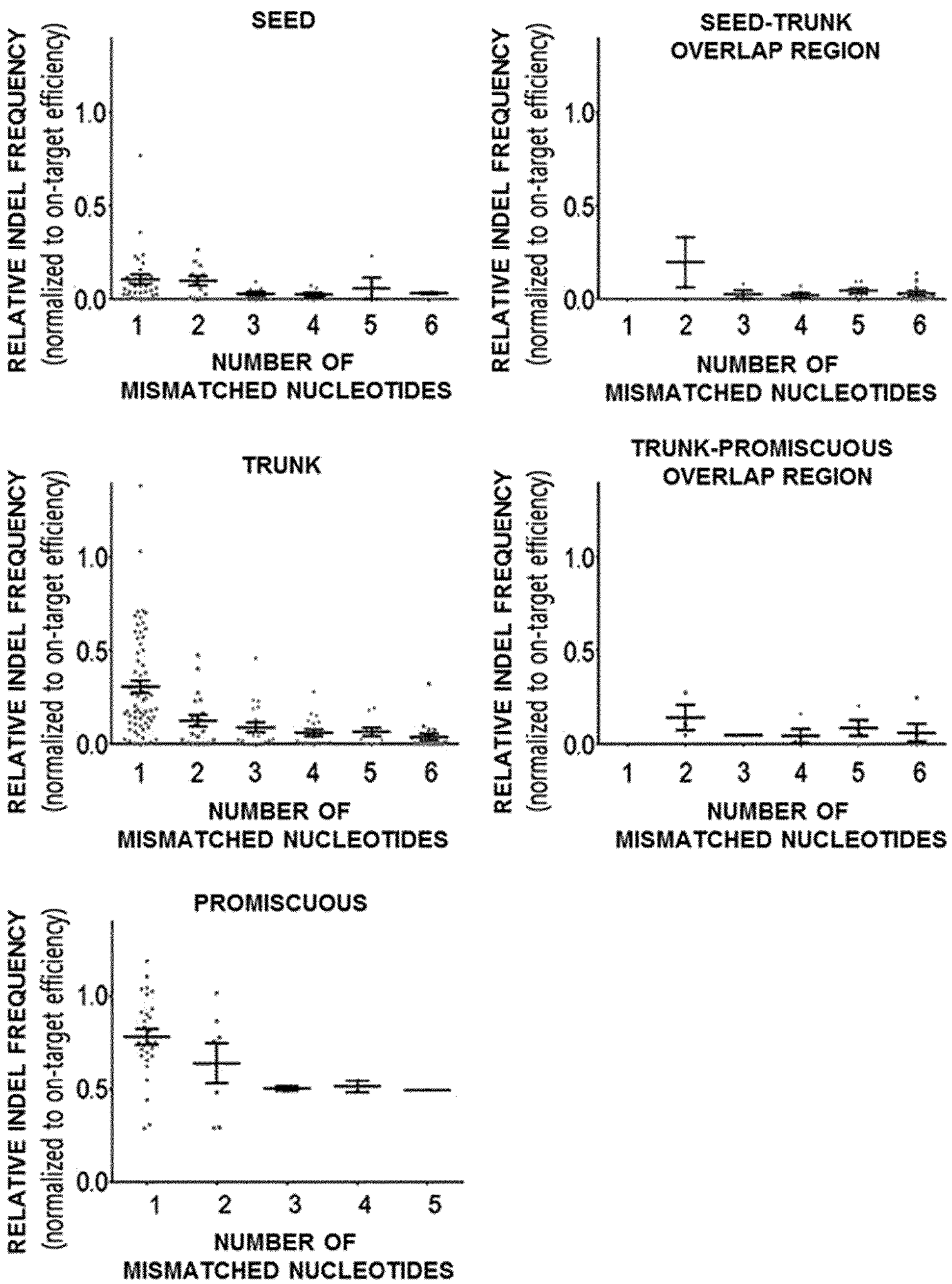
SEED
SEED-TRUNK OVERLAP REGION
TRUNK
TRUNK-PROMISCUOUS OVERLAP REGION
PROMISCUOUS
RELATIVE INDEL FREQUENCY (normalized to on-target efficiency)
NUMBER OF MISMATCHED NUCLEOTIDES
0.0
0.5
1.0
1
2
3
4
5
6

FIG. 30

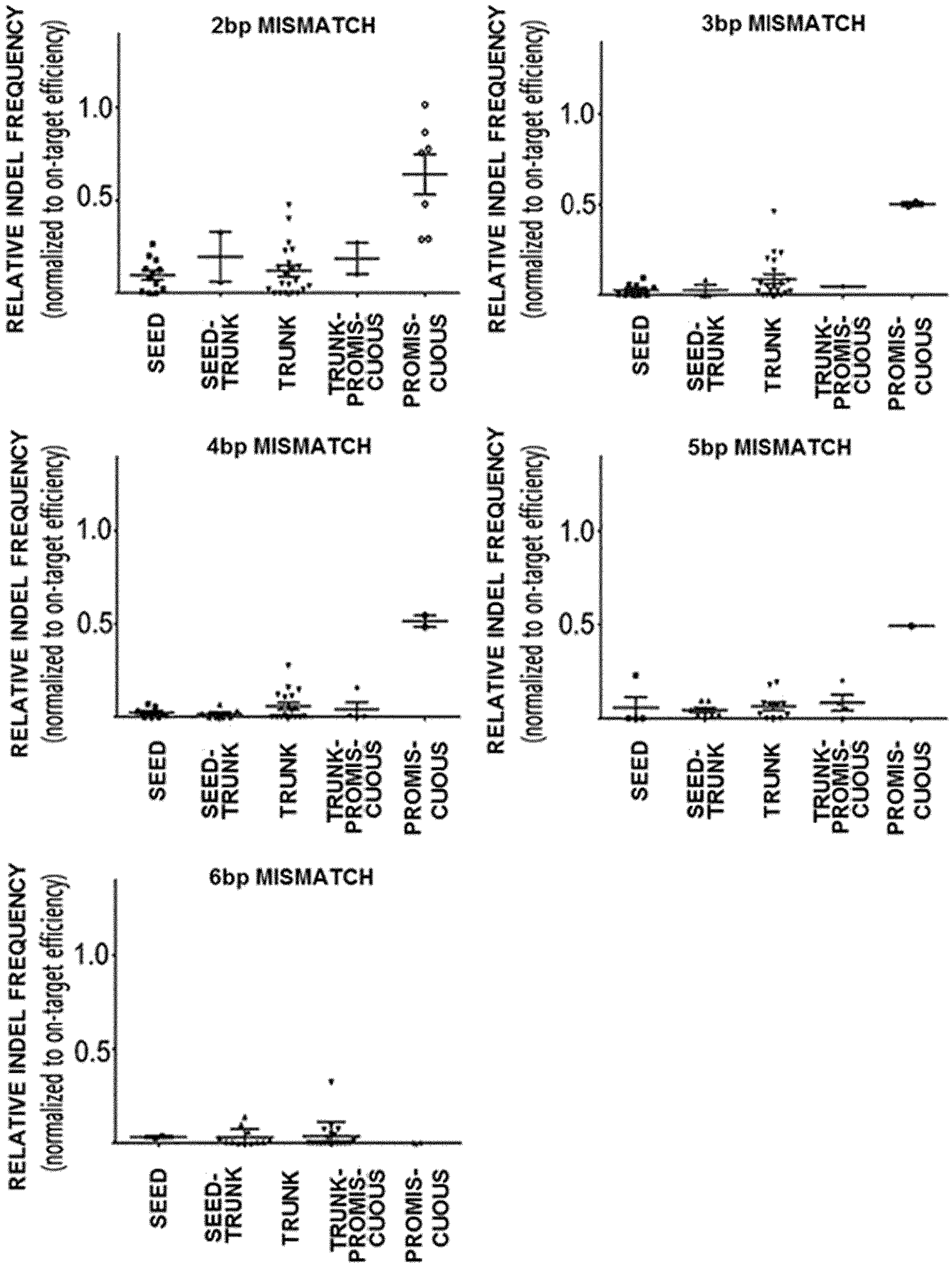
2bp MISMATCH
3bp MISMATCH
4bp MISMATCH
5bp MISMATCH
6bp MISMATCH
RELATIVE INDEL FREQUENCY
(normalized to on-target efficiency)
1.0
0.5
SEED
SEED-TRUNK
TRUNK
TRUNK-PROMIS-CUOUS
PROMIS-CUOUS

FIG. 31

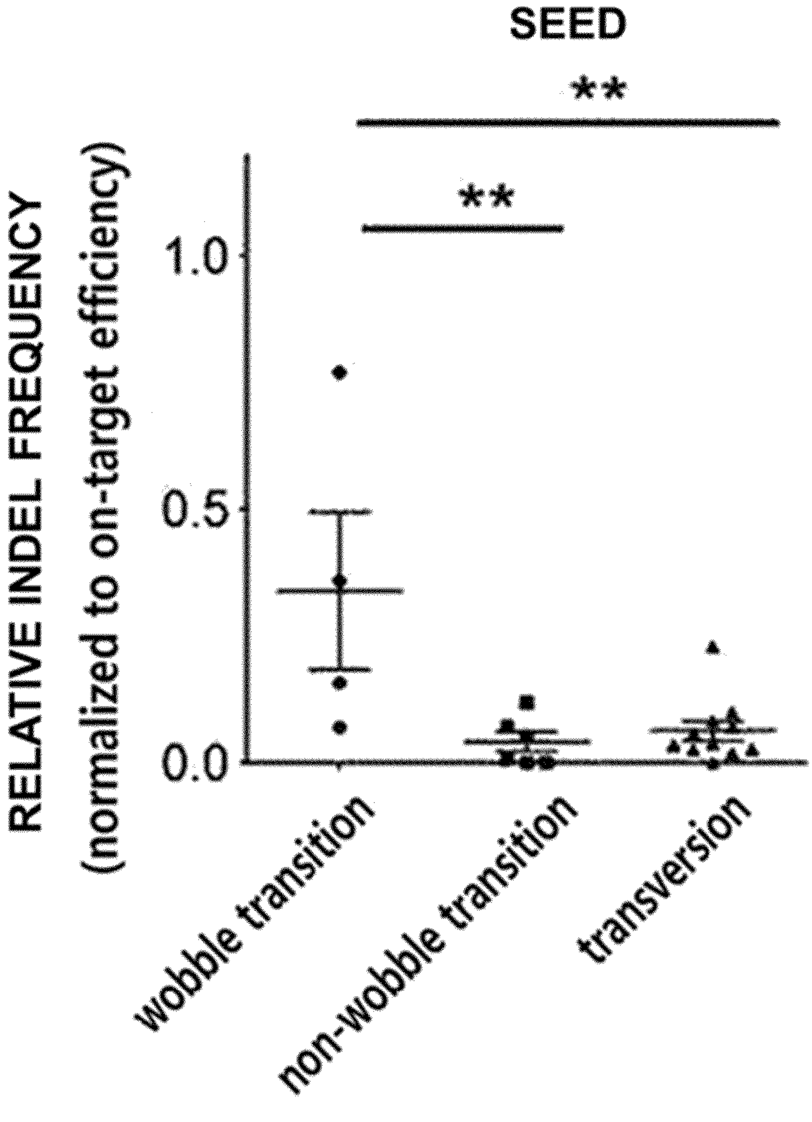
SEED
**
**
RELATIVE INDEL FREQUENCY
(normalized to on-target efficiency)
1.0
0.5
0.0
wobble transition
non-wobble transition
transversion

FIG. 32

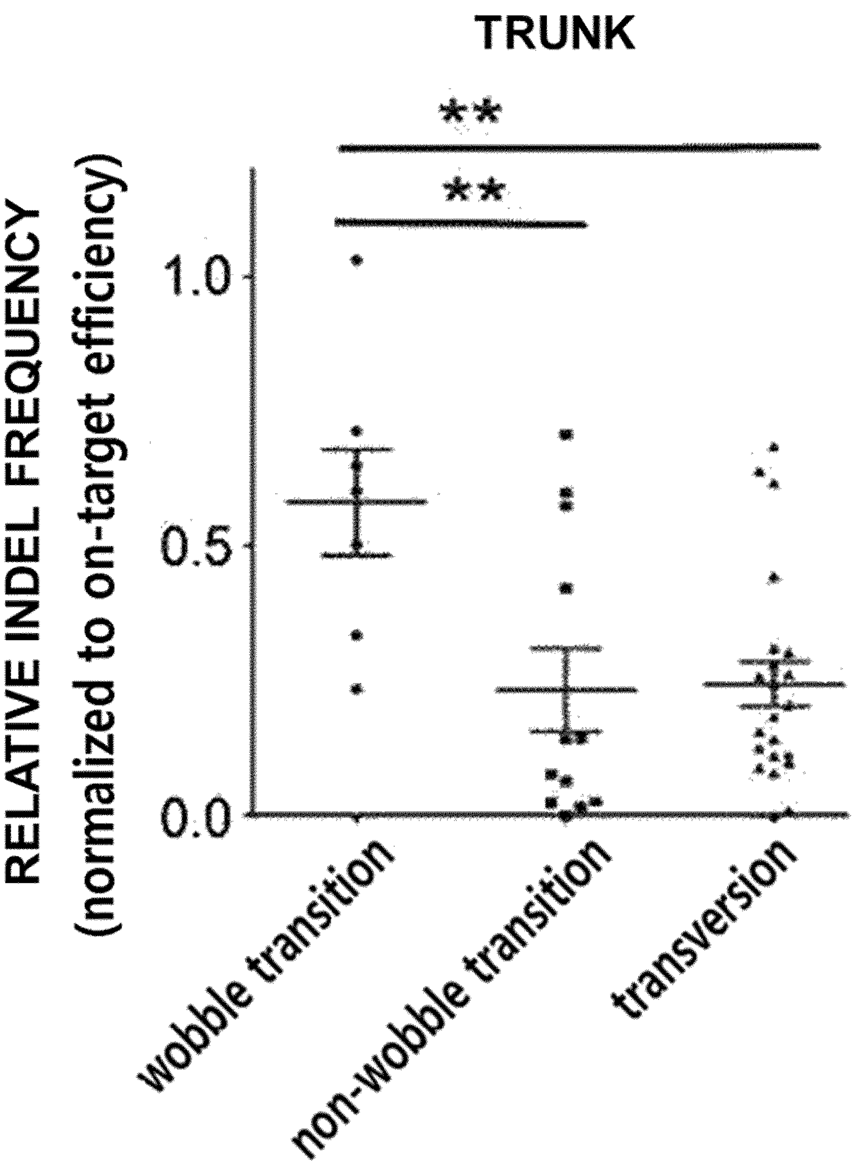
TRUNK
**
**
RELATIVE INDEL FREQUENCY
(normalized to on-target efficiency)
1.0
0.5
0.0
wobble transition
non-wobble transition
transversion

FIG. 33

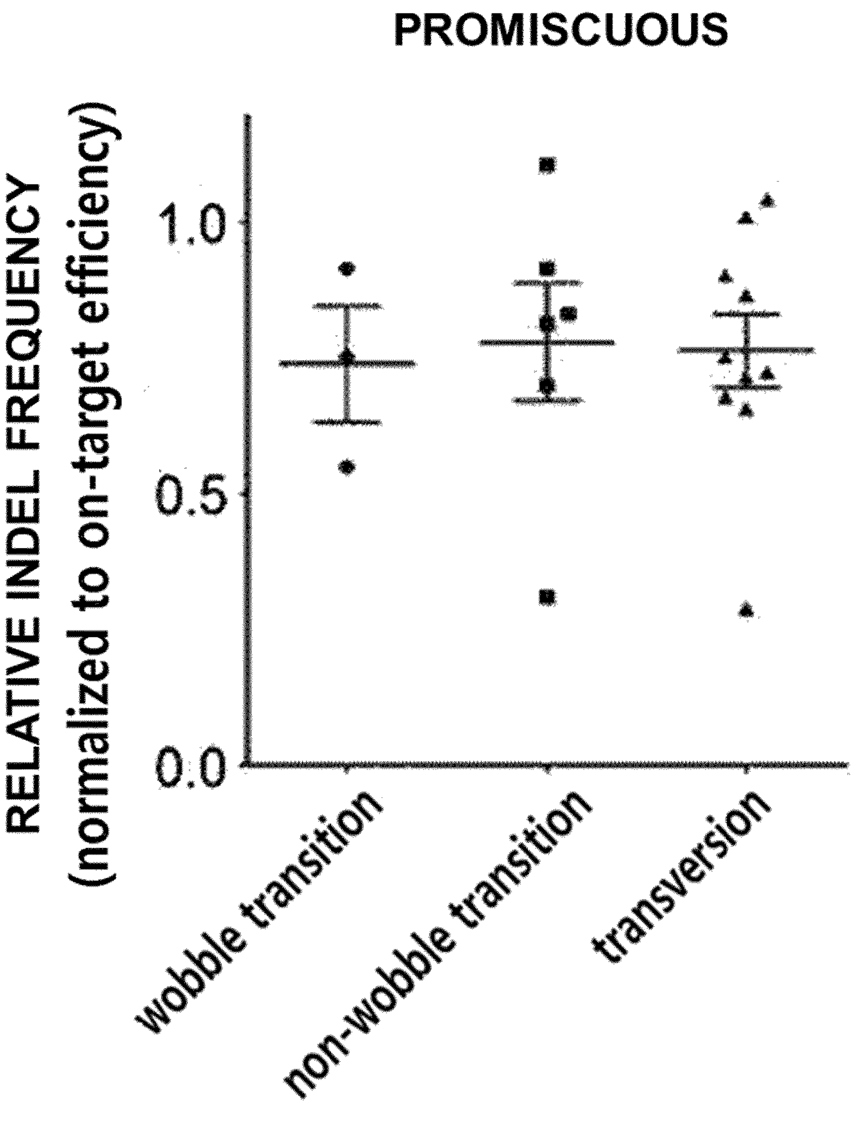
PROMISCUOUS
RELATIVE INDEL FREQUENCY
(normalized to on-target efficiency)
1.0
0.5
0.0
wobble transition
non-wobble transition
transversion

FIG. 34

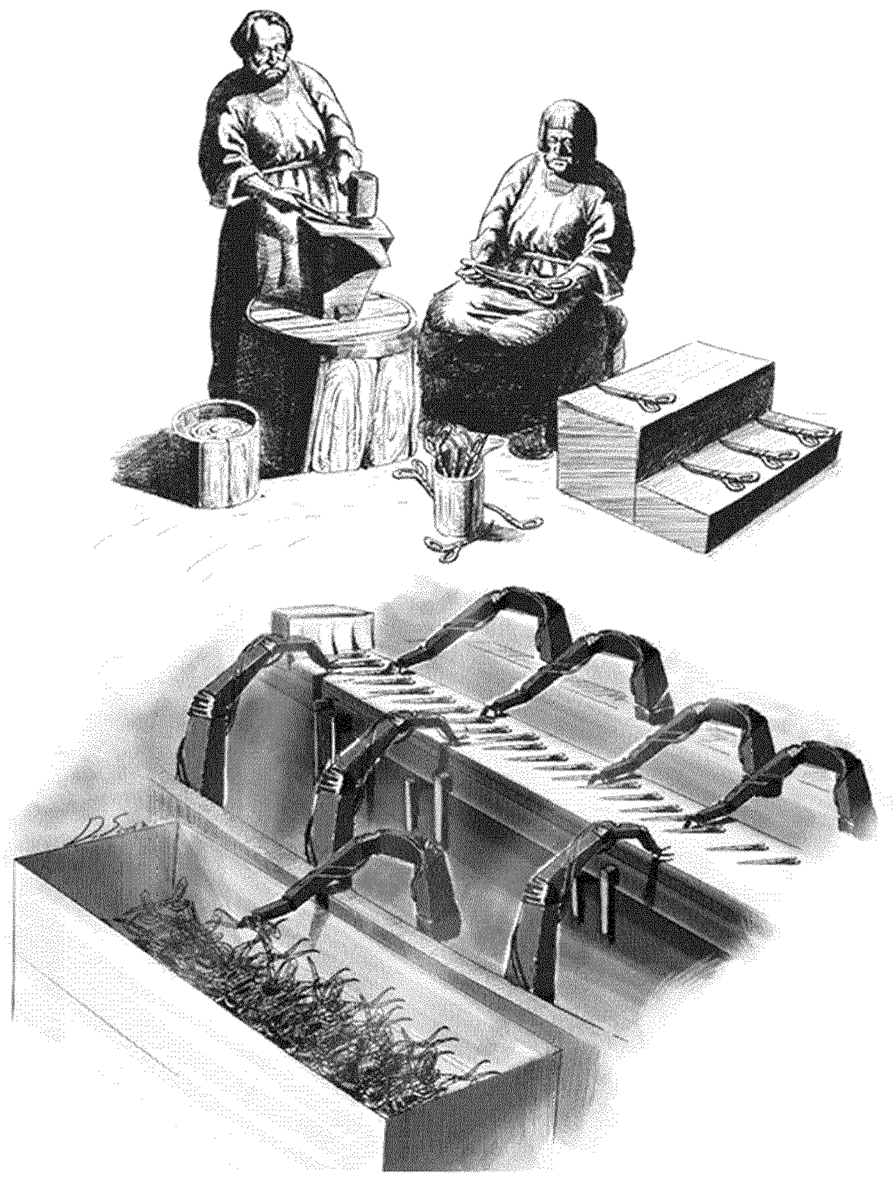

FIG. 35

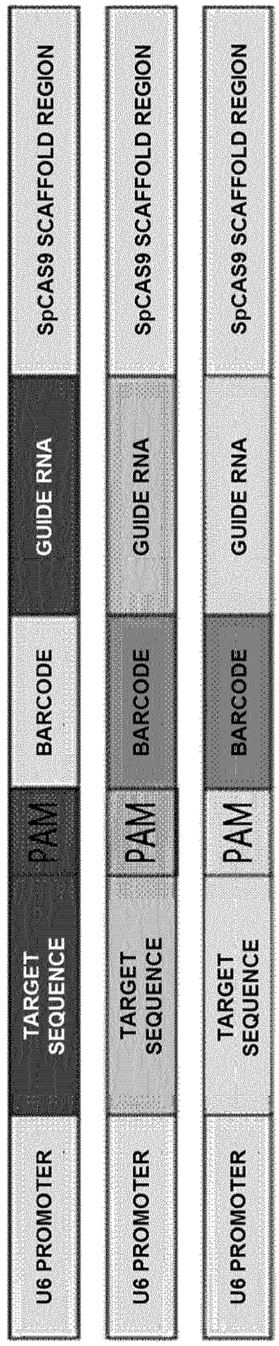
U6 PROMOTER
TARGET SEQUENCE
PAM
BARCODE
GUIDE RNA
SpCAS9 SCAFFOLD REGION
U6 PROMOTER
TARGET SEQUENCE
PAM
BARCODE
GUIDE RNA
SpCAS9 SCAFFOLD REGION
U6 PROMOTER
TARGET SEQUENCE
PAM
BARCODE
GUIDE RNA
SpCAS9 SCAFFOLD REGION

FIG. 36

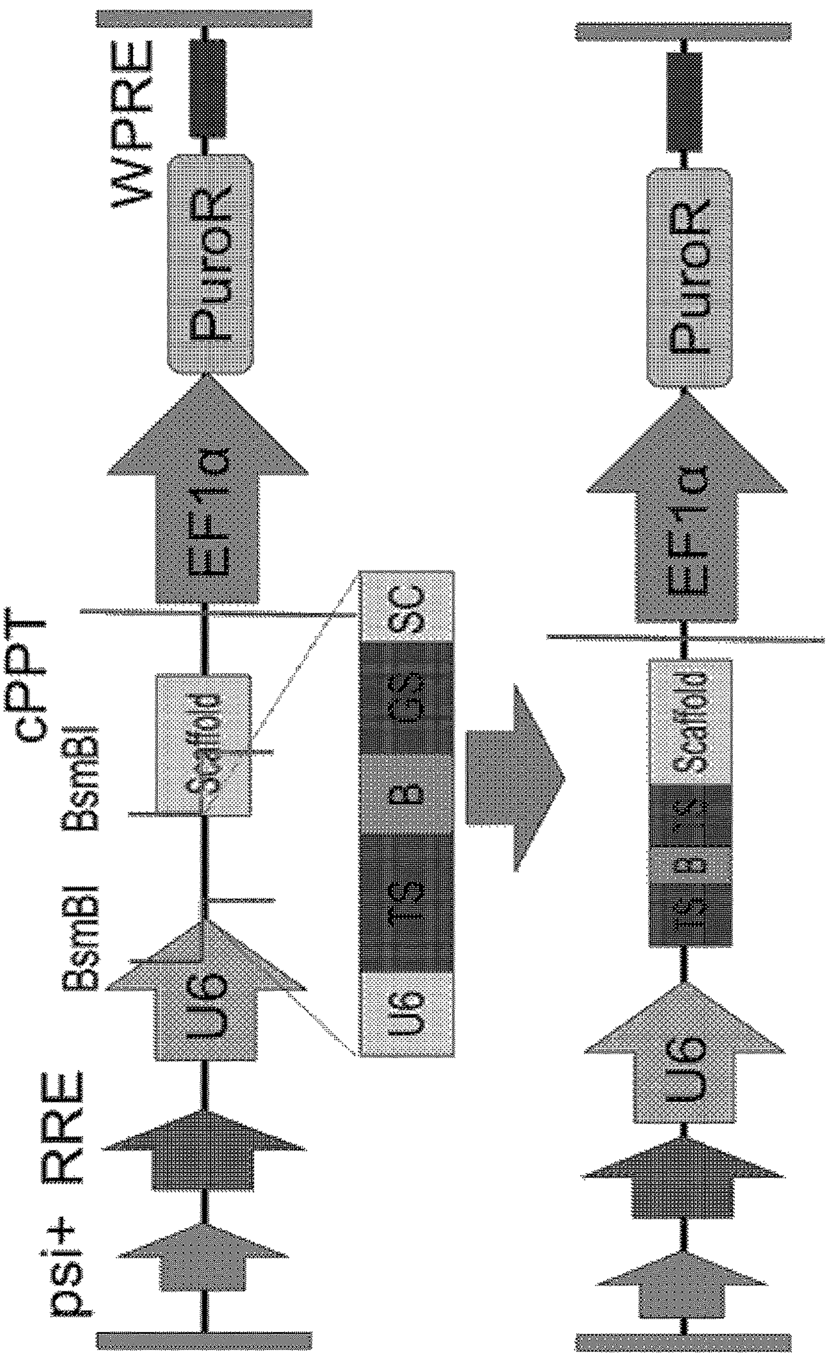
psi+
RRE
U6
BsmBI
BsmBI
Scaffold
cPPT
EF1a
PuroR
WPRE
U6
TS
B
GS
SC
U6
TS
B
TS
Scaffold
EF1a
PuroR

FIG. 37

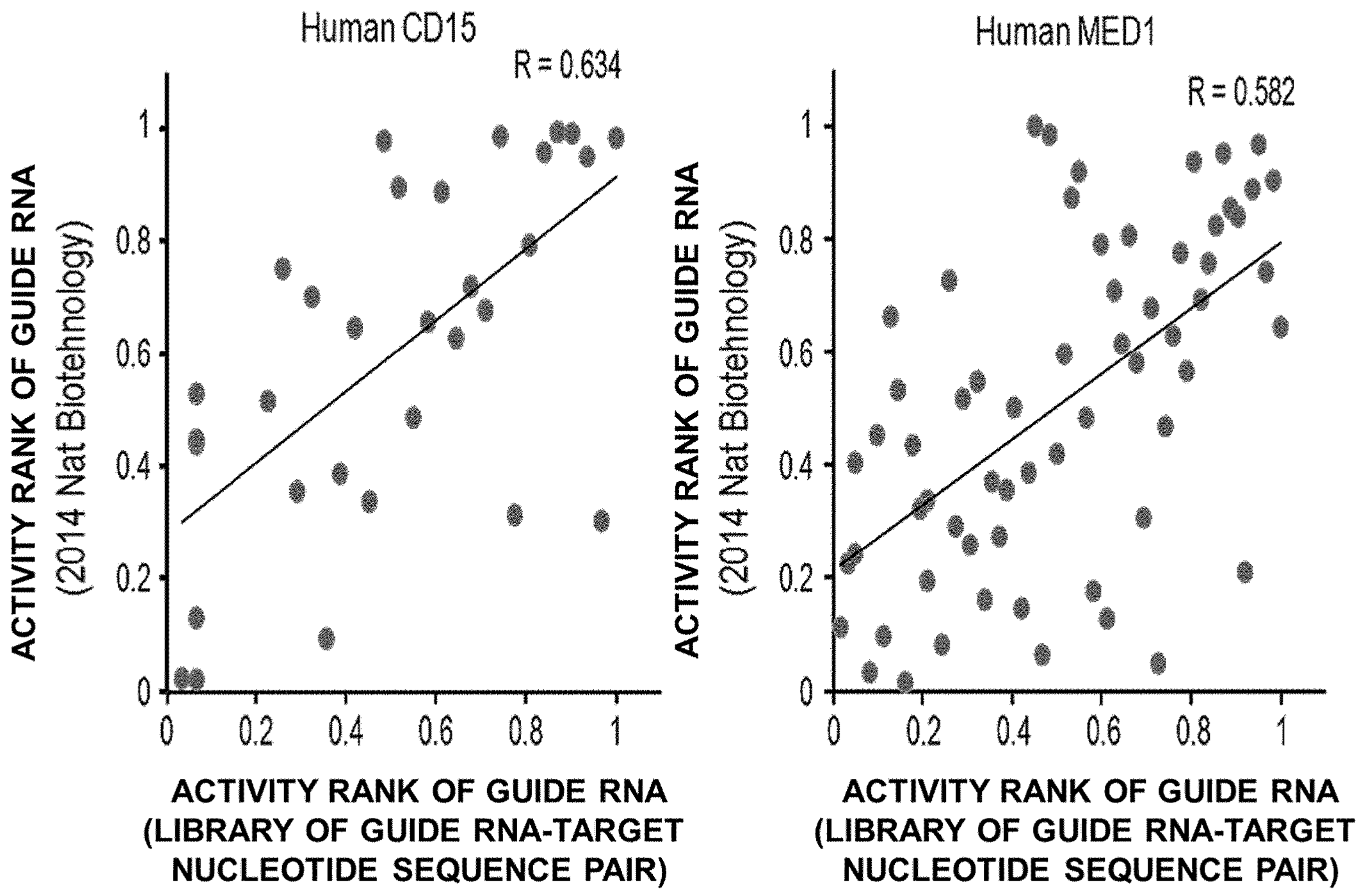
Human CD15
R = 0.634
ACTIVITY RANK OF GUIDE RNA (2014 Nat Biotehnology)
ACTIVITY RANK OF GUIDE RNA (LIBRARY OF GUIDE RNA-TARGET NUCLEOTIDE SEQUENCE PAIR)
Human MED1
R = 0.582
ACTIVITY RANK OF GUIDE RNA (2014 Nat Biotehnology)
ACTIVITY RANK OF GUIDE RNA (LIBRARY OF GUIDE RNA-TARGET NUCLEOTIDE SEQUENCE PAIR)

FIG. 38

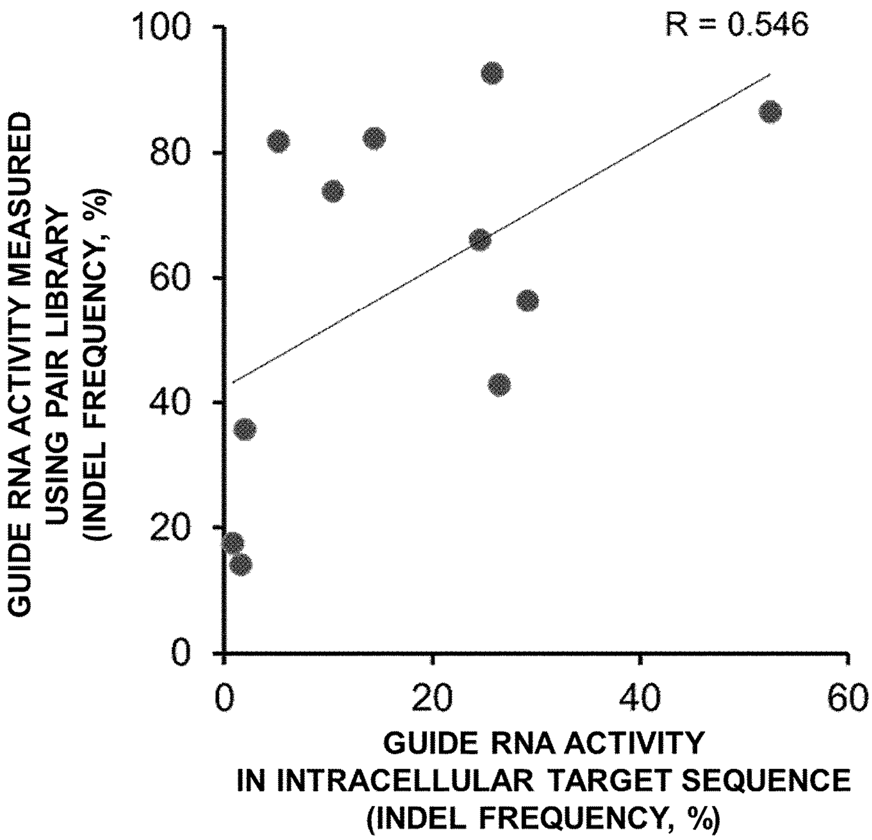
R = 0.546
100
80
60
40
20
0
0
20
40
60
GUIDE RNA ACTIVITY MEASURED
USING PAIR LIBRARY
(INDEL FREQUENCY, %)
GUIDE RNA ACTIVITY
IN INTRACELLULAR TARGET SEQUENCE
(INDEL FREQUENCY, %)

FIG. 39

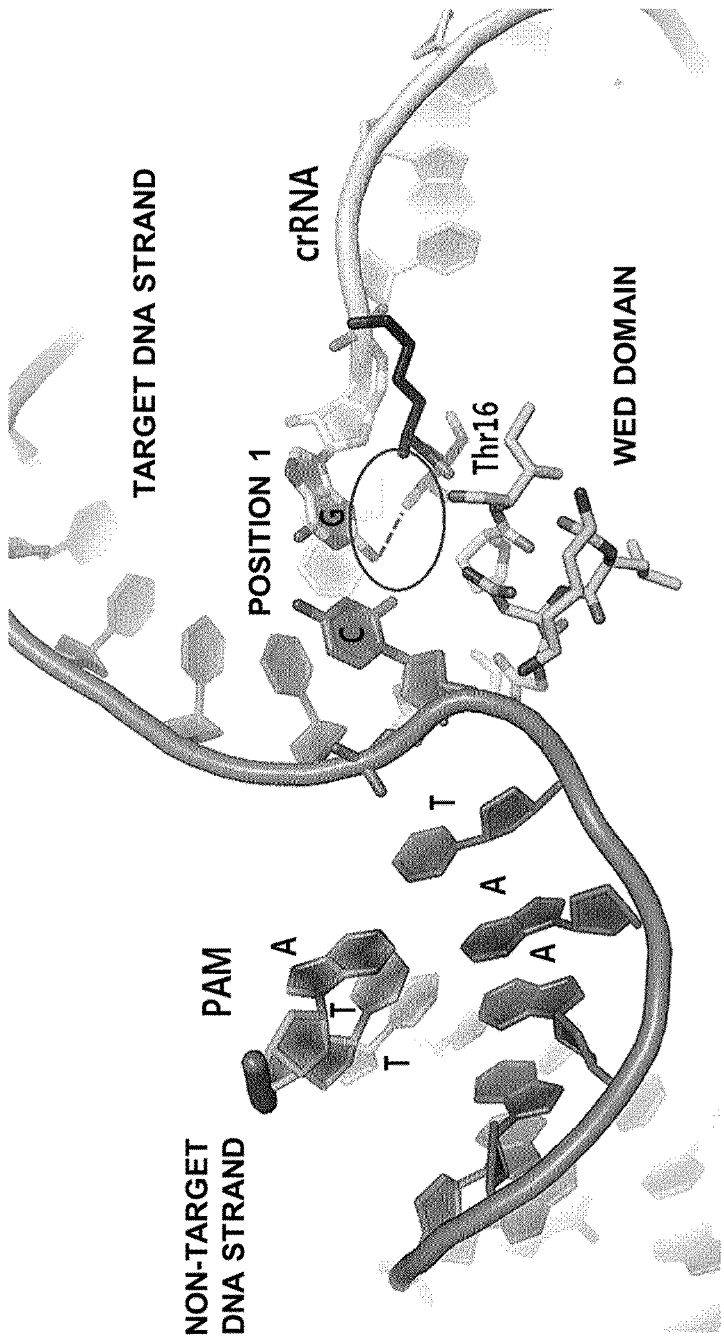
TARGET DNA STRAND
crRNA
WED DOMAIN
Thr16
POSITION 1
G
C
T
A
A
PAM
A
T
T
NON-TARGET
DNA STRAND

FIG. 40

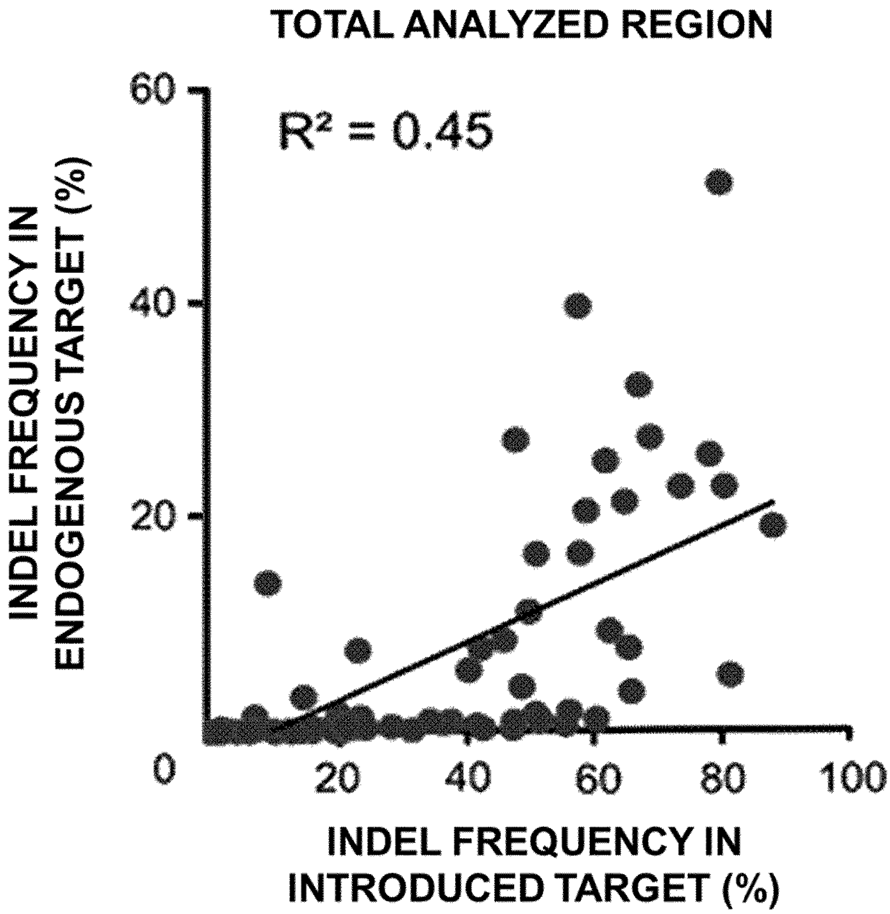
TOTAL ANALYZED REGION
R² = 0.45
INDEL FREQUENCY IN ENDOGENOUS TARGET (%)
60
40
20
0
20
40
60
80
100
INDEL FREQUENCY IN INTRODUCED TARGET (%)

FIG. 41

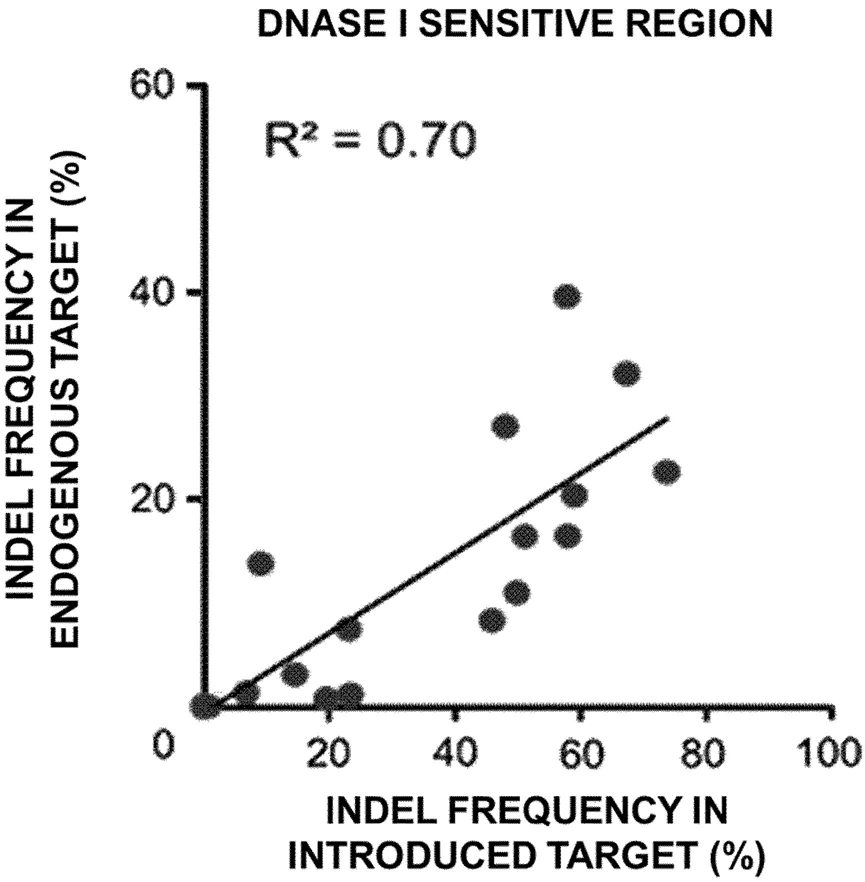
DNASE I SENSITIVE REGION
R² = 0.70
INDEL FREQUENCY IN ENDOGENOUS TARGET (%)
0
20
40
60
20
40
60
80
100
INDEL FREQUENCY IN INTRODUCED TARGET (%)

FIG. 42

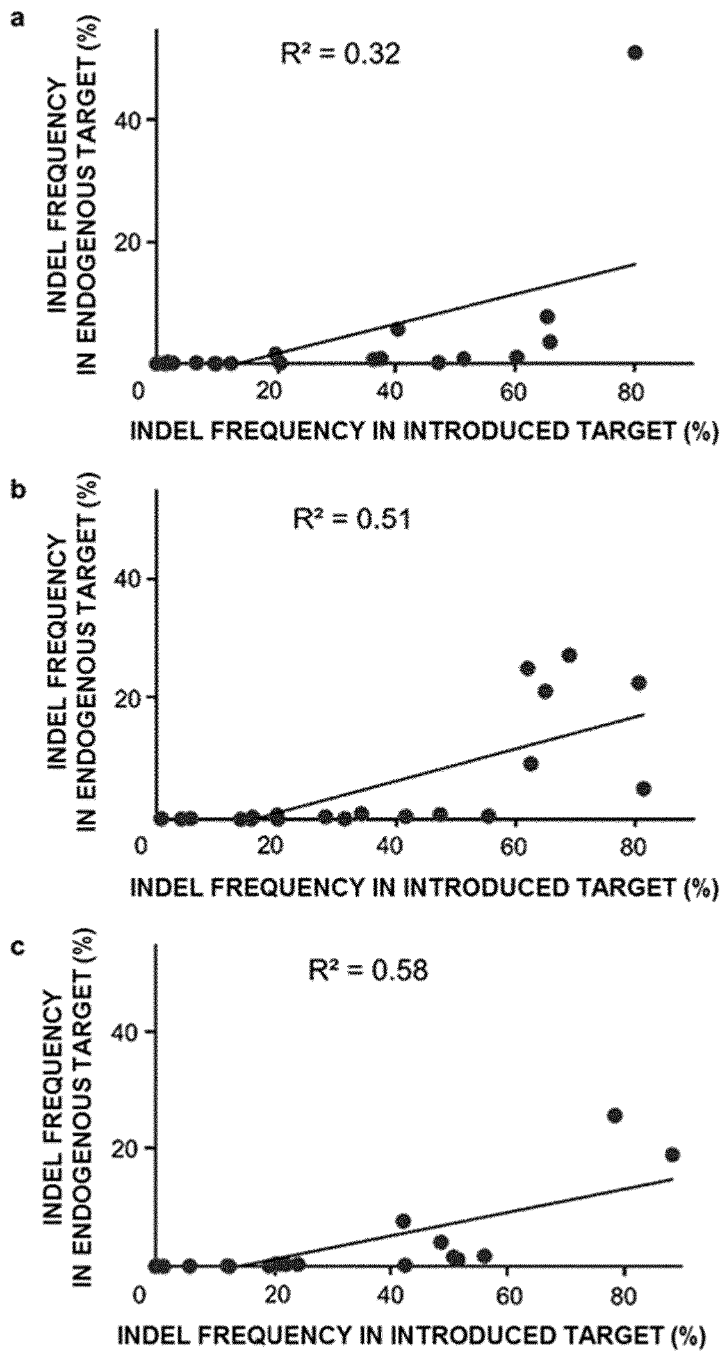
a
R² = 0.32
INDEL FREQUENCY IN ENDOGENOUS TARGET (%)
40
20
0
20
40
60
80
INDEL FREQUENCY IN INTRODUCED TARGET (%)
b
R² = 0.51
INDEL FREQUENCY IN ENDOGENOUS TARGET (%)
40
20
0
20
40
60
80
INDEL FREQUENCY IN INTRODUCED TARGET (%)
c
R² = 0.58
INDEL FREQUENCY IN ENDOGENOUS TARGET (%)
40
20
0
20
40
60
80
INDEL FREQUENCY IN INTRODUCED TARGET (%)

FIG. 43

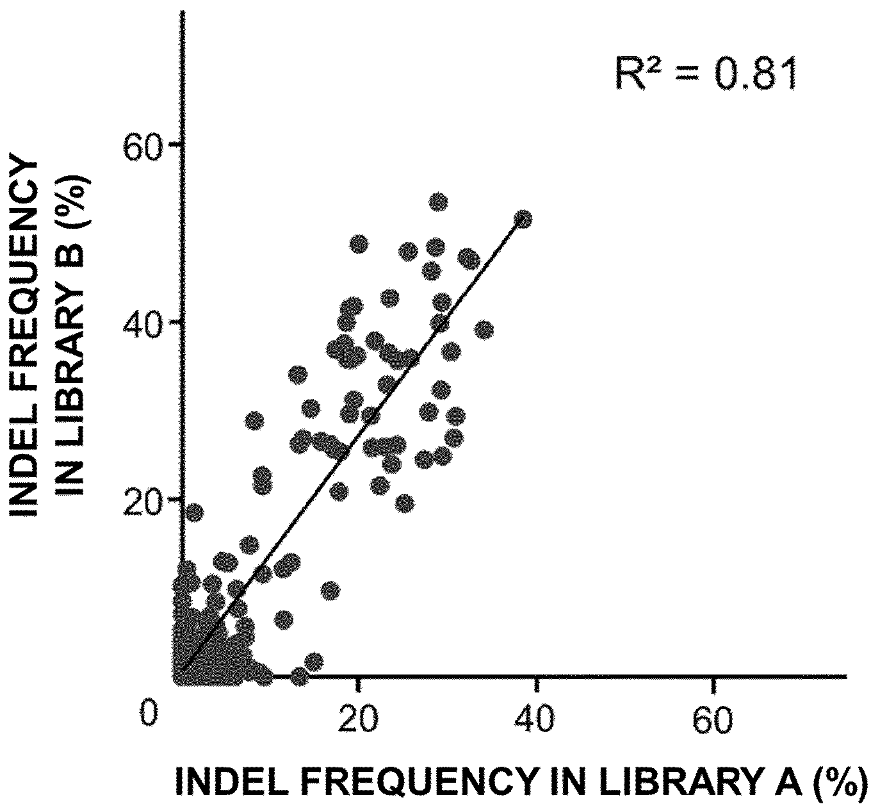
R² = 0.81
60
40
20
0
20
40
60
INDEL FREQUENCY IN LIBRARY B (%)
INDEL FREQUENCY IN LIBRARY A (%)

FIG. 44

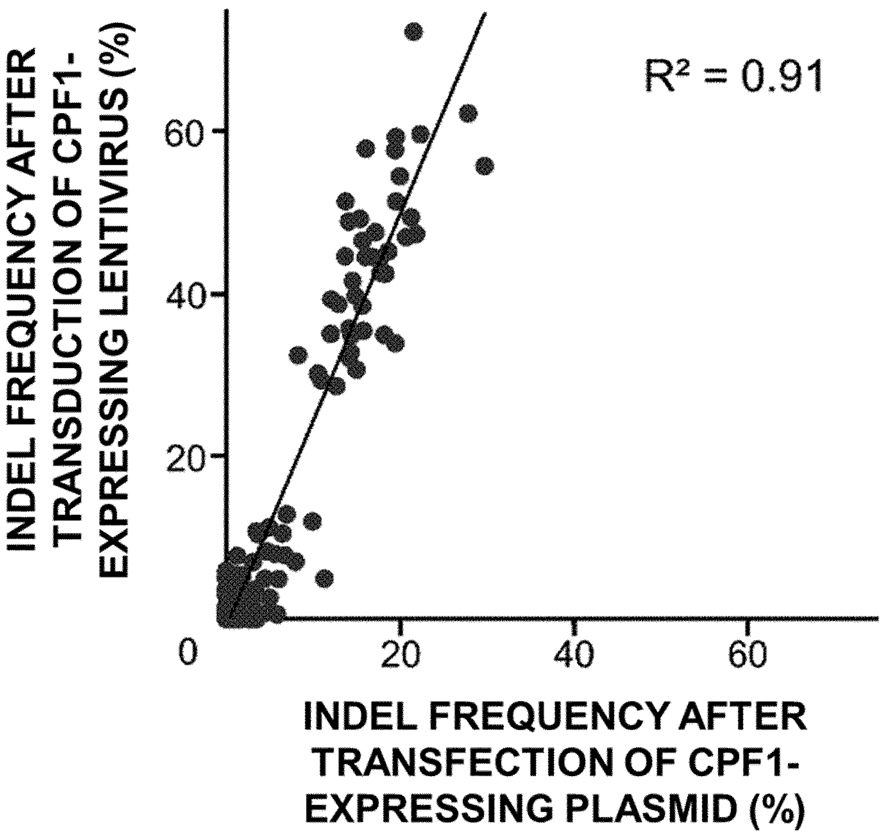
R² = 0.91
INDEL FREQUENCY AFTER TRANSDUCTION OF CPF1-EXPRESSING LENTIVIRUS (%)
0
20
40
60
INDEL FREQUENCY AFTER TRANSFECTION OF CPF1-EXPRESSING PLASMID (%)

FIG. 45

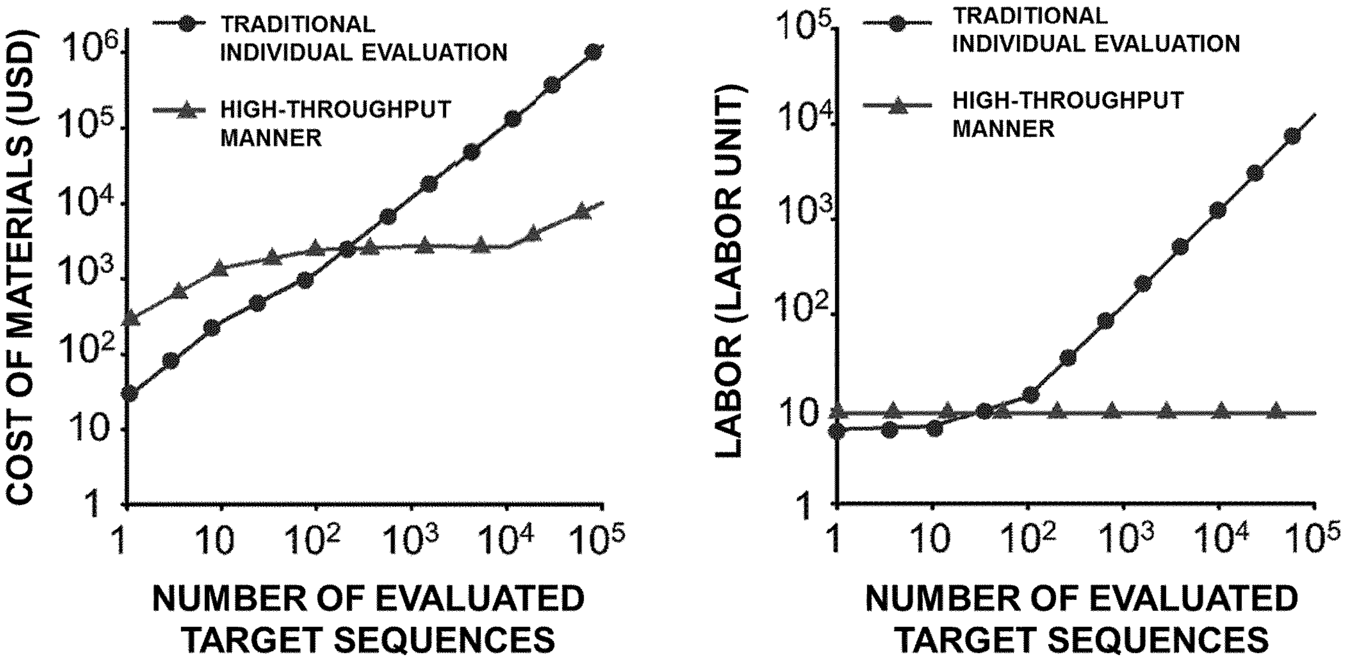
TRADITIONAL INDIVIDUAL EVALUATION
HIGH-THROUGHPUT MANNER
COST OF MATERIALS (USD)
1
10
$10^2$
$10^3$
$10^4$
$10^5$
$10^6$
NUMBER OF EVALUATED TARGET SEQUENCES
TRADITIONAL INDIVIDUAL EVALUATION
HIGH-THROUGHPUT MANNER
LABOR (LABOR UNIT)
1
10
$10^2$
$10^3$
$10^4$
$10^5$
NUMBER OF EVALUATED TARGET SEQUENCES

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3009511 A2
- KR 2017004610 W
- KR 1020160052365


### Non-patent literature cited in the description


- *Cell,* 2014, vol. 157, 1262-1278
- *ACS Synth Biol.,* 10 February 2017
- *Sci Rep,* 2016, vol. 6, 30870
- *Nat Biotechnol,* vol. 34, 184-191
- *Nature,* 2016, vol. 539, 479
- *Cell,* 2015, vol. 163, 759-771
- *Nat Biotechnol,* 2014, vol. 32, 1262-1267
- **PENG et al.** *FEBS Journal,* 2015, vol. 282, 2089-2096
- *Cell,* 2015, vol. 163 (3), 759-71
- *Nat Biotechnol,* 2016, vol. 34, 184-191

<110> Industry-Academic Cooperation Foundation Yonsei University

<120> Method for evaluating, in vivo, activity of RNA-guided nuclease in high-throughput manner

<130> OPP18-054-PCT-US

<140> PCT/KR2017/004610
<141> 2017-04-28

<150> KR 10-2016-0052365
<151> 2016-04-28

<160> 66

<170> KoPatentIn 3.0

<210> 1
<211> 27
<212> DNA
<213> Artificial Sequence

<220>
<223> 5’ end of Oligonucleotide sequence

<400> 1
aacaccgtaa tttctactct tgtagat 27

<210> 2
<211> 22
<212> DNA
<213> Artificial Sequence

<220>
<223> 3’ end of Oligonucleotide sequence

<400> 2
agcttggcgt aactagatct tg 22

<210> 3
<211> 12669
<212> DNA
<213> Artificial Sequence

<220>
<223> Lenti_AsCpf1-Blast

<400> 3

```
gtcgacggat cgggagatct cccgatcccc tatggtgcac tctcagtaca atctgctctg        60
atgccgcata gttaagccag tatctgctcc ctgcttgtgt gttggaggtc gctgagtagt       120
gcgcgagcaa aatttaagct acaacaaggc aaggcttgac cgacaattgc atgaagaatc       180
tgcttagggt taggcgtttt gcgctgcttc gcgatgtacg ggccagatat acgcgttgac       240
attgattatt gactagttat taatagtaat caattacggg gtcattagtt catagcccat       300
```

atatggagtt ccgcgttaca taacttacgg taaatggccc gcctggctga ccgcccaacg 360
acccccgccc attgacgtca ataatgacgt atgttcccat agtaacgcca atagggactt 420
tccattgacg tcaatgggtg gagtatttac ggtaaactgc ccacttggca gtacatcaag 480
tgtatcatat gccaagtacg ccccctattg acgtcaatga cggtaaatgg cccgcctggc 540
attatgccca gtacatgacc ttatgggact ttcctacttg gcagtacatc tacgtattag 600
tcatcgctat taccatggtg atgcggtttt ggcagtacat caatgggcgt ggatagcggt 660
ttgactcacg gggatttcca agtctccacc ccattgacgt caatgggagt ttgttttggc 720
accaaaatca acgggacttt ccaaaatgtc gtaacaactc cgccccattg acgcaaatgg 780
gcggtaggcg tgtacggtgg gaggtctata taagcagcgc gttttgcctg tactgggtct 840
ctctggttag accagatctg agcctgggag ctctctggct aactagggaa cccactgctt 900
aagcctcaat aaagcttgcc ttgagtgctt caagtagtgt gtgcccgtct gttgtgtgac 960
tctggtaact agagatccct cagacccttt tagtcagtgt ggaaaatctc tagcagtggc 1020
gcccgaacag ggacttgaaa gcgaaaggga aaccagagga gctctctcga cgcaggactc 1080
ggcttgctga agcgcgcacg gcaagaggcg aggggcggcg actggtgagt acgccaaaaa 1140
ttttgactag cggaggctag aaggagagag atgggtgcga gagcgtcagt attaagcggg 1200
ggagaattag atcgcgatgg gaaaaaattc ggttaaggcc agggggaaag aaaaaatata 1260
aattaaaaca tatagtatgg gcaagcaggg agctagaacg attcgcagtt aatcctggcc 1320
tgttagaaac atcagaaggc tgtagacaaa tactgggaca gctacaacca tcccttcaga 1380
caggatcaga agaacttaga tcattatata atacagtagc aaccctctat tgtgtgcatc 1440
aaaggataga gataaaagac accaaggaag ctttagacaa gatagaggaa gagcaaaaca 1500
aaagtaagac caccgcacag caagcggccg ctgatcttca gacctggagg aggagatatg 1560
agggacaatt ggagaagtga attatataaa tataaagtag taaaaattga accattagga 1620
gtagcaccca ccaaggcaaa gagaagagtg gtgcagagag aaaaaagagc agtgggaata 1680
ggagctttgt tccttgggtt cttgggagca gcaggaagca ctatgggcgc agcgtcaatg 1740
acgctgacgg tacaggccag acaattattg tctggtatag tgcagcagca gaacaatttg 1800
ctgagggcta ttgaggcgca acagcatctg ttgcaactca cagtctgggg catcaagcag 1860
ctccaggcaa gaatcctggc tgtggaaaga tacctaaagg atcaacagct cctggggatt 1920
tggggttgct ctggaaaact catttgcacc actgctgtgc cttggaatgc tagttggagt 1980
aataaatctc tggaacagat ttggaatcac acgacctgga tggagtggga cagagaaatt 2040
aacaattaca caagcttaat acactcctta attgaagaat cgcaaaacca gcaagaaaag 2100
aatgaacaag aattattgga attagataaa tgggcaagtt tgtggaattg gtttaacata 2160
acaaattggc tgtggtatat aaaattattc ataatgatag taggaggctt ggtaggttta 2220

agaatagttt ttgctgtact ttctatagtg aatagagtta ggcagggata ttcaccatta 2280

tcgtttcaga cccacctccc aaccccgagg ggacccgaca ggcccgaagg aatagaagaa 2340

gaaggtggag agagagacag agacagatcc attcgattag tgaacggatc ggcactgcgt 2400

gcgccaattc tgcagacaaa tggcagtatt catccacaat tttaaaagaa aaggggggat 2460

tggggggtac agtgcagggg aaagaatagt agacataata gcaacagaca tacaaactaa 2520

agaattacaa aaacaaatta caaaaattca aaattttcgg gtttattaca gggacagcag 2580

agatccagtt tggttaatta gctagctagg tcttgaaagg agtgggaatt ggctccggtg 2640

cccgtcagtg ggcagagcgc acatcgccca cagtccccga gaagttgggg ggaggggtcg 2700

gcaattgatc cggtgcctag agaaggtggc gcggggtaaa ctgggaaagt gatgtcgtgt 2760

actggctccg cctttttccc gagggtgggg gagaaccgta tataagtgca gtagtcgccg 2820

tgaacgttct ttttcgcaac gggtttgccg ccagaacaca ggaccggttc tagcgtttaa 2880

acttaagctt ggtaccgcca ccatgacaca gttcgagggc tttaccaacc tgtatcaggt 2940

gagcaagaca ctgcggtttg agctgatccc acagggcaag accctgaagc acatccagga 3000

gcagggcttc atcgaggagg acaaggcccg caatgatcac tacaaggagc tgaagcccat 3060

catcgatcgg atctacaaga cctatgccga ccagtgcctg cagctggtgc agctggattg 3120

ggagaacctg agcgccgcca tcgactccta tagaaaggag aaaaccgagg agacaaggaa 3180

cgccctgatc gaggagcagg ccacatatcg caatgccatc cacgactact tcatcggccg 3240

gacagacaac ctgaccgatg ccatcaataa gagacacgcc gagatctaca agggcctgtt 3300

caaggccgag ctgtttaatg gcaaggtgct gaagcagctg ggcaccgtga ccacaaccga 3360

gcacgagaac gccctgctgc ggagcttcga caagtttaca acctacttct ccggctttta 3420

tgagaacagg aagaacgtgt tcagcgccga ggatatcagc acagccatcc cacaccgcat 3480

cgtgcaggac aacttcccca agtttaagga gaattgtcac atcttcacac gcctgatcac 3540

cgccgtgccc agcctgcggg agcactttga gaacgtgaag aaggccatcg gcatcttcgt 3600

gagcacctcc atcgaggagg tgttttcctt ccctttttat aaccagctgc tgacacagac 3660

ccagatcgac ctgtataacc agctgctggg aggaatctct cgggaggcag gcaccgagaa 3720

gatcaagggc ctgaacgagg tgctgaatct ggccatccag aagaatgatg agacagccca 3780

catcatcgcc tccctgccac acagattcat cccccctgttt aagcagatcc tgtccgatag 3840

gaacaccctg tctttcatcc tggaggagtt taagagcgac gaggaagtga tccagtcctt 3900

ctgcaagtac aagacactgc tgagaaacga gaacgtgctg gagacagccg aggccctgtt 3960

taacgagctg aacagcatcg acctgacaca catcttcatc agccacaaga agctggagac 4020

aatcagcagc gccctgtgcg accactggga tacactgagg aatgccctgt atgagcggag 4080

aatctccgag ctgacaggca agatcaccaa gtctgccaag gagaaggtgc agcgcagcct 4140
gaagcacgag gatatcaacc tgcaggagat catctctgcc gcaggcaagg agctgagcga 4200
ggccttcaag cagaaaacca gcgagatcct gtcccacgca cacgccgccc tggatcagcc 4260
actgcctaca accctgaaga agcaggagga gaaggagatc ctgaagtctc agctggacag 4320
cctgctgggc ctgtaccacc tgctggactg gtttgccgtg gatgagtcca acgaggtgga 4380
ccccgagttc tctgcccggc tgaccggcat caagctggag atggagcctt ctctgagctt 4440
ctacaacaag gccagaaatt atgccaccaa gaagccctac tccgtggaga agttcaagct 4500
gaactttcag atgcctacac tggcctctgg ctgggacgtg aataaggaga agaacaatgg 4560
cgccatcctg tttgtgaaga acggcctgta ctatctgggc atcatgccaa agcagaaggg 4620
caggtataag gccctgagct tcgagcccac agagaaaacc agcgagggct ttgataagat 4680
gtactatgac tacttccctg atgccgccaa gatgatccca aagtgcagca cccagctgaa 4740
ggccgtgaca gcccactttc agacccacac aaccccccatc ctgctgtcca acaatttcat 4800
cgagcctctg gagatcacaa aggagatcta cgacctgaac aatcctgaga aggagccaaa 4860
gaagtttcag acagcctacg ccaagaaaac cggcgaccag aagggctaca gagaggccct 4920
gtgcaagtgg atcgacttca caagggattt tctgtccaag tataccaaga caacctctat 4980
cgatctgtct agcctgcggc catcctctca gtataaggac ctgggcgagt actatgccga 5040
gctgaatccc ctgctgtacc acatcagctt ccagagaatc gccgagaagg agatcatgga 5100
tgccgtggag acaggcaagc tgtacctgtt ccagatctat aacaaggact ttgccaaggg 5160
ccaccacggc aagcctaatc tgcacacact gtattggacc ggcctgtttt ctccagagaa 5220
cctggccaag acaagcatca agctgaatgg ccaggccgag ctgttctacc gccctaagtc 5280
caggatgaag aggatggcac accggctggg agagaagatg ctgaacaaga agctgaagga 5340
tcagaaaacc ccaatccccg acaccctgta ccaggagctg tacgactatg tgaatcacag 5400
actgtcccac gacctgtctg atgaggccag ggccctgctg cccaacgtga tcaccaagga 5460
ggtgtctcac gagatcatca aggataggcg ctttaccagc gacaagttct tttccacgt 5520
gcctatcaca ctgaactatc aggccgccaa ttccccatct aagttcaacc agagggtgaa 5580
tgcctacctg aaggagcacc ccgagacacc tatcatcggc atcgatcggg gcgagagaaa 5640
cctgatctat atcacagtga tcgactccac cggcaagatc ctggagcagc ggagcctgaa 5700
caccatccag cagtttgatt accagaagaa gctggacaac agggagaagg agagggtggc 5760
agcaaggcag gcctggtctg tggtgggcac aatcaaggat ctgaagcagg gctatctgag 5820
ccaggtcatc cacgagatcg tggacctgat gatccactac caggccgtgg tggtgctgga 5880
gaacctgaat ttcggcttta agagcaagag gaccggcatc gccgagaagg ccgtgtacca 5940
gcagttcgag aagatgctga tcgataagct gaattgcctg gtgctgaagg actatccagc 6000

agagaaagtg ggaggcgtgc tgaacccata ccagctgaca gaccagttca cctcctttgc 6060
caagatgggc acccagtctg gcttcctgtt ttacgtgcct gccccatata catctaagat 6120
cgatcccctg accggcttcg tggacccctt cgtgtggaaa accatcaaga atcacgagag 6180
ccgcaagcac ttcctggagg gcttcgactt tctgcactac gacgtgaaaa ccggcgactt 6240
catcctgcac tttaagatga acagaaatct gtccttccag aggggcctgc ccggctttat 6300
gcctgcatgg gatatcgtgt tcgagaagaa cgagacacag tttgacgcca agggcacccc 6360
tttcatcgcc ggcaagagaa tcgtgccagt gatcgagaat cacagattca ccggcagata 6420
ccgggacctg tatcctgcca acgagctgat cgccctgctg gaggagaagg gcatcgtgtt 6480
cagggatggc tccaacatcc tgccaaagct gctggagaat gacgattctc acgccatcga 6540
caccatggtg gccctgatcc gcagcgtgct gcagatgcgg aactccaatg ccgccacagg 6600
cgaggactat atcaacagcc ccgtgcgcga tctgaatggc gtgtgcttcg actcccggtt 6660
tcagaaccca gagtggccca tggacgccga tgccaatggc gcctaccaca tcgccctgaa 6720
gggccagctg ctgctgaatc acctgaagga gagcaaggat ctgaagctgc agaacggcat 6780
ctccaatcag gactggctgg cctacatcca ggagctgcgc aacaaaaggc cggcggccac 6840
gaaaaaggcc ggccaggcaa aaaagaaaaa gggatccggc gcaacaaact tctctctgct 6900
gaaacaagcc ggagatgtcg aagagaatcc tggaccgatg gccaagcctt tgtctcaaga 6960
agaatccacc ctcattgaaa gagcaacggc tacaatcaac agcatcccca tctctgaaga 7020
ctacagcgtc gccagcgcag ctctctctag cgacggccgc atcttcactg gtgtcaatgt 7080
atatcatttt actgggggac cttgtgcaga actcgtggtg ctgggcactg ctgctgctgc 7140
ggcagctggc aacctgactt gtatcgtcgc gatcggaaat gagaacaggg gcatcttgag 7200
cccctgcgga cggtgccgac aggtgcttct cgatctgcat cctgggatca aagccatagt 7260
gaaggacagt gatggacagc cgacggcagt tgggattcgt gaattgctgc cctctggtta 7320
tgtgtgggag ggctaagaat tcgatatcaa gcttatcggt aatcaacctc tggattacaa 7380
aatttgtgaa agattgactg gtattcttaa ctatgttgct ccttttacgc tatgtggata 7440
cgctgcttta atgcctttgt atcatgctat tgcttcccgt atggctttca ttttctcctc 7500
cttgtataaa tcctggttgc tgtctcttta tgaggagttg tggcccgttg tcaggcaacg 7560
tggcgtggtg tgcactgtgt ttgctgacgc aacccccact ggttggggca ttgccaccac 7620
ctgtcagctc ctttccggga ctttcgcttt ccccctccct attgccacgg cggaactcat 7680
cgccgcctgc cttgcccgct gctggacagg ggctcggctg ttgggcactg acaattccgt 7740
ggtgttgtcg ggaaatcat cgtcctttcc ttggctgctc gcctgtgttg ccacctggat 7800
tctgcgcggg acgtccttct gctacgtccc ttcggccctc aatccagcgg accttccttc 7860

ccgcggcctg ctgccggctc tgcggcctct tccgcgtctt cgccttcgcc ctcagacgag 7920
tcggatctcc ctttgggccg cctccccgca tcgataccgt cgacctcgag acctagaaaa 7980
acatggagca atcacaagta gcaatacagc agctaccaat gctgattgtg cctggctaga 8040
agcacaagag gaggaggagg tgggttttcc agtcacacct caggtacctt taagaccaat 8100
gacttacaag gcagctgtag atcttagcca ctttttaaaa gaaaaggggg gactggaagg 8160
gctaattcac tcccaacgaa gacaagatat ccttgatctg tggatctacc acacacaagg 8220
ctacttccct gattggcaga actacacacc agggccaggg atcagatatc cactgacctt 8280
tggatggtgc tacaagctag taccagttga gcaagagaag gtagaagaag ccaatgaagg 8340
agagaacacc cgcttgttac accctgtgag cctgcatggg atggatgacc cggagagaga 8400
agtattagag tggaggtttg acagccgcct agcatttcat cacatggccc gagagctgca 8460
tccggactgt actgggtctc tctggttaga ccagatctga gcctgggagc tctctggcta 8520
actagggaac ccactgctta agcctcaata aagcttgcct tgagtgcttc aagtagtgtg 8580
tgcccgtctg ttgtgtgact ctggtaacta gagatccctc agaccctttt agtcagtgtg 8640
gaaaatctct agcagggccc gtttaaaccc gctgatcagc ctcgactgtg ccttctagtt 8700
gccagccatc tgttgtttgc ccctcccccg tgccttcctt gaccctggaa ggtgccactc 8760
ccactgtcct ttcctaataa aatgaggaaa ttgcatcgca ttgtctgagt aggtgtcatt 8820
ctattctggg gggtggggtg gggcaggaca gcaaggggga ggattgggaa gacaatagca 8880
ggcatgctgg ggatgcggtg ggctctatgg cttctgaggc ggaaagaacc agctggggct 8940
ctagggggta tccccacgcg ccctgtagcg gcgcattaag cgcggcgggt gtggtggtta 9000
cgcgcagcgt gaccgctaca cttgccagcg ccctagcgcc cgctcctttc gctttcttcc 9060
cttcctttct cgccacgttc gccggctttc cccgtcaagc tctaaatcgg gggctccctt 9120
tagggttccg atttagtgct ttacggcacc tcgaccccaa aaaacttgat tagggtgatg 9180
gttcacgtag tgggccatcg ccctgataga cggtttttcg ccctttgacg ttggagtcca 9240
cgttctttaa tagtggactc ttgttccaaa ctggaacaac actcaaccct atctcggtct 9300
attcttttga tttataaggg attttgccga tttcggccta ttggttaaaa aatgagctga 9360
tttaacaaaa atttaacgcg aattaattct gtggaatgtg tgtcagttag ggtgtggaaa 9420
gtccccaggc tccccagcag gcagaagtat gcaaagcatg catctcaatt agtcagcaac 9480
caggtgtgga aagtccccag gctccccagc aggcagaagt atgcaaagca tgcatctcaa 9540
ttagtcagca accatagtcc cgcccctaac tccgcccatc ccgcccctaa ctccgcccag 9600
ttccgcccat tctccgcccc atggctgact aattttttt atttatgcag aggccgaggc 9660
cgcctctgcc tctgagctat tccagaagta gtgaggaggc ttttttggag gcctaggctt 9720
ttgcaaaaag ctcccgggag cttgtatatc cattttcgga tctgatcagc acgtgttgac 9780

aattaatcat cggcatagta tatcggcata gtataatacg acaaggtgag gaactaaacc 9840
atggccaagt tgaccagtgc cgttccggtg ctcaccgcgc gcgacgtcgc cggagcggtc 9900
gagttctgga ccgaccggct cgggttctcc cgggacttcg tggaggacga cttcgccggt 9960
gtggtccggg acgacgtgac cctgttcatc agcgcggtcc aggaccaggt ggtgccggac 10020
aacaccctgg cctgggtgtg ggtgcgcggc ctggacgagc tgtacgccga gtggtcggag 10080
gtcgtgtcca cgaacttccg ggacgcctcc gggccggcca tgaccgagat cggcgagcag 10140
ccgtgggggc gggagttcgc cctgcgcgac ccggccggca actgcgtgca cttcgtggcc 10200
gaggagcagg actgacacgt gctacgagat ttcgattcca ccgccgcctt ctatgaaagg 10260
ttgggcttcg gaatcgtttt ccgggacgcc ggctggatga tcctccagcg cggggatctc 10320
atgctggagt tcttcgccca ccccaacttg tttattgcag cttataatgg ttacaaataa 10380
agcaatagca tcacaaattt cacaaataaa gcattttttt cactgcattc tagttgtggt 10440
ttgtccaaac tcatcaatgt atcttatcat gtctgtatac cgtcgacctc tagctagagc 10500
ttggcgtaat catggtcata gctgtttcct gtgtgaaatt gttatccgct cacaattcca 10560
cacaacatac gagccggaag cataaagtgt aaagcctggg gtgcctaatg agtgagctaa 10620
ctcacattaa ttgcgttgcg ctcactgccc gctttccagt cgggaaacct gtcgtgccag 10680
ctgcattaat gaatcggcca acgcgcgggg agaggcggtt tgcgtattgg gcgctcttcc 10740
gcttcctcgc tcactgactc gctgcgctcg gtcgttcggc tgcggcgagc ggtatcagct 10800
cactcaaagg cggtaatacg gttatccaca gaatcagggg ataacgcagg aaagaacatg 10860
tgagcaaaag gccagcaaaa ggccaggaac cgtaaaaagg ccgcgttgct ggcgtttttc 10920
cataggctcc gcccccctga cgagcatcac aaaaatcgac gctcaagtca gaggtggcga 10980
aacccgacag gactataaag ataccaggcg tttccccctg gaagctccct cgtgcgctct 11040
cctgttccga ccctgccgct taccggatac ctgtccgcct ttctcccttc gggaagcgtg 11100
gcgctttctc atagctcacg ctgtaggtat ctcagttcgg tgtaggtcgt tcgctccaag 11160
ctgggctgtg tgcacgaacc ccccgttcag cccgaccgct gcgccttatc cggtaactat 11220
cgtcttgagt ccaacccggt aagacacgac ttatcgccac tggcagcagc cactggtaac 11280
aggattagca gagcgaggta tgtaggcggt gctacagagt tcttgaagtg gtggcctaac 11340
tacggctaca ctagaagaac agtatttggt atctgcgctc tgctgaagcc agttaccttc 11400
ggaaaaagag ttggtagctc ttgatccggc aaacaaacca ccgctggtag cggtggtttt 11460
tttgtttgca agcagcagat tacgcgcaga aaaaaaggat ctcaagaaga tcctttgatc 11520
ttttctacgg ggtctgacgc tcagtggaac gaaaactcac gttaagggat tttggtcatg 11580
agattatcaa aaggatctt cacctagatc cttttaaatt aaaaatgaag ttttaaatca 11640

atctaaagta tatatgagta aacttggtct gacagttacc aatgcttaat cagtgaggca 11700

cctatctcag cgatctgtct atttcgttca tccatagttg cctgactccc cgtcgtgtag 11760

ataactacga tacgggaggg cttaccatct ggccccagtg ctgcaatgat accgcgagac 11820

ccacgctcac cggctccaga tttatcagca ataaaccagc cagccggaag ggccgagcgc 11880

agaagtggtc ctgcaacttt atccgcctcc atccagtcta ttaattgttg ccgggaagct 11940

agagtaagta gttcgccagt taatagtttg cgcaacgttg ttgccattgc tacaggcatc 12000

gtggtgtcac gctcgtcgtt tggtatggct tcattcagct ccggttccca acgatcaagg 12060

cgagttacat gatcccccat gttgtgcaaa aaagcggtta gctccttcgg tcctccgatc 12120

gttgtcagaa gtaagttggc cgcagtgtta tcactcatgg ttatggcagc actgcataat 12180

tctcttactg tcatgccatc cgtaagatgc ttttctgtga ctggtgagta ctcaaccaag 12240

tcattctgag aatagtgtat gcggcgaccg agttgctctt gcccggcgtc aatacgggat 12300

aataccgcgc cacatagcag aactttaaaa gtgctcatca ttggaaaacg ttcttcgggg 12360

cgaaaactct caaggatctt accgctgttg agatccagtt cgatgtaacc cactcgtgca 12420

cccaactgat cttcagcatc ttttactttc accagcgttt ctgggtgagc aaaaacagga 12480

aggcaaaatg ccgcaaaaaa gggaataagg gcgacacgga aatgttgaat actcatactc 12540

ttcctttttc aatattattg aagcatttat cagggttatt gtctcatgag cggatacata 12600

tttgaatgta tttagaaaaa taaacaaata ggggttccgc gcacatttcc ccgaaaagtg 12660

ccacctgac 12669

<210> 4
<211> 12432
<212> DNA
<213> Artificial Sequence

<220>
<223> Lenti_LbCpf1-Blast

<400> 4

```
gtcgacggat cgggagatct cccgatcccc tatggtgcac tctcagtaca atctgctctg      60
atgccgcata gttaagccag tatctgctcc ctgcttgtgt gttggaggtc gctgagtagt     120
gcgcgagcaa aatttaagct acaacaaggc aaggcttgac cgacaattgc atgaagaatc     180
tgcttagggt taggcgtttt gcgctgcttc gcgatgtacg ggccagatat acgcgttgac     240
attgattatt gactagttat taatagtaat caattacggg gtcattagtt catagcccat     300
atatggagtt ccgcgttaca taacttacgg taaatggccc gcctggctga ccgcccaacg     360
acccccgccc attgacgtca ataatgacgt atgttcccat agtaacgcca atagggactt     420
tccattgacg tcaatgggtg gagtatttac ggtaaactgc ccacttggca gtacatcaag     480
```

```
tgtatcatat gccaagtacg ccccctattg acgtcaatga cggtaaatgg cccgcctggc        540
attatgccca gtacatgacc ttatgggact ttcctacttg gcagtacatc tacgtattag        600
tcatcgctat taccatggtg atgcggtttt ggcagtacat caatgggcgt ggatagcggt        660
ttgactcacg gggatttcca agtctccacc ccattgacgt caatgggagt ttgttttggc        720
accaaaatca acgggacttt ccaaaatgtc gtaacaactc cgccccattg acgcaaatgg        780
gcggtaggcg tgtacggtgg gaggtctata taagcagcgc gttttgcctg tactgggtct        840
ctctggttag accagatctg agcctgggag ctctctggct aactagggaa cccactgctt        900
aagcctcaat aaagcttgcc ttgagtgctt caagtagtgt gtgcccgtct gttgtgtgac        960
tctggtaact agagatccct cagacccttt tagtcagtgt ggaaaatctc tagcagtggc       1020
gcccgaacag ggacttgaaa gcgaaaggga aaccagagga gctctctcga cgcaggactc       1080
ggcttgctga agcgcgcacg gcaagaggcg aggggcggcg actggtgagt acgccaaaaa       1140
ttttgactag cggaggctag aaggagagag atgggtgcga gagcgtcagt attaagcggg       1200
ggagaattag atcgcgatgg gaaaaaattc ggttaaggcc agggggaaag aaaaaatata       1260
aattaaaaca tatagtatgg gcaagcaggg agctagaacg attcgcagtt aatcctggcc       1320
tgttagaaac atcagaaggc tgtagacaaa tactgggaca gctacaacca tcccttcaga       1380
caggatcaga agaacttaga tcattatata atacagtagc aaccctctat tgtgtgcatc       1440
aaaggataga gataaaagac accaaggaag ctttagacaa gatagaggaa gagcaaaaca       1500
aaagtaagac caccgcacag caagcggccg ctgatcttca gacctggagg aggagatatg       1560
agggacaatt ggagaagtga attatataaa tataaagtag taaaaattga accattagga       1620
gtagcaccca ccaaggcaaa gagaagagtg gtgcagagag aaaaaagagc agtgggaata       1680
ggagctttgt tccttgggtt cttgggagca gcaggaagca ctatgggcgc agcgtcaatg       1740
acgctgacgg tacaggccag acaattattg tctggtatag tgcagcagca gaacaatttg       1800
ctgagggcta ttgaggcgca acagcatctg ttgcaactca cagtctgggg catcaagcag       1860
ctccaggcaa gaatcctggc tgtggaaaga tacctaaagg atcaacagct cctggggatt       1920
tggggttgct ctggaaaact catttgcacc actgctgtgc cttggaatgc tagttggagt       1980
aataaatctc tggaacagat ttggaatcac acgacctgga tggagtggga cagagaaatt       2040
aacaattaca caagcttaat acactcctta attgaagaat cgcaaaacca gcaagaaaag       2100
aatgaacaag aattattgga attagataaa tgggcaagtt tgtggaattg gtttaacata       2160
acaaattggc tgtggtatat aaaattattc ataatgatag taggaggctt ggtaggttta       2220
agaatagttt ttgctgtact ttctatagtg aatagagtta ggcagggata ttcaccatta       2280
tcgtttcaga cccacctccc aaccccgagg ggacccgaca ggcccgaagg aatagaagaa       2340
gaaggtggag agagagacag agacagatcc attcgattag tgaacggatc ggcactgcgt       2400
```

gcgccaattc tgcagacaaa tggcagtatt catccacaat tttaaaagaa aaggggggat 2460

tggggggtac agtgcagggg aaagaatagt agacataata gcaacagaca tacaaactaa 2520

agaattacaa aaacaaatta caaaaattca aaattttcgg gtttattaca gggacagcag 2580

agatccagtt tggttaatta gctagctagg tcttgaaagg agtgggaatt ggctccggtg 2640

cccgtcagtg ggcagagcgc acatcgccca cagtccccga gaagttgggg ggaggggtcg 2700

gcaattgatc cggtgcctag agaaggtggc gcggggtaaa ctgggaaagt gatgtcgtgt 2760

actggctccg cctttttccc gagggtgggg gagaaccgta tataagtgca gtagtcgccg 2820

tgaacgttct ttttcgcaac gggtttgccg ccagaacaca ggaccggttc tagcgtttaa 2880

acttaagctt ggtaccgcca ccatgagcaa gctggagaag tttacaaact gctactccct 2940

gtctaagacc ctgaggttca aggccatccc tgtgggcaag acccaggaga acatcgacaa 3000

taagcggctg ctggtggagg acgagaagag agccgaggat tataagggcg tgaagaagct 3060

gctggatcgc tactatctgt cttttatcaa cgacgtgctg cacagcatca agctgaagaa 3120

tctgaacaat tacatcagcc tgttccggaa gaaaaccaga accgagaagg agaataagga 3180

gctggagaac ctggagatca atctgcggaa ggagatcgcc aaggccttca agggcaacga 3240

gggctacaag tccctgttta agaaggatat catcgagaca atcctgccag agttcctgga 3300

cgataaggac gagatcgccc tggtgaacag cttcaatggc tttaccacag ccttcaccgg 3360

cttctttgat aacagagaga atatgttttc cgaggaggcc aagagcacat ccatcgcctt 3420

caggtgtatc aacgagaatc tgacccgcta catctctaat atggacatct tcgagaaggt 3480

ggacgccatc tttgataagc acgaggtgca ggagatcaag gagaagatcc tgaacagcga 3540

ctatgatgtg gaggatttct ttgagggcga gttctttaac tttgtgctga cacaggaggg 3600

catcgacgtg tataacgcca tcatcggcgg cttcgtgacc gagagcggcg agaagatcaa 3660

gggcctgaac gagtacatca acctgtataa tcagaaaacc aagcagaagc tgcctaagtt 3720

taagccactg tataagcagg tgctgagcga tcgggagtct ctgagcttct acggcgaggg 3780

ctatacatcc gatgaggagg tgctggaggt gtttagaaac accctgaaca agaacagcga 3840

gatcttcagc tccatcaaga agctggagaa gctgttcaag aattttgacg agtactctag 3900

cgccggcatc tttgtgaaga acggccccgc catcagcaca atctccaagg atatcttcgg 3960

cgagtggaac gtgatccggg acaagtggaa tgccgagtat gacgatatcc acctgaagaa 4020

gaaggccgtg gtgaccgaga agtacgagga cgatcggaga aagtccttca agaagatcgg 4080

ctccttttct ctggagcagc tgcaggagta cgccgacgcc gatctgtctg tggtggagaa 4140

gctgaaggag atcatcatcc agaaggtgga tgagatctac aaggtgtatg gctcctctga 4200

gaagctgttc gacgccgatt ttgtgctgga gaagagcctg aagaagaacg acgccgtggt 4260

ggccatcatg aaggacctgc tggattctgt gaagagcttc gagaattaca tcaaggcctt 4320
ctttggcgag ggcaaggaga caaacaggga cgagtccttc tatggcgatt ttgtgctggc 4380
ctacgacatc ctgctgaagg tggaccacat ctacgatgcc atccgcaatt atgtgaccca 4440
gaagccctac tctaaggata agttcaagct gtattttcag aaccctcagt tcatgggcgg 4500
ctgggacaag gataaggaga cagactatcg ggccaccatc ctgagatacg gctccaagta 4560
ctatctggcc atcatggata agaagtacgc caagtgcctg cagaagatcg acaaggacga 4620
tgtgaacggc aattacgaga agatcaacta taagctgctg cccggcccta ataagatgct 4680
gccaaaggtg ttcttttcta agaagtggat ggcctactat aaccccagcg aggacatcca 4740
gaagatctac aagaatggca cattcaagaa gggcgatatg tttaacctga atgactgtca 4800
caagctgatc gacttcttta aggatagcat ctcccggtat ccaaagtggt ccaatgccta 4860
cgatttcaac ttttctgaga cagagaagta taaggacatc gccggctttt acagagaggt 4920
ggaggagcag ggctataagg tgagcttcga gtctgccagc aagaaggagg tggataagct 4980
ggtggaggag ggcaagctgt atatgttcca gatctataac aaggactttt ccgataagtc 5040
tcacggcaca cccaatctgc acaccatgta cttcaagctg ctgtttgacg agaacaatca 5100
cggacagatc aggctgagcg gaggagcaga gctgttcatg aggcgcgcct ccctgaagaa 5160
ggaggagctg gtggtgcacc cagccaactc ccctatcgcc aacaagaatc cagataatcc 5220
caagaaaacc acaaccctgt cctacgacgt gtataaggat aagaggtttt ctgaggacca 5280
gtacgagctg cacatcccaa tcgccatcaa taagtgcccc aagaacatct tcaagatcaa 5340
tacagaggtg cgcgtgctgc tgaagcacga cgataacccc tatgtgatcg gcatcgatag 5400
gggcgagcgc aatctgctgt atatcgtggt ggtggacggc aagggcaaca tcgtggagca 5460
gtattccctg aacgagatca tcaacaactt caacggcatc aggatcaaga cagattacca 5520
ctctctgctg gacaagaagg agaaggagag gttcgaggcc cgccagaact ggacctccat 5580
cgagaatatc aaggagctga aggccggcta tatctctcag gtggtgcaca agatctgcga 5640
gctggtggag aagtacgatg ccgtgatcgc cctggaggac ctgaactctg gctttaagaa 5700
tagccgcgtg aaggtggaga agcaggtgta tcagaagttc gagaagatgc tgatcgataa 5760
gctgaactac atggtggaca agaagtctaa tccttgtgca acaggcggcg ccctgaaggg 5820
ctatcagatc accaataagt tcgagagctt taagtccatg tctacccaga acggcttcat 5880
cttttacatc cctgcctggc tgacatccaa gatcgatcca tctaccggct ttgtgaacct 5940
gctgaaaacc aagtatacca gcatcgccga ttccaagaag ttcatcagct cctttgacag 6000
gatcatgtac gtgcccgagg aggatctgtt cgagtttgcc ctggactata agaacttctc 6060
tcgcacagac gccgattaca tcaagaagtg gaagctgtac tcctacggca accggatcag 6120
aatcttccgg aatcctaaga agaacaacgt gttcgactgg gaggaggtgt gcctgaccag 6180

cgcctataag gagctgttca acaagtacgg catcaattat cagcagggcg atatcagagc 6240
cctgctgtgc gagcagtccg acaaggcctt ctactctagc tttatggccc tgatgagcct 6300
gatgctgcag atgcggaaca gcatcacagg ccgcaccgac gtggattttc tgatcagccc 6360
tgtgaagaac tccgacggca tcttctacga tagccggaac tatgaggccc aggagaatgc 6420
catcctgcca aagaacgccg acgccaatgg cgcctataac atcgccagaa aggtgctgtg 6480
ggccatcggc cagttcaaga aggccgagga cgagaagctg gataaggtga agatcgccat 6540
ctctaacaag gagtggctgg agtacgccca gaccagcgtg aagcacaaaa ggccggcggc 6600
cacgaaaaag gccggccagg caaaaaagaa aaagggatcc ggcgcaacaa acttctctct 6660
gctgaaacaa gccggagatg tcgaagagaa tcctggaccg atggccaagc ctttgtctca 6720
agaagaatcc accctcattg aaagagcaac ggctacaatc aacagcatcc ccatctctga 6780
agactacagc gtcgccagcg cagctctctc tagcgacggc cgcatcttca ctggtgtcaa 6840
tgtatatcat tttactgggg gaccttgtgc agaactcgtg gtgctgggca ctgctgctgc 6900
tgcggcagct ggcaacctga cttgtatcgt cgcgatcgga aatgagaaca ggggcatctt 6960
gagcccctgc ggacggtgcc gacaggtgct tctcgatctg catcctggga tcaaagccat 7020
agtgaaggac agtgatggac agccgacggc agttgggatt cgtgaattgc tgccctctgg 7080
ttatgtgtgg gagggctaag aattcgatat caagcttatc ggtaatcaac ctctggatta 7140
caaaatttgt gaaagattga ctggtattct taactatgtt gctcctttta cgctatgtgg 7200
atacgctgct ttaatgcctt tgtatcatgc tattgcttcc cgtatggctt tcattttctc 7260
ctccttgtat aaatcctggt tgctgtctct ttatgaggag ttgtggcccg ttgtcaggca 7320
acgtggcgtg gtgtgcactg tgtttgctga cgcaaccccc actggttggg gcattgccac 7380
cacctgtcag ctcctttccg ggactttcgc tttccccctc cctattgcca cggcggaact 7440
catcgccgcc tgccttgccc gctgctggac aggggctcgg ctgttgggca ctgacaattc 7500
cgtggtgttg tcggggaaat catcgtcctt tccttggctg ctcgcctgtg ttgccacctg 7560
gattctgcgc gggacgtcct tctgctacgt cccttcggcc ctcaatccag cggaccttcc 7620
ttcccgcggc ctgctgccgg ctctgcggcc tcttccgcgt cttcgccttc gccctcagac 7680
gagtcggatc tccctttggg ccgcctcccc gcatcgatac cgtcgacctc gagacctaga 7740
aaaacatgga gcaatcacaa gtagcaatac agcagctacc aatgctgatt gtgcctggct 7800
agaagcacaa gaggaggagg aggtgggttt tccagtcaca cctcaggtac ctttaagacc 7860
aatgacttac aaggcagctg tagatcttag ccacttttta aaagaaaagg ggggactgga 7920
agggctaatt cactcccaac gaagacaaga tatccttgat ctgtggatct accacacaca 7980
aggctacttc cctgattggc agaactacac accagggcca gggatcagat atccactgac 8040

ctttggatgg tgctacaagc tagtaccagt tgagcaagag aaggtagaag aagccaatga 8100
aggagagaac acccgcttgt tacaccctgt gagcctgcat gggatggatg acccggagag 8160
agaagtatta gagtggaggt ttgacagccg cctagcattt catcacatgg cccgagagct 8220
gcatccggac tgtactgggt ctctctggtt agaccagatc tgagcctggg agctctctgg 8280
ctaactaggg aacccactgc ttaagcctca ataaagcttg ccttgagtgc ttcaagtagt 8340
gtgtgcccgt ctgttgtgtg actctggtaa ctagagatcc ctcagaccct tttagtcagt 8400
gtggaaaatc tctagcaggg cccgtttaaa cccgctgatc agcctcgact gtgccttcta 8460
gttgccagcc atctgttgtt tgcccctccc ccgtgccttc cttgaccctg gaaggtgcca 8520
ctcccactgt cctttcctaa taaaatgagg aaattgcatc gcattgtctg agtaggtgtc 8580
attctattct ggggggtggg gtggggcagg acagcaaggg ggaggattgg gaagacaata 8640
gcaggcatgc tggggatgcg gtgggctcta tggcttctga ggcggaaaga accagctggg 8700
gctctagggg gtatccccac gcgccctgta gcggcgcatt aagcgcggcg ggtgtggtgg 8760
ttacgcgcag cgtgaccgct acacttgcca gcgccctagc gcccgctcct ttcgctttct 8820
tcccttcctt tctcgccacg ttcgccggct ttccccgtca agctctaaat cgggggctcc 8880
ctttagggtt ccgatttagt gctttacggc acctcgaccc caaaaaactt gattagggtg 8940
atggttcacg tagtgggcca tcgccctgat agacggtttt tcgccctttg acgttggagt 9000
ccacgttctt taatagtgga ctcttgttcc aaactggaac aacactcaac cctatctcgg 9060
tctattcttt tgatttataa gggattttgc cgatttcggc ctattggtta aaaaatgagc 9120
tgatttaaca aaaatttaac gcgaattaat tctgtggaat gtgtgtcagt tagggtgtgg 9180
aaagtcccca ggctccccag caggcagaag tatgcaaagc atgcatctca attagtcagc 9240
aaccaggtgt ggaaagtccc caggctcccc agcaggcaga agtatgcaaa gcatgcatct 9300
caattagtca gcaaccatag tcccgcccct aactccgccc atcccgcccc taactccgcc 9360
cagttccgcc cattctccgc cccatggctg actaattttt tttatttatg cagaggccga 9420
ggccgcctct gcctctgagc tattccagaa gtagtgagga ggcttttttg gaggcctagg 9480
cttttgcaaa aagctcccgg gagcttgtat atccattttc ggatctgatc agcacgtgtt 9540
gacaattaat catcggcata gtatatcggc atagtataat acgacaaggt gaggaactaa 9600
accatggcca agttgaccag tgccgttccg gtgctcaccg cgcgcgacgt cgccggagcg 9660
gtcgagttct ggaccgaccg gctcgggttc tcccgggact tcgtggagga cgacttcgcc 9720
ggtgtggtcc gggacgacgt gaccctgttc atcagcgcgg tccaggacca ggtggtgccg 9780
gacaacaccc tggcctgggt gtgggtgcgc ggcctggacg agctgtacgc cgagtggtcg 9840
gaggtcgtgt ccacgaactt ccgggacgcc tccgggccgg ccatgaccga gatcggcgag 9900
cagccgtggg ggcgggagtt cgccctgcgc gacccggccg gcaactgcgt gcacttcgtg 9960

gccgaggagc aggactgaca cgtgctacga gatttcgatt ccaccgccgc cttctatgaa 10020
aggttgggct tcggaatcgt tttccgggac gccggctgga tgatcctcca gcgcggggat 10080
ctcatgctgg agttcttcgc ccaccccaac ttgtttattg cagcttataa tggttacaaa 10140
taaagcaata gcatcacaaa tttcacaaat aaagcatttt tttcactgca ttctagttgt 10200
ggtttgtcca aactcatcaa tgtatcttat catgtctgta taccgtcgac ctctagctag 10260
agcttggcgt aatcatggtc atagctgttt cctgtgtgaa attgttatcc gctcacaatt 10320
ccacacaaca tacgagccgg aagcataaag tgtaaagcct ggggtgccta atgagtgagc 10380
taactcacat taattgcgtt gcgctcactg cccgctttcc agtcgggaaa cctgtcgtgc 10440
cagctgcatt aatgaatcgg ccaacgcgcg gggagaggcg gtttgcgtat tgggcgctct 10500
tccgcttcct cgctcactga ctcgctgcgc tcggtcgttc ggctgcggcg agcggtatca 10560
gctcactcaa aggcggtaat acggttatcc acagaatcag gggataacgc aggaaagaac 10620
atgtgagcaa aaggccagca aaaggccagg aaccgtaaaa aggccgcgtt gctggcgttt 10680
ttccataggc tccgcccccc tgacgagcat cacaaaaatc gacgctcaag tcagaggtgg 10740
cgaaacccga caggactata aagataccag gcgtttcccc ctggaagctc cctcgtgcgc 10800
tctcctgttc cgaccctgcc gcttaccgga tacctgtccg cctttctccc ttcgggaagc 10860
gtggcgcttt ctcatagctc acgctgtagg tatctcagtt cggtgtaggt cgttcgctcc 10920
aagctgggct gtgtgcacga accccccgtt cagcccgacc gctgcgcctt atccggtaac 10980
tatcgtcttg agtccaaccc ggtaagacac gacttatcgc cactggcagc agccactggt 11040
aacaggatta gcagagcgag gtatgtaggc ggtgctacag agttcttgaa gtggtggcct 11100
aactacggct acactagaag aacagtattt ggtatctgcg ctctgctgaa gccagttacc 11160
ttcggaaaaa gagttggtag ctcttgatcc ggcaaacaaa ccaccgctgg tagcggtggt 11220
ttttttgttt gcaagcagca gattacgcgc agaaaaaaag gatctcaaga agatcctttg 11280
atcttttcta cggggtctga cgctcagtgg aacgaaaact cacgttaagg gattttggtc 11340
atgagattat caaaaaggat cttcacctag atccttttaa attaaaaatg aagttttaaa 11400
tcaatctaaa gtatatatga gtaaacttgg tctgacagtt accaatgctt aatcagtgag 11460
gcacctatct cagcgatctg tctatttcgt tcatccatag ttgcctgact ccccgtcgtg 11520
tagataacta cgatacggga gggcttacca tctggcccca gtgctgcaat gataccgcga 11580
gacccacgct caccggctcc agatttatca gcaataaacc agccagccgg aagggccgag 11640
cgcagaagtg gtcctgcaac tttatccgcc tccatccagt ctattaattg ttgccgggaa 11700
gctagagtaa gtagttcgcc agttaatagt ttgcgcaacg ttgttgccat tgctacaggc 11760
atcgtggtgt cacgctcgtc gtttggtatg gcttcattca gctccggttc ccaacgatca 11820

aggcgagtta catgatcccc catgttgtgc aaaaaagcgg ttagctcctt cggtcctccg 11880

atcgttgtca gaagtaagtt ggccgcagtg ttatcactca tggttatggc agcactgcat 11940

aattctctta ctgtcatgcc atccgtaaga tgcttttctg tgactggtga gtactcaacc 12000

aagtcattct gagaatagtg tatgcggcga ccgagttgct cttgcccggc gtcaatacgg 12060

gataataccg cgccacatag cagaacttta aaagtgctca tcattggaaa acgttcttcg 12120

gggcgaaaac tctcaaggat cttaccgctg ttgagatcca gttcgatgta acccactcgt 12180

gcacccaact gatcttcagc atcttttact ttcaccagcg tttctgggtg agcaaaaaca 12240

ggaaggcaaa atgccgcaaa aaagggaata agggcgacac ggaaatgttg aatactcata 12300

ctcttccttt ttcaatatta ttgaagcatt tatcagggtt attgtctcat gagcggatac 12360

atatttgaat gtatttagaa aaataaacaa ataggggttc cgcgcacatt tccccgaaaa 12420

gtgccacctg ac 12432

<210> 5
<211> 8329
<212> DNA
<213> Artificial Sequence

<220>
<223> Lenti_crRNA-Puro

<400> 5

```
ccgggtgcaa agatggataa agttttaaac agagaggaat ctttgcagct aatggacctt        60
ctaggtcttg aaaggagtgg gaattggctc cggtgcccgt cagtgggcag agcgcacatc       120
gcccacagtc cccgagaagt tgggggggagg ggtcggcaat tgatccggtg cctagagaag      180
gtggcgcggg gtaaactggg aaagtgatgt cgtgtactgg ctccgccttt ttcccgaggg       240
tgggggagaa ccgtatataa gtgcagtagt cgccgtgaac gttctttttc gcaacgggtt       300
tgccgccaga acacaggtaa gtgccgtgtg tggttcccgc gggcctggcc tctttacggg       360
ttatggccct tgcgtgcctt gaattacttc cactggctgc agtacgtgat tcttgatccc       420
gagcttcggg ttggaagtgg gtgggagagt tcgaggcctt gcgcttaagg agccccttcg       480
cctcgtgctt gagttgaggc ctggcctggg cgctggggcc gccgcgtgcg aatctggtgg       540
caccttcgcg cctgtctcgc tgctttcgat aagtctctag ccatttaaaa tttttgatga       600
cctgctgcga cgcttttttt ctggcaagat agtcttgtaa atgcgggcca agatctgcac       660
actggtattt cggtttttgg ggccgcgggc ggcgacgggg cccgtgcgtc ccagcgcaca       720
tgttcggcga ggcggggcct gcgagcgcgg ccaccgagaa tcggacgggg gtagtctcaa       780
gctggccggc ctgctctggt gcctggcctc gcgccgccgt gtatcgcccc gccctgggcg       840
gcaaggctgg cccggtcggc accagttgcg tgagcggaaa gatggccgct tcccggccct       900
```

gctgcaggga gctcaaaatg gaggacgcgg cgctcgggag agcgggcggg tgagtcaccc 960
acacaaagga aaagggcctt tccgtcctca gccgtcgctt catgtgactc cacggagtac 1020
cgggcgccgt ccaggcacct cgattagttc tcgagctttt ggagtacgtc gtctttaggt 1080
tggggggagg ggttttatgc gatggagttt ccccacactg agtgggtgga gactgaagtt 1140
aggccagctt ggcacttgat gtaattctcc ttggaatttg ccctttttga gtttggatct 1200
tggttcattc tcaagcctca gacagtggtt caaagttttt ttcttccatt tcaggtgtcg 1260
tgacgtacgg ccaccatgac cgagtacaag cccacggtgc gcctcgccac ccgcgacgac 1320
gtccccaggg ccgtacgcac cctcgccgcc gcgttcgccg actaccccgc cacgcgccac 1380
accgtcgatc cggaccgcca catcgagcgg gtcaccgagc tgcaagaact cttcctcacg 1440
cgcgtcgggc tcgacatcgg caaggtgtgg gtcgcggacg acggcgccgc cgtggcggtc 1500
tggaccacgc cggagagcgt cgaagcgggg gcggtgttcg ccgagatcgg cccgcgcatg 1560
gccgagttga gcggttcccg gctggccgcg cagcaacaga tggaaggcct cctggcgccg 1620
caccggccca aggagcccgc gtggttcctg gccaccgtcg gagtctcgcc cgaccaccag 1680
ggcaagggtc tgggcagcgc cgtcgtgctc cccggagtgg aggcggccga gcgcgccggg 1740
gtgcccgcct tcctggagac ctccgcgccc cgcaacctcc ccttctacga gcggctcggc 1800
ttcaccgtca ccgccgacgt cgaggtgccc gaaggaccgc gcacctggtg catgacccgc 1860
aagcccggtg cctgaacgcg ttaagtcgac aatcaacctc tggattacaa aatttgtgaa 1920
agattgactg gtattcttaa ctatgttgct ccttttacgc tatgtggata cgctgcttta 1980
atgcctttgt atcatgctat tgcttcccgt atggctttca ttttctcctc cttgtataaa 2040
tcctggttgc tgtctcttta tgaggagttg tggcccgttg tcaggcaacg tggcgtggtg 2100
tgcactgtgt ttgctgacgc aacccccact ggttggggca ttgccaccac ctgtcagctc 2160
ctttccggga ctttcgcttt ccccctccct attgccacgg cggaactcat cgccgcctgc 2220
cttgcccgct gctggacagg ggctcggctg ttgggcactg acaattccgt ggtgttgtcg 2280
gggaaatcat cgtcctttcc ttggctgctc gcctgtgttg ccacctggat tctgcgcggg 2340
acgtccttct gctacgtccc ttcggccctc aatccagcgg accttccttc ccgcggcctg 2400
ctgccggctc tgcggcctct tccgcgtctt cgccttcgcc ctcagacgag tcggatctcc 2460
ctttgggccg cctccccgcg tcgactttaa gaccaatgac ttacaaggca gctgtagatc 2520
ttagccactt tttaaaagaa aaggggggac tggaagggct aattcactcc caacgaagac 2580
aagatctgct ttttgcttgt actgggtctc tctggttaga ccagatctga gcctgggagc 2640
tctctggcta actagggaac ccactgctta agcctcaata aagcttgcct tgagtgcttc 2700
aagtagtgtg tgcccgtctg ttgtgtgact ctggtaacta gagatccctc agaccctttt 2760
agtcagtgtg gaaaatctct agcagtacgt atagtagttc atgtcatctt attattcagt 2820

atttataact tgcaaagaaa tgaatatcag agagtgagag gaacttgttt attgcagctt 2880

ataatggtta caaataaagc aatagcatca caaatttcac aaataaagca tttttttcac 2940

tgcattctag ttgtggtttg tccaaactca tcaatgtatc ttatcatgtc tggctctagc 3000

tatcccgccc ctaactccgc ccatcccgcc cctaactccg cccagttccg cccattctcc 3060

gccccatggc tgactaattt tttttattta tgcagaggcc gaggccgcct cggcctctga 3120

gctattccag aagtagtgag gaggcttttt tggaggccta gggacgtacc caattcgccc 3180

tatagtgagt cgtattacgc gcgctcactg gccgtcgttt tacaacgtcg tgactgggaa 3240

aaccctggcg ttacccaact taatcgcctt gcagcacatc cccctttcgc cagctggcgt 3300

aatagcgaag aggcccgcac cgatcgccct tcccaacagt tgcgcagcct gaatggcgaa 3360

tgggacgcgc cctgtagcgg cgcattaagc gcggcgggtg tggtggttac gcgcagcgtg 3420

accgctacac ttgccagcgc cctagcgccc gctcctttcg ctttcttccc ttcctttctc 3480

gccacgttcg ccggctttcc ccgtcaagct ctaaatcggg ggctcccttt agggttccga 3540

tttagtgctt tacggcacct cgaccccaaa aaacttgatt agggtgatgg ttcacgtagt 3600

gggccatcgc cctgatagac ggtttttcgc cctttgacgt tggagtccac gttctttaat 3660

agtggactct tgttccaaac tggaacaaca ctcaacccta tctcggtcta ttcttttgat 3720

ttataaggga ttttgccgat ttcggcctat tggttaaaaa atgagctgat ttaacaaaaa 3780

tttaacgcga attttaacaa aatattaacg cttacaattt aggtggcact tttcggggaa 3840

atgtgcgcgg aacccctatt tgtttatttt tctaaataca ttcaaatatg tatccgctca 3900

tgagacaata accctgataa atgcttcaat aatattgaaa aaggaagagt atgagtattc 3960

aacatttccg tgtcgccctt attccctttt ttgcggcatt ttgccttcct gtttttgctc 4020

acccagaaac gctggtgaaa gtaaaagatg ctgaagatca gttgggtgca cgagtgggtt 4080

acatcgaact ggatctcaac agcggtaaga tccttgagag ttttcgcccc gaagaacgtt 4140

ttccaatgat gagcactttt aaagttctgc tatgtggcgc ggtattatcc cgtattgacg 4200

ccgggcaaga gcaactcggt cgccgcatac actattctca gaatgacttg gttgagtact 4260

caccagtcac agaaaagcat cttacggatg gcatgacagt aagagaatta tgcagtgctg 4320

ccataaccat gagtgataac actgcggcca acttacttct gacaacgatc ggaggaccga 4380

aggagctaac cgcttttttg cacaacatgg gggatcatgt aactcgcctt gatcgttggg 4440

aaccggagct gaatgaagcc ataccaaacg acgagcgtga caccacgatg cctgtagcaa 4500

tggcaacaac gttgcgcaaa ctattaactg gcgaactact tactctagct tcccggcaac 4560

aattaataga ctggatggag gcggataaag ttgcaggacc acttctgcgc tcggcccttc 4620

cggctggctg gtttattgct gataaatctg gagccggtga gcgtgggtct cgcggtatca 4680

ttgcagcact ggggccagat ggtaagccct cccgtatcgt agttatctac acgacgggga 4740

gtcaggcaac tatggatgaa cgaaatagac agatcgctga gataggtgcc tcactgatta 4800

agcattggta actgtcagac caagtttact catatatact ttagattgat ttaaaacttc 4860

atttttaatt taaaaggatc taggtgaaga tcctttttga taatctcatg accaaaatcc 4920

cttaacgtga gttttcgttc cactgagcgt cagaccccgt agaaaagatc aaaggatctt 4980

cttgagatcc tttttttctg cgcgtaatct gctgcttgca aacaaaaaaa ccaccgctac 5040

cagcggtggt ttgtttgccg gatcaagagc taccaactct ttttccgaag gtaactggct 5100

tcagcagagc gcagatacca aatactgttc ttctagtgta gccgtagtta ggccaccact 5160

tcaagaactc tgtagcaccg cctacatacc tcgctctgct aatcctgtta ccagtggctg 5220

ctgccagtgg cgataagtcg tgtcttaccg ggttggactc aagacgatag ttaccggata 5280

aggcgcagcg gtcgggctga acggggggtt cgtgcacaca gcccagcttg gagcgaacga 5340

cctacaccga actgagatac ctacagcgtg agctatgaga aagcgccacg cttcccgaag 5400

ggagaaaggc ggacaggtat ccggtaagcg gcagggtcgg aacaggagag cgcacgaggg 5460

agcttccagg gggaaacgcc tggtatcttt atagtcctgt cgggtttcgc cacctctgac 5520

ttgagcgtcg atttttgtga tgctcgtcag gggggcggag cctatggaaa aacgccagca 5580

acgcggcctt tttacggttc ctggcctttt gctggccttt tgctcacatg ttctttcctg 5640

cgttatcccc tgattctgtg gataaccgta ttaccgcctt tgagtgagct gataccgctc 5700

gccgcagccg aacgaccgag cgcagcgagt cagtgagcga ggaagcggaa gagcgcccaa 5760

tacgcaaacc gcctctcccc gcgcgttggc cgattcatta atgcagctgg cacgacaggt 5820

ttcccgactg gaaagcgggc agtgagcgca acgcaattaa tgtgagttag ctcactcatt 5880

aggcacccca ggctttacac tttatgcttc cggctcgtat gttgtgtgga attgtgagcg 5940

gataacaatt tcacacagga aacagctatg accatgatta cgccaagcgc gcaattaacc 6000

ctcactaaag ggaacaaaag ctggagctgc aagcttaatg tagtcttatg caatactctt 6060

gtagtcttgc aacatggtaa cgatgagtta gcaacatgcc ttacaaggag agaaaaagca 6120

ccgtgcatgc cgattggtgg aagtaaggtg gtacgatcgt gccttattag gaaggcaaca 6180

gacgggtctg acatggattg gacgaaccac tgaattgccg cattgcagag atattgtatt 6240

taagtgccta gctcgataca taaacgggtc tctctggtta gaccagatct gagcctggga 6300

gctctctggc taactaggga acccactgct taagcctcaa taaagcttgc cttgagtgct 6360

tcaagtagtg tgtgcccgtc tgttgtgtga ctctggtaac tagagatccc tcagaccctt 6420

ttagtcagtg tggaaaatct ctagcagtgg cgcccgaaca gggacttgaa agcgaaaggg 6480

aaaccagagg agctctctcg acgcaggact cggcttgctg aagcgcgcac ggcaagaggc 6540

gaggggcggc gactggtgag tacgccaaaa attttgacta gcggaggcta gaaggagaga 6600

gatgggtgcg agagcgtcag tattaagcgg gggagaatta gatcgcgatg ggaaaaaatt 6660

cggttaaggc cagggggaaa gaaaaaatat aaattaaaac atatagtatg ggcaagcagg 6720

gagctagaac gattcgcagt taatcctggc ctgttagaaa catcagaagg ctgtagacaa 6780

atactgggac agctacaacc atcccttcag acaggatcag aagaacttag atcattatat 6840

aatacagtag caaccctcta ttgtgtgcat caaaggatag agataaaaga caccaaggaa 6900

gctttagaca agatagagga agagcaaaac aaaagtaaga ccaccgcaca gcaagcggcc 6960

gctgatcttc agacctggag gaggagatat gagggacaat tggagaagtg aattatataa 7020

atataaagta gtaaaaattg aaccattagg agtagcaccc accaaggcaa agagaagagt 7080

ggtgcagaga gaaaaaagag cagtgggaat aggagctttg ttccttgggt tcttgggagc 7140

agcaggaagc actatgggcg cagcgtcaat gacgctgacg gtacaggcca gacaattatt 7200

gtctggtata gtgcagcagc agaacaattt gctgagggct attgaggcgc aacagcatct 7260

gttgcaactc acagtctggg gcatcaagca gctccaggca agaatcctgg ctgtggaaag 7320

atacctaaag gatcaacagc tcctggggat ttggggttgc tctggaaaac tcatttgcac 7380

cactgctgtg ccttggaatg ctagttggag taataaatct ctggaacaga tttggaatca 7440

cacgacctgg atggagtggg acagagaaat taacaattac acaagcttaa tacactcctt 7500

aattgaagaa tcgcaaaacc agcaagaaaa gaatgaacaa gaattattgg aattagataa 7560

atgggcaagt ttgtggaatt ggtttaacat aacaaattgg ctgtggtata taaaattatt 7620

cataatgata gtaggaggct tggtaggttt aagaatagtt tttgctgtac tttctatagt 7680

gaatagagtt aggcagggat attcaccatt atcgtttcag acccacctcc caaccccgag 7740

gggacccaga gagggcctat ttcccatgat tccttcatat ttgcatatac gatacaaggc 7800

tgttagagag ataattagaa ttaatttgac tgtaaacaca aagatattag tacaaaatac 7860

gtgacgtaga aagtaataat ttcttgggta gtttgcagtt ttaaaattat gttttaaaat 7920

ggactatcat atgcttaccg taacttgaaa gtatttcgat ttcttggctt tatatatctt 7980

gtggaaagga cgaaacaccg gagacgttga ctatcgtctc gctactctac cacttgtact 8040

tcagcggtca gcttactcga cttaacgtgc acgtgacacg ttctagaccg tacatgctta 8100

catgggatga agcttggcgt aactagatct tgagacaaat ggcagtattc atccacaatt 8160

ttaaaagaaa aggggggatt ggggggtaca gtgcagggga aagaatagta gacataatag 8220

caacagacat acaaactaaa gaattacaaa aacaaattac aaaaattcaa aattttcggg 8280

tttattacag ggacagcaga gatccacttt ggcgccggct cgaggggc 8329

<210> 6
<211> 55
<212> DNA

<213> Artificial Sequence

<220>
<223> Lenti_crRNA-Puro_clonning FP1

<400> 6
caccggagac gttgactatc gtctcgctac tctaccactt gtacttcagc ggtca 55

<210> 7
<211> 55
<212> DNA
<213> Artificial Sequence

<220>
<223> Lenti_crRNA-Puro_clonning RP1

<400> 7
aagctgaccg ctgaagtaca agtggtagag tagcgagacg atagtcaacg tctcc 55

<210> 8
<211> 60
<212> DNA
<213> Artificial Sequence

<220>
<223> Lenti_crRNA-Puro_clonning FP1

<400> 8

gcttactcga cttaacgtgc acgtgacacg ttctagaccg tacatgctta catgggatga 60

60

<210> 9
<211> 60
<212> DNA
<213> Artificial Sequence

<220>
<223> Lenti_crRNA-Puro_clonning RP2

<400> 9

agcttcatcc catgtaagca tgtacggtct agaacgtgtc acgtgcacgt taagtcgagt 60

60

<210> 10
<211> 60
<212> DNA
<213> Artificial Sequence

<220>
<223> AsCpf1 oligo library amplification FP

<400> 10

atttcttggc tttatatatc ttgtggaaag gacgaaacac cgtaatttct actcttgtag 60

60

<210> 11
<211> 60
<212> DNA
<213> Artificial Sequence

<220>
<223> LbCpf1 oligo library amplification FP

<400> 11

tttcttggct ttatatatct tgtggaaagg acgaaacacc gtaatttcta ctaagtgtag 60

60

<210> 12
<211> 57
<212> DNA
<213> Artificial Sequence

<220>
<223> As/LbCpf1 oligo library amplification RP

<400> 12
gagtaagctg accgctgaag tacaagtggt agagtagaga tctagttacg ccaagct 57

<210> 13
<211> 55
<212> DNA
<213> Artificial Sequence

<220>
<223> Targeted deep sequencing FP

<400> 13
acactctttc cctacacgac gctcttccga tctcttgtgg aaaggacgaa acacc 55

<210> 14
<211> 59
<212> DNA
<213> Artificial Sequence

<220>
<223> Targeted deep sequencing RP

<400> 14
gtgactggag ttcagacgtg tgctcttccg atctttgtgg atgaatactg ccatttgtc 59

<210> 15
<211> 29
<212> DNA
<213> Artificial Sequence

<220>
<223> Illumina indexing FP-1

<400> 15
aatgatacgg cgaccaccga gatctacac 29

<210> 16
<211> 20
<212> DNA
<213> Artificial Sequence

<220>
<223> Illumina indexing FP-2

<400> 16
acactctttc cctacacgac 20

<210> 17
<211> 24
<212> DNA
<213> Artificial Sequence

<220>
<223> Illumina indexing RP-1

<400> 17
caagcagaag acggcatacg agat 24

<210> 18
<211> 21
<212> DNA
<213> Artificial Sequence

<220>
<223> Illumina indexing RP-2

<400> 18
gtgactggag ttcagacgtg t 21

<210> 19
<211> 23
<212> DNA
<213> Artificial Sequence

<220>
<223> qPCR for WPRE FP

<400> 19
gatacgctgc tttaatgcct ttg 23

<210> 20
<211> 26
<212> DNA
<213> Artificial Sequence

<220>
<223> qPCR for WPRE RP

<400> 20
gagacagcaa ccaggattta tacaag 26

<210> 21
<211> 22
<212> DNA
<213> Artificial Sequence

<220>
<223> qPCR for ALB FP

<400> 21
gctgtcatct cttgtgggct gt 22

<210> 22
<211> 21
<212> DNA
<213> Artificial Sequence

<220>
<223> qPCR for ALB RP

<400> 22
actcatggga gctgctggtt c 21

<210> 23
<211> 26
<212> DNA
<213> Artificial Sequence

<220>
<223> Site for forward primer in oligonucleotide sequence

<400> 23
tatcttgtgg aaaggacgaa acaccg 26

<210> 24
<211> 29
<212> DNA
<213> Artificial Sequence

<220>
<223> Site for reverse primer in oligonucleotide sequence

<400> 24
gttttagagc tagaaatagc aagttaaaa 29

<210> 25
<211> 55
<212> DNA
<213> Artificial Sequence

<220>
<223> Forward primer for SpCas9 oligo library amplification

<400> 25
ttgaaagtat ttcgatttct tggctttata tatcttgtgg aaaggacgaa acacc 55

<210> 26
<211> 56
<212> DNA

<213> Artificial Sequence

<220>
<223> Reverse primer for SpCas9 oligo library amplification

<400> 26
tttcaagttg ataacggact agccttattt taacttgcta tttctagctc taaaac 56

<210> 27
<211> 61
<212> DNA
<213> Artificial Sequence

<220>
<223> Forward primer for targeted deep sequencing (SpCas9)

<400> 27

acactctttc cctacacgac gctcttccga tcttggacta tcatatgctt accgtaactt 60

g 61

<210> 28
<211> 61
<212> DNA
<213> Artificial Sequence

<220>
<223> Reverse primer for targeted deep sequencing (SpCas9)

<400> 28

gtgactggag ttcagacgtg tgctcttccg atcttttgtc tcaagatcta gttacgccaa 60

g 61

<210> 29
<211> 20
<212> DNA
<213> Artificial Sequence

<220>
<223> Forward primer for endogenous target 1-5

<400> 29
ttgctgtggc agagccagcg 20

<210> 30
<211> 27
<212> DNA
<213> Artificial Sequence

<220>
<223> Reverse primer for endogenous target 1-5

<400> 30

ttgcttcact ttaatccttt cttgcag 27

<210> 31
<211> 24
<212> DNA
<213> Artificial Sequence

<220>
<223> Forward primer for endogenous target 6-10

<400> 31
ctcctgcaag aaaggattaa agtg 24

<210> 32
<211> 28
<212> DNA
<213> Artificial Sequence

<220>
<223> Reverse primer for endogenous target 6-10

<400> 32
acctacctaa tagttacttc ctgaaggg 28

<210> 33
<211> 28
<212> DNA
<213> Artificial Sequence

<220>
<223> Forward primer for endogenous target 11-14

<400> 33
ctcgttcttt ccatcaaata gtgtggtg 28

<210> 34
<211> 28
<212> DNA
<213> Artificial Sequence

<220>
<223> Reverse primer for endogenous target 11-14

<400> 34
ctgcagtaat tgttactctg tgtcttcc 28

<210> 35
<211> 26
<212> DNA
<213> Artificial Sequence

<220>
<223> Forward primer for endogenous target 15-17

<400> 35
ttgagctgac ccataaatac aacagg 26

<210> 36

<211> 24
<212> DNA
<213> Artificial Sequence

<220>
<223> Reverse primer for endogenous target 15-17

<400> 36
ccctcttaac tggatcagca acgg 24

<210> 37
<211> 22
<212> DNA
<213> Artificial Sequence

<220>
<223> Forward primer for endogenous target 18

<400> 37
tggggtcgcc attgtagttc cc 22

<210> 38
<211> 26
<212> DNA
<213> Artificial Sequence

<220>
<223> Reverse primer for endogenous target 18

<400> 38
gtcacaaaga tcagcatcag gcatgg 26

<210> 39
<211> 20
<212> DNA
<213> Artificial Sequence

<220>
<223> Forward primer for endogenous target 19-22

<400> 39
cgttcacctg ggaggggaag 20

<210> 40
<211> 28
<212> DNA
<213> Artificial Sequence

<220>
<223> Reverse primer for endogenous target 19-22

<400> 40
tctgcaaaga actttattcc gagtaagc 28

<210> 41
<211> 28
<212> DNA
<213> Artificial Sequence

<220>
<223> Forward primer for endogenous target 23-28

<400> 41
cccaaaagac atattcaccc agaatccc 28

<210> 42
<211> 26
<212> DNA
<213> Artificial Sequence

<220>
<223> Reverse primer for endogenous target 23-28

<400> 42
caacatcaag gtgtgggcag ggctgc 26

<210> 43
<211> 21
<212> DNA
<213> Artificial Sequence

<220>
<223> Forward primer for endogenous target 29-30

<400> 43
acctggagtc tgcagagctg g 21

<210> 44
<211> 24
<212> DNA
<213> Artificial Sequence

<220>
<223> Reverse primer for endogenous target 29-30

<400> 44
aagcggtaaa caaaggatag ctgg 24

<210> 45
<211> 25
<212> DNA
<213> Artificial Sequence

<220>
<223> Forward primer for endogenous target 31-35

<400> 45
ccatgggaaa cgaatacagg tctcg 25

<210> 46
<211> 23
<212> DNA
<213> Artificial Sequence

<220>
<223> Reverse primer for endogenous target 31-35

<400> 46
cttcagaaga aaaacctcca ctc 23

<210> 47
<211> 25
<212> DNA
<213> Artificial Sequence

<220>
<223> Forward primer for endogenous target 36-37

<400> 47
aactgagaaa cagccagaga ggaag 25

<210> 48
<211> 23
<212> DNA
<213> Artificial Sequence

<220>
<223> Reverse primer for endogenous target 36-37

<400> 48
catctgatgc tgactcagag cgc 23

<210> 49
<211> 17
<212> DNA
<213> Artificial Sequence

<220>
<223> Forward primer for endogenous target 38-42

<400> 49
gctgccaccc cctgctc 17

<210> 50
<211> 26
<212> DNA
<213> Artificial Sequence

<220>
<223> Reverse primer for endogenous target 38-42

<400> 50
atcagaatga aaaatctcac ccctcc 26

<210> 51
<211> 19
<212> DNA
<213> Artificial Sequence

<220>
<223> Forward primer for endogenous target 43-46

<400> 51
gtctccgtga tgggggtgg 19

<210> 52
<211> 31
<212> DNA
<213> Artificial Sequence

<220>
<223> Reverse primer for endogenous target 43-46

<400> 52
ctgccttgta agactttaaa tattctgctc c 31

<210> 53
<211> 27
<212> DNA
<213> Artificial Sequence

<220>
<223> Forward primer for endogenous target 47-48

<400> 53
aagccatatt cagttttagg gaaaagc 27

<210> 54
<211> 26
<212> DNA
<213> Artificial Sequence

<220>
<223> Reverse primer for endogenous target 47-48

<400> 54
atttccaagt aagctgcaag gaaagc 26

<210> 55
<211> 26
<212> DNA
<213> Artificial Sequence

<220>
<223> Forward primer for endogenous target 49-52

<400> 55
aagtcttaca aggcagagta aagatc 26

<210> 56
<211> 21
<212> DNA
<213> Artificial Sequence

<220>
<223> Reverse primer for endogenous target 49-52

<400> 56
gcagggtaaa acaatcggac c 21

<210> 57
<211> 26
<212> DNA

<213> Artificial Sequence

<220>
<223> Forward primer for endogenous target 53-57

<400> 57
caaccacctc agaagagcca gattcc 26

<210> 58
<211> 26
<212> DNA
<213> Artificial Sequence

<220>
<223> Reverse primer for endogenous target 53-57

<400> 58
ctctgtagtt atttgagcaa tgccac 26

<210> 59
<211> 27
<212> DNA
<213> Artificial Sequence

<220>
<223> Forward primer for endogenous target 58-64

<400> 59
cagtgaatat acaggattgg ggttgtg 27

<210> 60
<211> 23
<212> DNA
<213> Artificial Sequence

<220>
<223> Reverse primer for endogenous target 58-64

<400> 60
acaactggta aggtgggccc agg 23

<210> 61
<211> 27
<212> DNA
<213> Artificial Sequence

<220>
<223> Forward primer for endogenous target 65-72

<400> 61
caagcacaaa caaatcaggc taaatcc 27

<210> 62
<211> 23
<212> DNA
<213> Artificial Sequence

<220>

<223> Reverse primer for endogenous target 65-72

<400> 62
ccctgagctt gggggagagt tac 23

<210> 63
<211> 21
<212> DNA
<213> Artificial Sequence

<220>
<223> Forward primer for endogenous target 73-78

<400> 63
tcctctgggg aaagagtggc c 21

<210> 64
<211> 23
<212> DNA
<213> Artificial Sequence

<220>
<223> Reverse primer for endogenous target 73-78

<400> 64
tgtggggtcg ttcctgatga aac 23

<210> 65
<211> 26
<212> DNA
<213> Artificial Sequence

<220>
<223> Forward primer for endogenous target 79-82

<400> 65
aactggttta gctagtgcat acatgc 26

<210> 66
<211> 25
<212> DNA
<213> Artificial Sequence

<220>
<223> Reverse primer for endogenous target 79-82

<400> 66
ggtgggagtt tctgttacag gcaac 25